# EUROPEAN PATENT APPLICATION

(11) **EP 3 520 782 A2**
(43) Date of publication of application: **07.08.2019**
(21) Application number: 19155101.9
(22) Date of filing: 01.02.2019
(51) Int. Cl.: A61K 9/51, A61K 9/14, A61K 9/20, A61K 47/32, A61P 25/16, A61K 31/4709, A61P 35/00

(54) **PHARMACEUTICAL FORMULATIONS CONTAINING ADENOSINE A2A RECEPTOR ANTAGONISTS**

(30) Priority: 01.02.2018 US 201862625144 P; 01.02.2018 US 201862625156 P; 01.02.2018 US 201862625158 P
(71) Applicant: Corvus Pharmaceuticals, Inc., Burlingame, CA 94010 (US)
(72) Inventor: XU, Jingrong, Burlingame,, CA 94010 (US); JONES, William, Burlingame,, CA 94010 (US); FLICKER, Felicia, Burlingame,, CA 94010 (US); BERNER, Bret, Burlingame,, CA 94010 (US)
(74) Representative: HGF Limited

(57) **Abstract**

The disclosure provides, inter alia, compositions, oral formulations, amorphous solid dispersions, extrudates, crystalline nanoparticles, and microprecipitated bulk powders containing adenosine A2A receptor antagonists, and methods of using the compositions, oral formulations, amorphous solid dispersions, extrudates, crystalline nanoparticles, and microprecipitated bulk powder to treat cancer.

## Description

### CROSS-REFERENCE TO RELATED APPLICATIONS

This application claims priority to US Application No. 62/625,144 filed February 1, 2018; to US Application No. 62/625,156 filed February 1, 2018; and to US Application No. 62/625,158 filed February 1, 2018.

### BACKGROUND

The goal of immunotherapy is to drive cytotoxic T-cell responses to eradicate cancer. To prevent reaction to self-antigens multiple inhibitory checkpoint signals exist. Extracellular adenosine is produced during acute, inflammatory processes by conversion from adenosine triphosphate through ectonucleotidases CD73 and CD39 expressed on the cell surface of multiple tissue types. Adenosine is normally upregulated to protect a host from over-injury in response to such stimuli as infection or ischemia by binding to its extracellular, G-protein coupled receptors on target cells and begin healing. However, multiple tumor types can actively sustain extracellular adenosine levels well beyond acute phase reactions to dampen a host's immune response through multiple mechanisms. Increases in adenosine in the microenvironment by malignant cells recruits regulatory T-cells to the area and further drive up adenosine levels.

Cancer cells appear to directly utilize adenosine. As a result, adenosine causes inefficient presentation of tumor antigens to the adaptive system and enhances tumor growth. Adenosine A2A receptor antagonists have been designed, and are in clinical development, to treat cancer. Thus, there is a need in the art for formulations of adenosine A2A receptor antagonists that would provide the ideal combination of exposure, safety, and stability necessary for commercial pharmaceutical products. To that end, studies have been conducted, as described herein, to develop such formulations. The disclosure is directed to these, as well as other, important ends.

### BRIEF SUMMARY

Provided herein are amorphous solid dispersions comprising an adenosine A2A receptor antagonist and a thermoplastic polymer. In aspects, the adenosine A2A receptor antagonist is a compound of Formula (I), Formula (II), Formula (III), Formula (IIIA), or Formula (IIIB). In aspects, the amorphous solid dispersions have a glass transition temperature of about 65°C or higher. In aspects, the thermoplastic polymer comprises a polyvinylpyrrolidone polymer, a polyvinylpyrrolidone copolymer, an acrylic polymer, an acrylic copolymer, hydroxypropyl methylcellulose acetate succinate, or a combination of two or more thereof. In aspects, the amorphous solid dispersions contain about 10 wt% to about 40 wt% of the adenosine A2A receptor antagonist and about 60 wt% to about 90% of the thermoplastic polymer. In aspects, the amorphous solid dispersions are oral formulations, such as tablets, capsules, granules, or powders.

Provided herein are compositions comprising an amorphous adenosine A2A receptor antagonist dispersed in a thermoplastic polymer. In aspects, the amorphous adenosine A2A receptor antagonist is an amorphous compound of Formula (I), Formula (II), Formula (III), Formula (IIIA), or Formula (IIIB). In aspects, the compositions have a glass transition temperature of about 65°C or higher. In aspects, the thermoplastic polymer comprises a polyvinylpyrrolidone polymer, a polyvinylpyrrolidone copolymer, an acrylic polymer, an acrylic copolymer, hydroxypropyl methylcellulose acetate succinate, or a combination of two or more thereof. In aspects, the compositions contain about 10 wt% to about 40 wt% of the adenosine A2A receptor antagonist and about 60 wt% to about 90% of the thermoplastic polymer. In aspects, the compositions are oral formulations, such as tablets, capsules, granules, or powders.

Provided herein are extrudates comprising an amorphous adenosine A2A receptor antagonist and a thermoplastic polymer. In aspects, the amorphous adenosine A2A receptor antagonist is an amorphous compound of Formula (I), Formula (II), Formula (III), Formula (IIIA), or Formula (IIIB). In aspects, the extrudates have a glass transition temperature of about 65°C or higher. In aspects, the thermoplastic polymer comprises a polyvinylpyrrolidone polymer, a polyvinylpyrrolidone copolymer, an acrylic polymer, an acrylic copolymer, hydroxypropyl methylcellulose acetate succinate, or a combination of two or more thereof. In aspects, the extrudates contain about 10 wt% to about 40 wt% of the adenosine A2A receptor antagonist and about 60 wt% to about 90% of the thermoplastic polymer. In aspects, the extrudates are oral formulations, such as tablets, capsules, granules, or powders.

Provided herein are methods of treating cancer in patients by administering therapeutically effective amounts of the amorphous solid dispersions, compositions, extrudates, and oral formulations described herein to treat the cancer. In aspects, the cancer is lung cancer (e.g., non-small cell lung cancer), melanoma (e.g., malignant melanoma), renal cell cancer, breast cancer (e.g., triple negative breast cancer), colorectal cancer, bladder cancer, prostate cancer, or a head and neck cancer.

The disclosure provides processes for preparing the amorphous solid dispersions, the compositions, and the extrudates described herein by the steps of (i) adding the adenosine A2A receptor antagonist and the thermoplastic polymer to a hot melt extrusion device; (ii) melting the crystalline adenosine A2A receptor antagonist and the thermoplastic polymer at temperatures close to or above the melting point of the drug and substantially less than the decomposition temperature of the drug ; (iii) mixing the adenosine A2A receptor antagonist and the thermoplastic polymer to form a homogenous blend; (iv) extruding the homogenous blend to form the amorphous solid dispersion or the solid composition. In embodiments, steps (ii) and (iii) can occur simultaneously or step (iii) can begin as soon as the crystalline adenosine A2A receptor antagonist and the thermoplastic polymer begin melting. In aspects, the adenosine A2A receptor antagonist starting material in step (i) may be crystalline, and subsequently convert to the amorphous form during the hot melt extrusion process. In aspects, the adenosine A2A receptor antagonist starting material in step (i) may be amorphous.

Provided herein are amorphous solid dispersions comprising an adenosine A2A receptor antagonist and a pharmaceutically acceptable polymer. In aspects, the adenosine A2A receptor antagonist is a compound of Formula (I), Formula (II), Formula (III), Formula (IIIA), or Formula (IIIB). The pharmaceutically acceptable polymer can be an enteric polymer. In aspects, the amorphous solid dispersions are oral formulations, such as tablets, capsules, granules, or powders.

Provided herein are compositions comprising an amorphous adenosine A2A receptor antagonist dispersed in a pharmaceutically acceptable polymer. In aspects, the adenosine A2A receptor antagonist is a compound of Formula (I), Formula (II), Formula (III), Formula (IIIA), or Formula (IIIB). The pharmaceutically acceptable polymer can be an enteric polymer. In aspects, the compositions are solid compositions. In aspects, the compositions are oral formulations, such as tablets, capsules, granules, or powders.

Provided herein are microprecipitated bulk powders comprising an amorphous adenosine A2A receptor antagonist and a pharmaceutically acceptable polymer. In aspects, the adenosine A2A receptor antagonist is a compound of Formula (I), Formula (II), Formula (III), Formula (IIIA), or Formula (IIIB). The pharmaceutically acceptable polymer can be an enteric polymer. In aspects, the microprecipitated bulk powders are oral formulations, such as tablets, capsules, granules, or powders.

Provided herein are methods of treating cancer in patients by administering therapeutically effective amount of the amorphous solid dispersions, compositions, microprecipitated bulk powders, and oral formulations described herein to treat the cancer. In aspects, the cancer is lung cancer (e.g., non-small cell lung cancer), melanoma (e.g., malignant melanoma), renal cell cancer, breast cancer (e.g., triple negative breast cancer), colorectal cancer, bladder cancer, prostate cancer, or a head and neck cancer.

The disclosure provides processes for manufacturing the amorphous solid dispersions, compositions, and microprecipitated bulk powders described herein by coprecipitating the adenosine A2A receptor antagonist and the pharmaceutically acceptable polymer.

Provided herein are pharmaceutical compositions comprising drug nanoparticles comprising adenosine A2A receptor antagonists and a pharmaceutically acceptable excipient, such as a polymer, a surfactant, or a combination thereof. In aspects, the drug nanoparticles have a particle size distribution with a D90 of about 500 nm or less. In aspects, the drug nanoparticles are crystalline. In aspects, the adenosine A2A receptor antagonist is a compound of Formula (I), Formula (II), Formula (III), Formula (IIIA), Formula (IIIB), or a pharmaceutically acceptable salt of any of the foregoing. In some aspects, the pharmaceutical compositions have an outer coating layer.

Provided herein are tablets comprising drug nanoparticles comprising adenosine A2A receptor antagonists and a pharmaceutically acceptable excipient, such as a polymer, a surfactant, or a combination thereof. In aspects, the drug nanoparticles have a particle size distribution with a D90 of about 500 nm or less. In aspects, the drug nanoparticles are crystalline. In aspects, the adenosine A2A receptor antagonist is a compound of Formula (I), Formula (II), Formula (III), Formula (IIIA), Formula (IIIB), or a pharmaceutically acceptable salt of any of the foregoing. In aspects, the tablets have an outer coating layer. In aspects, the outer coating layer is an outer enteric coating layer.

Provided herein are granules comprising drug nanoparticles comprising adenosine A2A receptor antagonists and a pharmaceutically acceptable excipient, such as a polymer, a surfactant, or a combination thereof. In aspects, the drug nanoparticles have a particle size distribution with a D90 of about 500 nm or less. In aspects, the drug nanoparticles are crystalline. In aspects, the adenosine A2A receptor antagonist is a compound of Formula (I), Formula (II), Formula (III), Formula (IIIA), Formula (IIIB), or a pharmaceutically acceptable salt of any of the foregoing. In aspects, the granules have an outer coating layer. In aspects, the granules, optionally with an outer coating layer, are compressed into a tablet, are placed within a capsule shell, or are placed within a sachet or stick pack. In aspects, the outer coating layer is an outer enteric coating layer. In aspects, the granules, optionally with an outer enteric coating layer, are compressed in to a tablet, are placed with a capsule shell, or are placed within a sachet or stick pack.

Provided herein are powders comprising drug nanoparticles comprising adenosine A2A receptor antagonists and a pharmaceutically acceptable excipient, such as a polymer, a surfactant, or a combination thereof. In aspects, the drug nanoparticles have a particle size distribution with a D90 of about 500 nm or less. In aspects, the drug nanoparticles are crystalline. In aspects, the adenosine A2A receptor antagonist is a compound of Formula (I), Formula (II), Formula (III), Formula (IIIA), Formula (IIIB), or a pharmaceutically acceptable salt of any of the foregoing. In aspects, the powder is compressed into a tablet, placed within a capsule shell, or placed within a sachet or stick pack.

Provided herein are beads comprising an inert core and an outer layer, wherein the outer layer comprises drug nanoparticles which comprise adenosine A2A receptor antagonists and a pharmaceutically acceptable excipient, such as a polymer, a surfactant, or a combination thereof. In aspects, the drug nanoparticles have a particle size distribution with a D90 of about 500 nm or less. In aspects, the drug nanoparticles are crystalline. In aspects, the adenosine A2A receptor antagonist is a compound of Formula (I), Formula (II), Formula (III), Formula (IIIA), Formula (IIIB), or a pharmaceutically acceptable salt of any of the foregoing. In aspects, the beads have an outer coating layer. In aspects, the beads, optionally with an outer enteric layer, are compressed into a tablet, are placed within a capsule shell, or are placed within a sachet or stick pack. In aspects, the outer coating layer is an outer enteric coating layer.

Provided herein are capsules having encapsulated within the capsule shell the pharmaceutical compositions, granules, powders, or beads described herein. In aspects, the capsules have an outer coating layer. In aspects, the capsules have an outer enteric coating layer. In aspects, the outer coating layer is an outer enteric coating layer.

Provided herein are methods of treating cancer, such as non-small cell lung cancer, melanoma, renal cell cancer, breast cancer, colorectal cancer, bladder cancer, prostate cancer, and head and neck cancers, by administering to a patient in need of treatment an effective amount of the pharmaceutical compositions, tablets, capsules, granules, powders, pellets, minitablets, or beads described herein. In aspects, the pharmaceutical compositions, oral formulations, tablets, capsules, granules, powders, or beads described herein are administered to the patient once daily (QD). In aspects, the pharmaceutical compositions, oral formulations, tablets, capsules, granules, powders, or beads described herein are administered to the patient twice daily (BID).

The pharmaceutical compositions, amorphous dispersions, extrudates, microprecipitated bulk powders, crystalline nanoparticles, and oral formulations (e.g., tablets, capsules, granules, powders, beads) described herein have unexpectedly superior properties. In aspects the pharmaceutical compositions, amorphous dispersions, extrudates, microprecipitated bulk powders, crystalline nanoparticles, oral formulations (e.g., tablets, capsules, granules, powders, beads) described herein have unexpectedly superior bioavailability. In aspects the pharmaceutical compositions, amorphous dispersions, extrudates, microprecipitated bulk powders, crystalline nanoparticles, oral formulations (e.g., tablets, capsules, granules, powders, beads) described herein have unexpectedly superior stability. In aspects the pharmaceutical compositions, amorphous dispersions, extrudates, microprecipitated bulk powders, crystalline nanoparticles, oral formulations (e.g., tablets, capsules, granules, powders, beads) described herein have an unexpectedly superior dissolution profile. In aspects the pharmaceutical compositions, amorphous dispersions, extrudates, microprecipitated bulk powders, crystalline nanoparticles, oral formulations (e.g., tablets, capsules, granules, powders, beads) described herein have an unexpectedly superior disintegration profile. In aspects the pharmaceutical compositions, amorphous dispersions, extrudates, microprecipitated bulk powders, crystalline nanoparticles, oral formulations (e.g., tablets, capsules, granules, powders, beads) described herein have unexpectedly superior pharmacokinetic properties. In aspects the pharmaceutical compositions, amorphous dispersions, extrudates, microprecipitated bulk powders, crystalline nanoparticles, oral formulations (e.g., tablets, capsules, granules, powders, beads) described herein have unexpectedly superior Cₘᵢₙ, Cₘₐₓ, Tₘₐₓ, or AUC.

These and other embodiments of the disclosure are described in more detail herein.

### BRIEF DESCRIPTION OF THE FIGURES

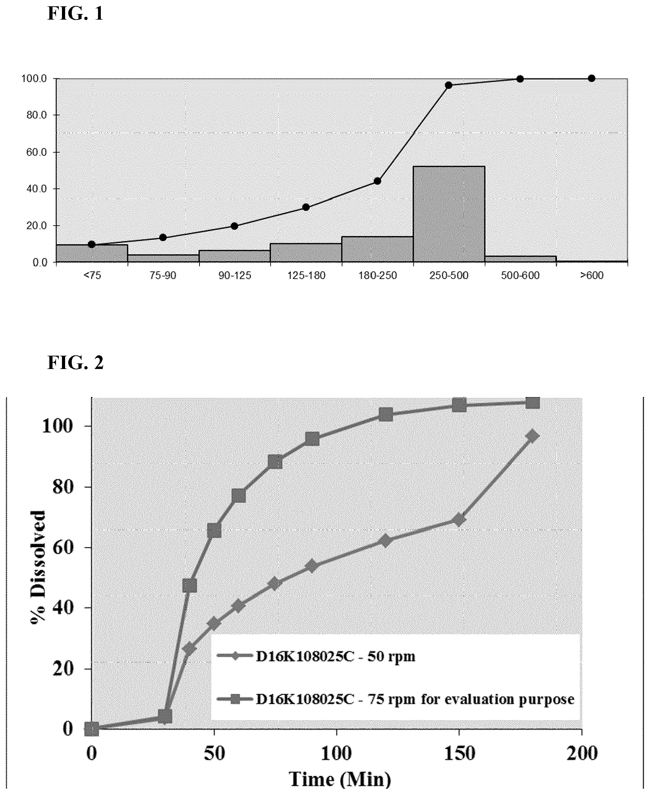

FIG. 1 shows a particle size distribution of the milled extrudate produced by the process described herein of the amorphous solid dispersion of Formula (III).
FIG. 2 shows the results of a two-stage dissolution method performed on a tablet comprising an extrudate using 900 mL of 0.1 N HCl in a USP-Type II apparatus (paddle) at 50 rpm for 30 minutes, followed by immersion in 900 mL of pH 6.8 phosphate buffer, USP-Type II apparatus (paddle) at 50 rpm, both at 37°C. Results from a trial at 75 rpm are also shown..

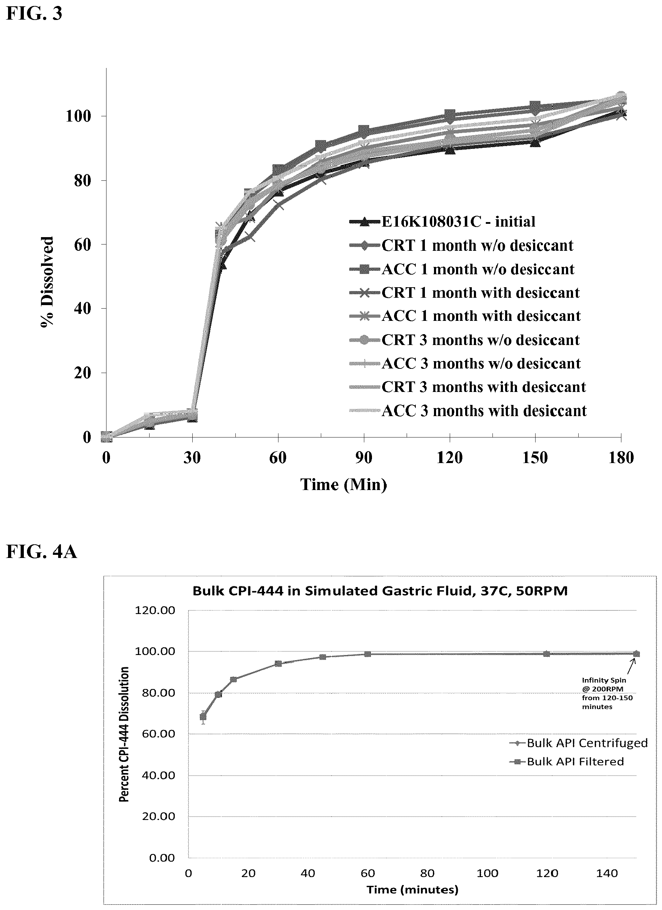

FIG. 3 shows the results of a two-stage dissolution method performed on a tablet comprising an extrudate using 900 mL of 0.1 N HCl in a USP-Type II apparatus (paddle) at 50 rpm for 30 minutes, followed by immersion in 900 mL of pH 6.8 phosphate buffer, USP-Type II apparatus (paddle) at 50 rpm, both at 37°C of formulations sampled after different storage times with and without desiccant.

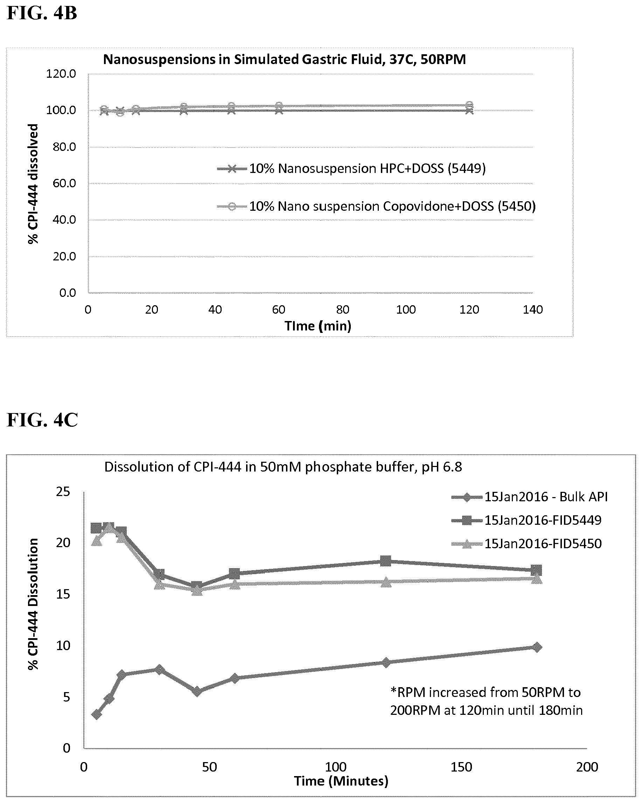

FIGS. 4A-4C are dissolution graphs. FIG. 4A shows the dissolution behavior of the adenosine A2A receptor antagonists before nanomilling in acid medium where the drug is fully dissolved (sink condition). FIG. 4B is the dissolution behavior of the adenosine A2A receptor antagonists after nanomilling in acid medium where the drug is fully dissolved (sink condition). FIG 4C shows the comparison of the dissolution of the adenosine A2A receptor antagonists before and after nanomilling in phosphate buffer at pH 6.8 (compound is only partially dissolved, non-sink condition).

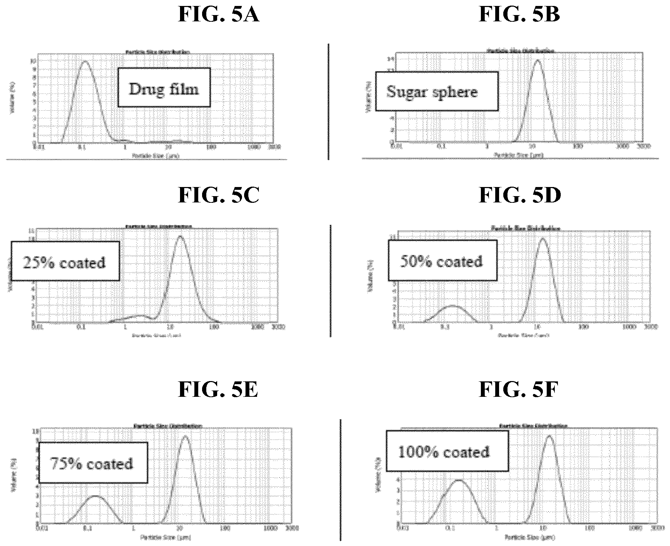

FIGS. 5A-5F show various particle size distributions (PSD) at different coating levels confirming nano drug layering process using fluid bed processer had no impact on the drug particle size distribution. FIG. 5A shows the particle size distribution of the drug film. FIG. 5B shows particle size distribution of inert cores. FIG. 5C shows particle size distribution of beads having a 25% of the full coating layer. FIG. 5D shows particle size distribution of beads having a 50% of the full coating layer. FIG. 5E shows particle size distribution of beads having a 75% of the full coating layer. FIG. 5F shows particle size distribution of beads having a 100% coating layer. The samples were sonicated in water before measurement. The PSD of nano-particles on sugar spheres was similar to that of PSD of nano particles in the film

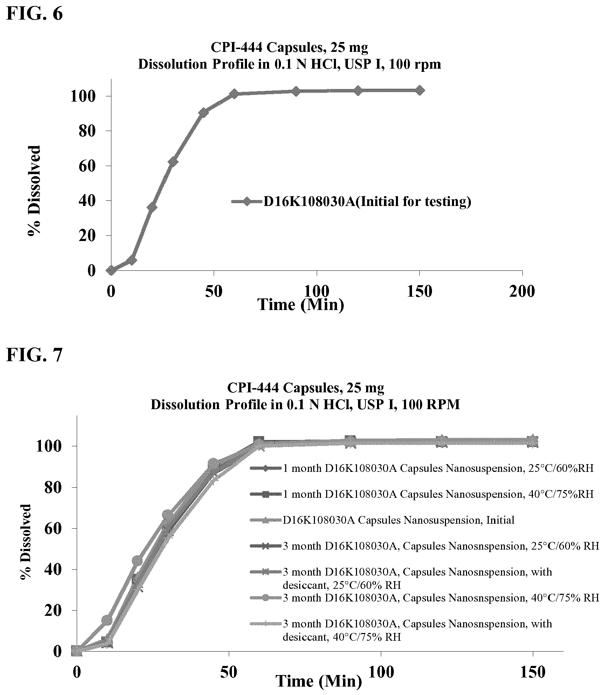

FIG. 6 shows the dissolution profile of capsules containing beads with a nanomilled drug coating.
FIG. 7 shows the dissolution profile of capsules containing beads with a nanomilled drug coating after various controlled and accelerated storage conditions.

### DETAILED DESCRIPTION

### Definitions

The terms used throughout this specification are used in accordance with their plain and ordinary meaning within chemistry and biology.

"Hot melt extrusion" or "HME" refers to a process whereby a blended composition is heated and/or compressed to a molten (or softened) state and subsequently forced through an orifice where the extruded product (extrudate) is formed into its final shape in which it solidifies upon cooling. The blended composition is conveyed through one or more heating zones typically by a screw mechanism. The screw or screws are rotated by a variable speed motor inside a cylindrical barrel where only a small gap exists between the outside diameter of the screw and the inside diameter of the barrel. In this conformation, high shear is created at the barrel wall and between the screw flights by which the various components of the powder blend are well mixed and disaggregated while being transported through the extruder.

"Extrudate" refers to a composition formed by hot melt extrusion. In aspects, the composition is amorphous.

"Microprecipitated bulk powder" or "MBP" is a composition comprising an amorphous drug produced by the co-precipitation of an adenosine A2A receptor antagonist and a pharmaceutically acceptable polymer. Briefly, an organic solvent is used to dissolve the adenosine A2A receptor antagonist and the pharmaceutically acceptable polymer; thereafter, an anti-solvent is added to co-precipitate the adenosine A2A receptor antagonist and pharmaceutically acceptable polymer. The solvent is then extracted by washing, and the amorphous co-precipitated materials are filtered and dried. In aspects, the organic solvent is a polar aprotic solvent.

"Organic solvent" refers to liquid substances that can form a solution by dissolving the adenosine A2A receptor antagonists and pharmaceutically acceptable polymers described herein. Exemplary organic solvents include polar aprotic solvents, tetrahydrofuran, ethyl acetate, ethanol, methanol, acetic acid, and the like.

"Polar aprotic solvent" refers to solvents that do not have an acidic hydrogen atom. Exemplary polar aprotic solvents include N,N-dimethylacetamide, N-methylpyrrolidone, acetone, dimethylformamide, acetonitrile, dimethyl sulfoxide, propylene carbonate, and the like. Generally, the polar aprotic solvent is miscible with an anti-solvent.

"Anti-solvent" refers to acidified water or a compound that is miscible with a polar aprotic solvent and that is capable of precipitating from solution the adenosine A2A receptor antagonists and pharmaceutically acceptable polymers described herein.

"Acidified water" refers to water having a pH of about 5 or less. In aspects, the pH is from about 1 to about 5; or from about 1 to about 4; or from about 2 to about 5; or from about 2 to about 4; or from about 2 to about 3; or about 2; or about 3. For purposes of serving as an anti-solvent, the acidified water is generally at a temperature from about 2°C to about 8°C.

"Crystalline" refers to a compound that has an ordered, three-dimensional lattice of atoms and/or molecules, as determined by methods known in the art, such as X-ray powder diffraction, IR-spectroscopy, Raman spectroscopy, differential scanning calorimetry (DSC), solid-state NMR, polarized light microscopy, or by its melting point. See, e.g., Remington: The Science and Practice of Pharmacy, 21st Ed., Lippincott, Williams and Wilkins, Baltimore, MD (2005); The United States Pharmacopeia, 23rd Ed., (1995). To be clear, where a compound is disclosed herein as being crystalline, a person having ordinary skill in the art will immediately recognize that the compound forms part of a crystalline solid that includes a plurality of that compound.

"Amorphous" refers to a solid compound (i.e. a solid that includes a plurality of a compound) or a solid composition without long-range crystalline order. In aspects, amorphous refers to a compound or composition that is substantially free of crystalline forms. That is, amorphous refers to a solid that includes a compound (e.g. a plurality of pharmaceutically active agents or drugs) wherein the solid is substantially free of crystalline forms. In aspects, an amorphous compound (i.e. a solid that includes a plurality of a compound) or composition has less than 10% of the compound (i.e. a solid that includes a plurality of a compound) or composition in a crystalline form; or less than 5%; or less than 2% of the compound(i.e. a solid that includes a plurality of a compound) or composition in crystalline form. In aspects, an amorphous compound or composition has less than 1% of the compound (i.e. a solid that includes a plurality of a compound) or composition in crystalline form. In aspects, an amorphous compound (i.e. a solid that includes a plurality of a compound) or composition does not have crystallinity as determined by X-ray powder diffraction, IR-spectroscopy, Raman spectroscopy, differential scanning calorimetry (DSC), solid-state NMR, polarized light microscopy, or by its melting point. In aspects, an amorphous compound (i.e. a solid that includes a plurality of a compound), an amorphous solid dispersion, an amorphous composition (i.e. a solid that includes a plurality of a composition), or an amorphous extrudate, as described herein, remain amorphous for a period of time longer than 18 months; longer than 2 years; longer than 3 years; longer than 4 years; or longer than 5 years. To be clear, where a compound (e.g. drug or active agent) is disclosed herein as being amorphous, a person having ordinary skill in the art will immediately recognize that the compound forms part of an amorphous solid that includes a plurality of that compound. Likewise, where a compound is disclosed herein as being a solid (e.g. "solid compound"), a person having ordinary skill in the art will immediately recognize that the compound forms part of a solid that includes a plurality of that compound.

"Solid dispersion" refers to an active agent homogenously dispersed in a pharmaceutically acceptable excipient. In aspects, an adenosine A2A receptor antagonist, such as those described herein, is homogenously dispersed in a thermoplastic polymer, such as those described herein, where the adenosine A2A receptor antagonist is amorphous. In aspects, an adenosine A2A receptor antagonist, such as those described herein, is homogenously dispersed in a thermoplastic polymer, such as those described herein, where the adenosine A2A receptor antagonist and thermoplastic polymer are both amorphous. Methods of making solid dispersions are well-known in the art and include, for example, hot melt extrusion techniques, co-precipitation techniques, spray-drying techniques, and the like. To be clear, where an active agent is disclosed herein as a solid dispersion, a person having ordinary skill in the art will immediately recognize that the compound forms part of a solid that includes a plurality of that compound dispersed therein.

The term "homogeneous," "homogeneously" and the like, as used herein in the context of formulations and dispersions, is used in accordance with the usual and customary meaning to refer to substantial homogeneity, including complete homogeneity. In aspects, the terms are used to refer to substantial homogeneity. In aspects, the terms are used to refer to complete homogeneity.

"Glass transition temperature" or "Tg" refers to the temperature where the reversible transition of amorphous materials from a hard and relatively brittle state into a viscous and rubbery state (as temperature increases). The glass transition temperatures of adenosine A2A receptor antagonists and thermoplastic polymers can be determined by process that are well-known in the art. In aspects, an amorphous compound, an amorphous solid dispersion, an amorphous composition, or an amorphous extrudate, as described herein, have a ratio of Tm/Tg in degrees Kelvin from about 1 to about 1.42, or from about 1.03 to about 1.36, where Tm is the melting point of the pure adenosine A2A receptor antagonist, and Tg is the glass transition temperature of the amorphous dispersion composed of the adenosine A2A receptor antagonist and the thermoplastic polymer. In aspects, the ratio of Tm/Tg in degrees Kelvin is from about 1 to about 1.42, or from about 1.03 to about 1.36, where Tg is the glass transition temperature of the amorphous dispersion at that composition of the drug and the thermoplastic polymer. In aspects, the ratio of Tm/Tg in degrees Kelvin is from about 1 to about 1.42, or from about 1.03 to about 1.36, where Tm is the melting point of the adenosine A2A receptor antagonist.

"Pharmaceutically acceptable polymer" refers to any polymer known in the art that can be used with the pharmaceutical compositions described herein and/or that can be used with microprecipitation or hot melt extrusion or formation of nanoparticles processes and/or that can be used to form a microprecipitated bulk powder, extrudate, or nanoparticles. In aspects, "pharmaceutically acceptable polymer" is a non-crosslinked pharmaceutically acceptable polymer. In aspects, "pharmaceutically acceptable polymer" is a crosslinked pharmaceutically acceptable polymer. Exemplary pharmaceutically acceptable polymers include polyvinylpyrrolide polymers, polyvinylpyrrolide copolymers, cellulose compounds, poly(lactide), poly(glycolide), poly(lactide-co-glycolide) copolymers, poloxamers, polysorbates (e.g., polysorbate 80, polysorbate 20), enteric coating materials, polyethylene oxide, pullulan, polyvinyl alcohol, sodium alginate, polyethylene glycol, xanthan gum, tragacanth gum, guar gum, acacia gum, arabic gum, polyacrylic acid, methylmethacrylate copolymer, carboxyvinyl copolymers, starch, gelatin, polydioxanes, polyoxalates, poly(α-esters), polyanhydrides, polyacetates, polycaprolactones, poly(orthoesters), polyamino acids, polyaminocarbonates, polyurethanes, polycarbonates, polyamides, poly(alkyl cyanoacrylates), and the like. In aspects, the pharmaceutically acceptable polymer is a thermoplastic polymer. In aspects, the pharmaceutically acceptable polymer is an enteric polymer. In aspects, the pharmaceutically acceptable polymer that can be used with microprecipitation processes or hot melt extrusion or formation of nanoparticles and/or that can be used to form a microprecipitated bulk powder is an enteric polymer.

"Thermoplastic polymer" refers to a polymer that goes through a glass transition temperature when heat is applied while maintaining good chemical stability. Thermoplastic polymers are suitable for use in hot melt extrusion processes. In aspects, the thermoplastic polymer is a pharmaceutically acceptable polymer.

"PVP" refers to polyvinylpyrrolidone or a polymer of vinylpyrrolidone. PVP is a class of polymers that can have an average molecular weight from about 1,000 Daltons to about 1,500,000 Daltons. In aspects, the PVP can be a crosslinked PVP. PVP is an exemplary pharmaceutically acceptable polymer and thermoplastic polymer. In aspects, the polyvinylpyrrolidone polymer has an average molecular weight from about 1,000 Daltons to about 1,000,000 Daltons; or from about 1,000 Daltons to about 500,000 Daltons; or from about 1,000 Daltons to about 200,000. In aspects, the polyvinylpyrrolidone polymer has an average molecular weight from about 1,000 Daltons to about 150,000 Daltons. In aspects, the polyvinylpyrrolidone polymer has an average molecular weight from about 10,000 Daltons to about 150,000 Daltons. In aspects, the polyvinylpyrrolidone polymer has an average molecular weight from about 50,000 Daltons to about 150,000 Daltons. In aspects, the polyvinylpyrrolidone polymer has an average molecular weight from about 60,000 Daltons to about 120,000 Daltons. PVP is commercially available from numerous sources, such as Sigma Aldrich.

"Polyvinylpyrrolidone copolymer" or "copolymer of vinylpyrrolidone" refers to copolymers comprising vinylpyrrolidone and one or more other monomers, such as acrylic monomers, styrene, vinyl acetate, and the like. Polyvinylpyrrolidone vinyl acetate copolymer and Copovidone are exemplary polyvinylpyrrolidone copolymers. In aspects, the polyvinylpyrrolidone copolymer has an average molecular weight from about 1,000 Daltons to about 1,000,000 Daltons; or from about 1,000 Daltons to about 500,000 Daltons; or from about 1,000 Daltons to about 200,000. In aspects, the polyvinylpyrrolidone copolymer has an average molecular weight from about 1,000 Daltons to about 150,000 Daltons. In aspects, the polyvinylpyrrolidone copolymer has an average molecular weight from about 10,000 Daltons to about 150,000 Daltons. In aspects, the polyvinylpyrrolidone copolymer has an average molecular weight from about 50,000 Daltons to about 150,000 Daltons. Polyvinylpyrrolidone copolymers are an exemplary pharmaceutically acceptable polymer and thermoplastic polymer.

"Polyvinylpyrrolidone vinyl acetate copolymer" or "copolymer of polyvinylpyrrolidone and vinyl acetate" or "copolymer of vinylpyrrolidone and vinyl acetate" refer to a class of copolymers of vinylpyrrolidone and vinyl acetate that having varying wt% ratios of vinylpyrrolidone to vinyl acetate, such as from about 30:70 to about 70:30, including 30:70, 35:65, 50:50, 60:40, and 70:30. The wt% ratios of vinylpyrrolidone to vinyl acetate can determine different properties of the copolymer, including the glass transition temperature. Polyvinylpyrrolidone vinyl acetate copolymer is an exemplary pharmaceutically acceptable polymer and thermoplastic polymer. Polyvinylpyrrolidone vinyl acetate copolymer is commercially available from numerous sources.

"Copovidone" refers to a copolymer of 1-vinyl-2-pyrrolidone and vinyl acetate at the weight ratio of 1-vinyl-2-pyrrolidone to vinyl acetate of about 3:2. "Copovidone" is commercially available as KOLLIDON® VA64 from BASF Corporation, Florham Park, NJ.

"Acrylic polymer" or "acrylates" or "polyacrylic acid" refers to polymers comprised of acrylate monomers, e.g., homopolymers of acrylic acid crosslinked with allyl ether pentaerythritol, allether of sucrose, or allyl ether of propylene. Exemplary acrylic monomers include acrylic acid, methacrylate (methacrylic acid), methyl acrylate, ethyl acrylate, butyl acrylate, 2-chloroethyl vinyl ether, 2-ethylhexyl acrylate, hydroxyethyl methacrylate, methyl methacrylate, ethyl methacrylate, butyl methacrylate, and the like. Acrylic polymers are commercially available in varying molecular weights, such as from about 2,000 Daltons to about 1,500,000 Daltons. Acrylic polymers are exemplary pharmaceutically acceptable polymers and thermoplastic polymers.

"Acrylic copolymer" refers to polymers comprised of at least two different acrylate monomers. Exemplary acrylic monomers include acrylic acid, methacrylate (methacrylic acid), methyl acrylate, ethyl acrylate, butyl acrylate, 2-chloroethyl vinyl ether, 2-ethylhexyl acrylate, hydroxyethyl methacrylate, methyl methacrylate, ethyl methacrylate, butyl methacrylate, and the like. Exemplary acrylic copolymers include copolymers of methacrylic acid and ethyl acrylate, and copolymer of methacrylic acid and methyl methacrylate. Acrylic copolymers are commercially available. Acrylic copolymers are exemplary pharmaceutically acceptable polymers and thermoplastic polymers.

"Methyl methacrylate polymer" refers to a polymer formed by monomeric units of methyl methacrylate. A methyl methacrylate polymer is an exemplary thermoplastic polymer.

"Ethyl acrylate polymer" refers to a polymer formed by monomeric units of ethyl acrylate. An ethyl acrylate polymer is an exemplary pharmaceutically acceptable polymer and thermoplastic polymer.

"A copolymer of methyl methacrylate and ethyl acrylate" refers to a copolymer formed by monomeric units of methyl methacrylate and ethyl acrylate. The weight ratio of methyl methacrylate to ethyl acrylate in the copolymer can vary, e.g., having a weight ratio of 10:1 to 1:10. A copolymer of methyl methacrylate and ethyl acrylate is an exemplary pharmaceutically acceptable polymer and thermoplastic polymer.

"A copolymer of methacrylic acid and methyl methacrylate" refers to a copolymer formed by monomeric units of methacrylic acid and methyl methacrylate. The weight ratio of methacrylic acid to methyl methacrylate in the copolymer can vary, e.g., having a weight ratio of 10:1 to 1:10. A copolymer of methacrylic acid and methyl methacrylate is an exemplary pharmaceutically acceptable polymer and thermoplastic polymer.

"EUDRAGIT® L100-55" or "L100-55" refers to a copolymer of methacrylic acid and ethyl acrylate at the weight ratio of methacrylic acid to ethyl acrylate of about 1:1. L100-55 is an exemplary pharmaceutically acceptable polymer and thermoplastic polymer. EUDRAGIT® is a registered trademark of Evonik Roehm GMBH.

"EUDRAGIT® L100" or "L100" refers to a copolymer of methacrylic acid and methyl methacrylate at the weight ratio of methacrylic acid to methyl methacrylate of 1:1. L100 is an exemplary pharmaceutically acceptable polymer and thermoplastic polymer. EUDRAGIT® is a registered trademark of Evonik Roehm GMBH.

"HPC" refers to hydroxypropyl cellulose. HPC is an ether of cellulose in which some of the hydroxyl groups in the repeating glucose units are hydroxypropylated using propylene oxide. HPC is an exemplary pharmaceutically acceptable polymer and thermoplastic polymer.

"HPMC" refers to hydroxypropyl methylcellulose or hypromellose. HPMC is a propylene glycol ether of methyl cellulose, hydroxypropyl and methyl combined with an anhydrous glucose ring by an ether bond. The percentage of each component (e.g., methoxyl groups, hydroxypropyl groups) can vary. HPMC is an exemplary pharmaceutically acceptable polymer and thermoplastic polymer. HPMC HME (Affinisol) is especially made for use as a thermoplastic polymer for hot melt extrusion as 3 grades of viscosities and molecular weights as HPMC HME 15 cP, 100 cP, and 4M.

"HPMC E3" is hydroxypropyl methylcellulose that has an average of about 29% methoxyl groups and about 10% hydroxypropyl groups. HPMC E3 is an exemplary pharmaceutically acceptable polymer and thermoplastic polymer.

"HPMCAS" refers to hydroxypropyl methylcellulose acetate succinate. HPMCAS is a mixture of acetic acid and monosuccinic acid esters of hydroxypropyl methylcellulose. Generally, HPMCAS comprises from about 12% to about 28% of methoxy groups, from about 4% to about 23% of hydroxypropoxy groups, from about 2% to about 16% acetyl groups, and about 4% to about 28% of succinoyl groups, calculated on a dry basis. HPMCAS is an exemplary pharmaceutically acceptable polymer and thermoplastic polymer. In aspects, HPMCAS is HPMCAS-LF.

"HPMCAS-LF" and "HPMC AS-LF" refer to hydroxypropyl methylcellulose acetate succinate that has a mean particle size of about 5 µm and a viscosity of about 3 mPa•s. HPMCAS-LF is an exemplary pharmaceutically acceptable polymer and thermoplastic polymer.

"Poloxamer" refers to the compound: HO-[CH₂CH₂-O]ₐ-[CH(CH₃)-CH₂-O]_{b}-[CH₂CH₂-O-]ₐ-H wherein a and b are varied. "Poloxamer 407" refers to the compound HO-[CH₂CH₂-O]ₐ-[CH(CH₃)-CH₂-O]_{b}-[CH₂CH₂-O-]ₐ-H wherein a is about 101 and b is about 56. Poloxomer 407 is available as LUTROL® F127 from BASF.

"Cellulose" or "cellulose compound" or "cellulose polymer" refers to an organic polysaccharide compound comprising a chain of β(1→4) liked D-glucose units having the formula (C₆H₁₀O₅)ₙ. Cellulose compounds include hydroxyalkyl cellulose compounds, alkyl cellulose compounds, and carboxyalkyl cellulose compounds.

"Hydroxyalkyl cellulose" refers to an ether derivative of a cellulose compound having a hydroxyalkyl group. Examples of hydroxyalkyl cellulose include hydroxypropyl cellulose, hydroxyethyl cellulose, hydroxyethylmethyl cellulose, ethyl hydroxyethyl cellulose, hydroxypropylmethyl cellulose, and the like.

"Alkyl cellulose" refers to an ether derivative of a cellulose compound having an alkyl group. Examples of alkyl cellulose include methyl cellulose, ethyl cellulose, ethylmethyl cellulose, and the like.

"Carboxyalkyl cellulose" refers to an ether derivative of a cellulose compound having a carboxyl alkyl group and their salts. Examples of carboxyalkyl cellulose include carboxymethyl cellulose, and the like.

"Pharmaceutically acceptable excipient" refers to a substance that aids the administration of an adenosine A2A receptor antagonist to and absorption by a patient, or to a substance that assists in the manufacture of the granules, beads, powders, capsules, sachets, stick packs, tablets, compositions, and formulations described herein. Pharmaceutically acceptable excipients are inert. Non-limiting examples of pharmaceutically acceptable excipients include pharmaceutically acceptable polymers, water, NaCl, normal saline solutions, normal sucrose, normal glucose, binders, fillers, disintegrants, lubricants, surfactants, coatings, sweeteners, flavors, salt solutions, alcohols, oils, gelatins, carbohydrates, colors, and the like. Such preparations can be mixed with auxiliary agents such as lubricants, preservatives, stabilizers, wetting agents, emulsifiers, salts for influencing osmotic pressure, buffers, coloring, and/or aromatic substances and the like. Pharmaceutically acceptable excipients are described in the Handbook of Pharmaceutical Excipients, 8th Edition, published by the Pharmaceutical Press (2017), and in the United States Food and Drug Administration Inactive Ingredient Database (July 2017), the disclosures of which are incorporated by reference herein. The pharmaceutical compositions (including extrudates, amorphous solid dispersions, oral formulations) described herein may comprise one or more pharmaceutically acceptable excipients. One of skill in the art will recognize that other pharmaceutically acceptable excipients would be useful in the compositions described herein.

"Capsule" refers to a shell encapsulating a composition. The shell can be a hard shell or a soft shell, and generally comprise gelatin, a polymer (such as a hydroxyalkyl cellulose, an alkyl cellulose, a carboxyalkyl cellulose, or a combination of two or more thereof), or a combination thereof. In aspects, the capsule shell comprises a hydroxyalkyl cellulose. In aspects, the capsule shell comprises hydroxypropylmethyl cellulose. In aspects, the capsule shell comprises an enteric polymer.

"Outer layer" refers to a layer completely surrounding a composition, tablet, capsule, bead, or granule. In aspects, the outer layer is a polymer, such as polyvinyl alcohol, a hydroxyalkyl cellulose, an alkyl cellulose, a carboxyalkyl cellulose, or a combination of two or more thereof. In aspects, the outer layer is a protective coating or an aesthetic coating. In aspects, the outer layer is an enteric coating. In aspects, the outer layer is a protective coating, an aesthetic coating, an enteric coating, or a combination of two or more thereof. In aspects, the outer layer is a single layer or the outer layer has multiple layers (e.g., 2, 3 layers).

"Enteric coating" refers to a coating containing one or more materials that dissolve or disintegrate in the small intestine, but not in the stomach or gastric environment. Exemplary enteric coating materials include enteric polymers.

"Enteric polymer" refers to a polymer that will dissolve or disintegrate at a pH from about 5 to about 7.4, but that will not dissolve or disintegrate at a pH from about 1 to about 4.5. Exemplary enteric polymers include polymerized gelatin, shellac, methacrylic acid copolymers, cellulose butyrate phthalate, cellulose hydrogen phthalate, cellulose propionate phthalate, polyvinyl acetate phthalate, cellulose acetate phthalate, cellulose acetate trimellitate, hydroxypropyl methylcellulose phthalate, hydroxypropyl methylcellulose acetate, dioxypropyl methylcellulose succinate, carboxymethyl ethylcellulose, hydroxypropyl methylcellulose acetate succinate, and acrylic acid polymers and copolymers, typically formed from methyl acrylate, ethyl acrylate, methyl methacrylate and/or ethyl methacrylate with copolymers of acrylic and methacrylic acid esters (e.g., EUDRAGIT® NE, EUDRAGIT® RL, EUDRAGIT® RS by Evonik Roehm GMBH).

"Inert core" refers to a pharmacologically inactive particle of any shape. The inert core can be soluble, insoluble, or a combination thereof. In aspects an inert core is spherical. In aspects, an inert core comprises sugar, microcrystalline cellulose, carnauba wax, mannitol, or a combination of two or more thereof. An inert core has a size greater than 25 microns; or from about 50 microns to about 3 mm; or from about 1 mm to about 2.5 mm. Inert cores are commercially available, e.g., Pharmatrans Sanaq AG, Switzerland. When used in reference to a bead, an "inert core" is surrounded by a "drug layer," which is a layer of any thickness comprising the adenosine A2A receptor antagonists described herein and a pharmaceutically acceptable excipient. The drug layer can be a single layer or multiple layers.

The term "drug nanoparticle" or "drug nanoparticles" or "crystalline nanoparticles" as used herein means a particle(s) composed of A2A receptor antagonist (or pharmaceutically acceptable salts thereof). In aspects, the drug nanoparticles are a solid. In aspects, the drug nanoparticle includes no other active pharmaceutical ingredients other than one or more A2A receptor antagonists. In aspects, the drug nanoparticle includes only a single type of A2A receptor antagonist. In aspects, the drug nanoparticle includes only a single type of A2A receptor antagonist and no other active pharmaceutical ingredient. In aspects, the drug nanoparticle includes only a single type of A2A receptor antagonist and no pharmaceutical excipients or other active pharmaceutical ingredients. In aspects, the drug nanoparticle includes only a single type of A2A receptor antagonist, optionally water molecules, and no pharmaceutical excipients or other active pharmaceutical ingredients. In aspects, the drug nanoparticles are crystalline. In aspects, the drug nanoparticle refers to all the drug particles in the composition. In aspects, the drug nanoparticles have a particle size distribution with a D90 of about one micron or less.

"Particle size distribution" or "PSD" or "size distribution" refers to the size of particles present in their relative proportions in a sample, as measured by laser diffraction spectroscopy. In aspects, the particle size distribution refers to the particle size distribution of all the drug particles in the composition. The particle size distribution of a sample is measured by laser diffraction spectroscopy. Laser diffraction spectrometers are commercially available as, e.g., MASTERSIZER® 3000 from Malvern Instruments Ltd, and HORIBA® LA-950 by Horiba Instruments, Inc., Irvine CA. For laser diffraction spectroscopy, particle size distribution is generally described by D values, such as D90. In aspects, the laser diffraction spectroscopy can be analyzed by the Fraunhofer theory. In aspects, the laser diffraction spectroscopy can be analyzed by the Mie scattering theory. In aspects, the laser diffraction spectroscopy can be analyzed by a combination of the Fraunhofer theory and the Mie scattering theory.

"D90" is the point in particle size distribution in which 90% of the total volume of material (e.g., drug particles) in the sample is below that specified size, as measured by laser diffraction spectroscopy. That is, D90 refers to a particle size distribution in which 90% of the total volume of material of drug particles in a sample is below a specified size. For example, if the D90 is 20 microns, this means that 90% of the materials in the sample have a particle size of 20 microns or less. "D90" is also known as Dv(90) or D₉₀. The particle size distribution of a sample is measured by laser diffraction spectroscopy. Laser diffraction spectrometers are commercially available as, e.g., MASTERSIZER® 3000 from Malvern Instruments Ltd, and HORIBA® LA-950 by Horiba Instruments, Inc., Irvine CA.

"D50" is the point in particle size distribution in which 50% of the total volume of material (e.g., drug particles) in the sample is below that specified size. That is, D50 refers to a particle size distribution in which 50% of the total volume of material of drug particles in a sample is below a specified size. For example, if the D50 is 6 microns, this means that 50% of the materials in the sample have a particle size of 6 microns or less. "D50" is also known as Dv(50) or D₅₀. The particle size distribution of a sample is measured by laser diffraction spectroscopy. Laser diffraction spectrometers are commercially available as, e.g., MASTERSIZER® 3000 from Malvern Instruments Ltd, and HORIBA® LA-950 by Horiba Instruments, Inc., Irvine CA.

"D10" is the point in particle size distribution in which 10% of the total volume of material (e.g., drug particles) in the sample is below that specified size. That is, D10 refers to a particle size distribution in which 10% of the total volume of material of drug particles in a sample is below a specified size. For example, if the D10 is 1.8 microns, this means that 10% of the materials in the sample have a particle size of 1.8 microns or less. "D10" is also known as Dv(10) or D₁₀. The particle size distribution of a sample is measured by laser diffraction spectroscopy. Laser diffraction spectrometers are commercially available as, e.g., MASTERSIZER® 3000 from Malvern Instruments Ltd, and HORIBA® LA-950 by Horiba Instruments, Inc., Irvine CA.

"Therapeutically effective amount" or "effective amount" refers to an amount sufficient to treat or prevent a disease or symptom (e.g. achieve the effect for which it is administered, treat a disease, reduce enzyme activity, increase enzyme activity, reduce protein function, reduce one or more symptoms of a disease or condition). In aspects, a therapeutically effective amount is an amount sufficient to treat cancer. The exact amounts will depend on the purpose of the treatment, whether the adenosine A2A receptor antagonists are being used as a sole therapeutic agent to treat cancer or whether they are being used in combination with other chemotherapeutic or immunomodulatory compounds. The dose of the adenosine A2A receptor antagonist can be different if it is being used as a sole therapeutic agent or if it is being used in combination therapy, and the skilled artisan will appreciate that the amount of adenosine A2A receptor antagonist may be adjusted higher or lower depending on the patient, other chemotherapeutic or immunomodulatory compounds being used, and the type of cancer being treated.

"QD" refers to administration of the compounds and compositions described herein once daily.

"BID" refers to administration of the compounds and compositions described herein twice daily (BID). In aspects, twice daily administration is after the patient wakes up and before the patient goes to bed. In aspects, twice daily administration is about every 8 hours to about every 16 hours. In aspects, twice daily administration is about every 9 hours to about every 15 hours. In aspects, twice daily administration is about every 10 hours to about every 14 hours. In aspects, twice daily administration is about every 11 hours to about every 13 hours. In aspects, twice daily administration is about every 12 hours.

"Patient" or "subject" refers to a mammal suffering from or prone to a disease (e.g., cancer) that can be treated by administration of a compound or pharmaceutical composition or by a method, as provided herein. Non-limiting examples of a patient include humans, bovines, rats, mice, dogs, cats, monkeys, goat, sheep, and the like. In aspects, a patient is human. In aspects, a patient is a dog or a cat In aspects, the patient is a human adult. In aspects, the patient is a human child.

A "month" refers to a period of time from about 28 days to about 31 days. In aspects, one month is 28 days. In aspects, one month is 29 days. In aspects, one month is 30 days. In aspects, one month is 31 days.

The term "rapidly-disintegrating" refers to a pharmaceutical composition described herein (e.g., oral formulation, amorphous solid dispersion, composition, extrudate, tablet) that can disintegrate within a short period of time. Disintegration is measured by US Pharmacopoeia (USP), Chapter <701>, Disintegration Test. In aspects, the composition is placed in a basket-rack assembly in pH 6.8, 50 mM phosphate buffer. In aspects, the composition is placed in a basket-rack assembly, in 1000 mL deionized water, at about 37°C, at 30 cycles/minute. In aspects, the short period of time is about 30 minutes or less. In aspects, the short period of time is about 20 minutes or less. In aspects, the short period of time is about 15 minutes or less. In aspects, the short period of time is about 10 minutes or less. In aspects, the short period of time is about 5 minutes or less. In aspects, the short period of time is about 2 minutes or less.

The term "rapidly-dissolving" refers to a pharmaceutical composition described herein (e.g., oral formulation, amorphous solid dispersion, composition, extrudate, tablet) that contains an adenosine A2A receptor antagonist that dissolves within a short period of time. Dissolution can be measured by US Pharmacopoeia (USP), Chapter <711>, Dissolution Test. In aspects, the composition is placed in a USP Apparatus 2 at a paddle speed of 50 rpm, in a dissolution medium in pH 6.8, 50 mM phosphate buffer. In aspects, the composition is placed in a USP Apparatus 2 at a paddle speed of 50 rpm, in a dissolution medium of 0.1 N HCl, 900 mL, at about 37°C. In aspects, a short period of time is dissolution of at least 50% of the composition in 60 minutes. In aspects, a short period of time is dissolution of at least 80% of the composition in 30 minutes. In aspects, a short period of time is dissolution of at least 50% of the composition in 20 minutes. In aspects, a short period of time is dissolution of at least 50% of the composition in 10 minutes. In aspects, a short period of time is dissolution of at least 50% of the composition in 5 minutes.

"Total impurities" refers to in the common pharmaceutical nomenclature as the total sum of all measured impurities and degradation products from the drug substance and is also referred to as total related substances. The total impurities are obtained from a stability-indicating HPLC method by integration of all peaks other than solvent or drug substance related peaks and for the extrudate or dosage forms, does not include peaks not related to the excipients. A gradient method was used with 0.1% (V/V) methanesulfonic acid in water as the first mobile phase and acetonitrile as the second. A C18 column with UV detection at 225 nm was used. Acetonitrile-water mixture was used for dilution of the samples.

"Kinetic solubility" refers to the concentration of the adenosine A2A receptor antagonist (e.g., the compound of Formula (III)) dissolved in a 50 mM phosphate buffer at a pH of 6.8, in the absence of surfactants, after rotation at 50 rpm for 4 hours. Kinetic solubility is a supersaturated or metastable and time-dependent state as opposed to true time-independent equilibrium solubility. The following procedure was used to study the kinetic solubility of the adenosine A2A receptor antagonist (Formula (III)) milled extrudate and the same crystalline drug substance (DS) as a control: Weighed about 1 g of extrudate or about 200 mg of DS into a bottle containing 120 mL of media, pH 6.8 phosphate buffer, 10 mM and 50 mM, alone or in combination with two different concentrations of sodium lauryl sulphate (SLS), 0.5% and 1.0%. The bottle was rotated at 50 rpm up to 4 hours at 37 °C. Samples were collected at 5, 10, 15, 20, 25, 30, 45, 60, 75, 90, 120, 180, and 240 minutes time points. The samples were filtered using 0.45 µm PVDF filter and diluted with acetonitrile in 1:1 ratio immediately. The samples were analyzed by HPLC. The concentration of phosphate buffer had only a minor effect on the solubility of the DS or the amorphous solid dispersion (extrudate), and the surfactant made it more difficult to distinguish the enhancement of the solubility. Consequently, pH 6.8 phosphate buffer at 50 mM without surfactant was selected as the best media for studying the kinetic solubility.

The terms "disease" or "condition" refer to a state of being or health status of a patient or subject capable of being treated with a compound, pharmaceutical composition, or method provided herein. In aspects, the disease is cancer, such as lung cancer (e.g., non-small cell lung cancer), melanoma (e.g., malignant melanoma), renal cell cancer, breast cancer (e.g., triple negative breast cancer), colorectal cancer (e.g., microsatellite instable colorectal cancer), bladder cancer, prostate cancer (e.g., metastatic castration resistant prostrate cancer, castration resistant prostrate cancer), or a head and neck cancer. In aspects, the disease is a metastatic cancer.

"Cancer" refers to all types of cancer, neoplasm or malignant tumors found in mammals, including leukemias, lymphomas, melanomas, neuroendocrine tumors, carcinomas and sarcomas. Exemplary cancers that may be treated with a compound, pharmaceutical composition, or method provided herein include lymphoma, sarcoma, bladder cancer, bone cancer, brain tumor, cervical cancer, colon cancer, esophageal cancer, gastric cancer, head and neck cancer, kidney cancer, myeloma, thyroid cancer, leukemia, prostate cancer, breast cancer (e.g. triple negative, ER positive, ER negative, chemotherapy resistant, herceptin resistant, HER2 positive, doxorubicin resistant, tamoxifen resistant, ductal carcinoma, lobular carcinoma, primary, metastatic), ovarian cancer, pancreatic cancer, liver cancer (e.g. hepatocellular carcinoma), lung cancer (e.g. non-small cell lung carcinoma, squamous cell lung carcinoma, adenocarcinoma, large cell lung carcinoma, small cell lung carcinoma, carcinoid, sarcoma), glioblastoma multiforme, glioma, melanoma, prostate cancer, castration-resistant prostate cancer, breast cancer, triple negative breast cancer, glioblastoma, ovarian cancer, lung cancer, squamous cell carcinoma (e.g., head, neck, or esophagus), colorectal cancer, leukemia, acute myeloid leukemia, lymphoma, B cell lymphoma, or multiple myeloma. Additional examples include, cancer of the thyroid, endocrine system, brain, breast, cervix, colon, head & neck, esophagus, liver, kidney, lung, non-small cell lung, melanoma, mesothelioma, ovary, sarcoma, stomach, uterus or Medulloblastoma, Hodgkin's Disease, Non-Hodgkin's Lymphoma, multiple myeloma, neuroblastoma, glioma, glioblastoma multiforme, ovarian cancer, rhabdomyosarcoma, primary thrombocytosis, primary macroglobulinemia, primary brain tumors, cancer, malignant pancreatic insulanoma, malignant carcinoid, urinary bladder cancer, premalignant skin lesions, testicular cancer, lymphomas, thyroid cancer, neuroblastoma, esophageal cancer, genitourinary tract cancer, malignant hypercalcemia, endometrial cancer, adrenal cortical cancer, neoplasms of the endocrine or exocrine pancreas, medullary thyroid cancer, medullary thyroid carcinoma, melanoma, colorectal cancer, papillary thyroid cancer, hepatocellular carcinoma, Paget's Disease of the Nipple, Phyllodes Tumors, Lobular Carcinoma, Ductal Carcinoma, cancer of the pancreatic stellate cells, cancer of the hepatic stellate cells, or prostate cancer.

As used herein, the terms "metastasis," "metastatic," "metastatic tumor," and "metastatic cancer" can be used interchangeably and refer to the spread of a proliferative disease or disorder, e.g., cancer, from one organ or another non-adjacent organ or body part. Cancer occurs at an originating site, e.g., breast, which site is referred to as a primary tumor, e.g., primary breast cancer. Some cancer cells in the primary tumor or originating site acquire the ability to penetrate and infiltrate surrounding normal tissue in the local area and/or the ability to penetrate the walls of the lymphatic system or vascular system circulating through the system to other sites and tissues in the body. A second clinically detectable tumor formed from cancer cells of a primary tumor is referred to as a metastatic or secondary tumor.

The terms "pharmaceutical composition," "composition," "formulation," "pharmaceutical composition described herein," and "composition described herein" may collectively refer to the oral formulations (e.g., tablets, granules, capsules, powders, beads), the amorphous solid dispersions, the compositions, the extrudates, the microprecipitated bulk powders, the crystalline nanoparticles, and the powders described herein.

"Adenosine A2A receptor" or "A2A receptor" refer to and include any of the recombinant or naturally-occurring forms of the adenosine A2A receptor also known as ADORA2A or isoforms or variants or homologs thereof that maintain adenosine A2A receptor activity (e.g. within at least 50%, 80%, 90%, 95%, 96%, 97%, 98%, 99% or 100% activity compared to adenosine A2A receptor). In some aspects, the isoforms, variants, or homologs have at least 90%, 95%, 96%, 97%, 98%, 99% or 100% amino acid sequence identity across the whole sequence or a portion of the sequence (*e.g*. a 50, 100, 150 or 200 continuous amino acid portion) compared to a naturally occurring adenosine A2A receptor. In aspects, the adenosine A2A receptor is substantially identical to the protein identified by the UniProt reference number P29274 or an isoform, variant, or homolog having substantial identity thereto. In aspects, the adenosine A2A receptor is substantially identical to the protein identified by the UniProt reference number Q60613 or an isoform, variant or homolog having substantial identity thereto.

"Adenosine A2A receptor antagonist" is a compound that inhibits an adenosine A2A receptor, e.g., by binding, partially or totally blocking, decreasing, preventing, delaying, inactivating, desensitizing, or down-regulating adenosine A2A receptor activity. "Inhibition," "inhibit," "inhibiting" and the like in reference to an adenosine A2A receptor interaction means negatively affecting (e.g., decreasing) the activity or function of the protein (e.g., decreasing the activity of an A2A receptor) relative to the activity or function of the protein in the absence of the inhibitor (e.g., an A2A receptor antagonist). In aspects, inhibition refers to reduction of a disease or symptoms of disease (e.g., cancer). Thus, inhibition includes, at least in part, partially or totally blocking stimulation, decreasing, preventing, or delaying activation, or inactivating, desensitizing, or down-regulating signal transduction or enzymatic activity of an adenosine A2A receptor. In aspects, the term adenosine A2A receptor antagonists includes pharmaceutically acceptable salts of adenosine A2A receptor antagonists.

In embodiments, the adenosine A2A receptor antagonist is a compound of Formula (I) or a pharmaceutically acceptable salt thereof:

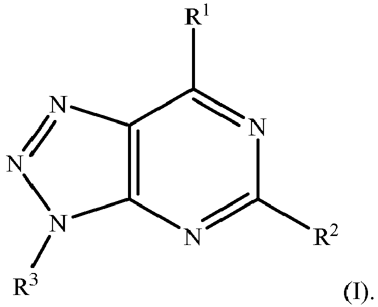

In Formula (I): R¹ is independently hydrogen, halogen, -CX^{a}₃, -CN, -SO₂Cl, -SOₙ₁R⁹, -SOᵥ₁NR⁹R¹⁰, -NHNH₂, -ONR⁹R¹⁰, -NHC=(O)NHNH₂, -NHC=(O)NR⁹R¹⁰, -N(O)ₘ₁,-NR⁹R¹⁰, -NH-O-R⁹, -C(O)R⁹, -C(O)-OR⁹, -C(0)NR⁹R¹⁰, -OR⁹, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl.

In Formula (I): R² is independently hydrogen, halogen, -CX^{b}₃, -CN, -SO₂Cl, -SOₙ₂R¹¹, -SOᵥ₂NR¹¹R¹², -NHNH₂, -ONR¹¹R¹², -NHC=(O)NHNH₂, -NHC=(O)NR¹¹R¹² -N(O)ₘ₂, -NR¹¹R¹², -NH-O-R¹¹, -C(O)R¹¹, -C(O)-OR¹¹, -C(O)NR¹¹R¹², -OR¹¹, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl.

In Formula (I): R³ is independently hydrogen, halogen, -CX^{c}₃, -CN, -SO₂Cl, -SOₙ₃R¹³, -SOᵥ₃NR¹³R¹⁴, -NHNH₂, -ONR¹³R¹⁴, -NHC=(O)NHNH₂, -NHC=(O)NR¹³R¹⁴, -N(O)ₘ₃, -NR¹³R¹⁴, -NH-O-R¹³, -C(O)R¹³, -C(O)-OR¹³, -C(O)NR¹³R¹⁴, -OR¹³, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl.

In Formula (I): R⁹, R¹⁰, R¹¹, R¹², R¹³ and R¹⁴ are independently hydrogen, halogen, =O, =S, -CF₃, -CN, -CCl₃, -COOH, -CH₂COOH, -CONH₂, -OH, -SH, -SO₂Cl,-SO₃H, -SO₄H, -SO₂NH₂, -NO₂, -NH₂, -NHNH₂, -ONH₂, -NHC=(O)NHNH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. In aspects, R⁹, R¹⁰, R¹¹, R¹², R¹³ and R¹⁴ are independently hydrogen, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl.

In Formula (I): X^{a}, X^{b} and X^{c} are independently -F, -Cl, -Br, or -I.

In Formula (I): The symbols n₁, n₂ and n₃ are independently an integer from 0 to 4. In aspects, n₁ is 0. In aspects, n₁ is 1. In aspects, n₁ is 3. In aspects, n₁ is 4. In aspects, n2 is 0. In aspects, n₂ is 1. In aspects, n₂ is 3. In aspects, n₂ is 4. In aspects, n₃ is 0. In aspects, n₃ is 1. In aspects, n₃ is 3. In aspects, n₃ is 4.

In Formula (I): The symbols m₁, m₂ and m₃ are independently an integer from 1 to 2. In aspects, m₁ is 0. In aspects, m₁ is 1. In aspects, m₁ is 2. In aspects, m₂ is 0. In aspects, m₂ is 1. In aspects, m₂ is 2. In aspects, m₃ is 0. In aspects, m₃ is 1. In aspects, m₂ is 2.

In Formula (I): The symbols v₁, v₂ and v₃ are independently an integer from 1 to 2. In aspects, v₁ is 0. In aspects, v₁ is 1. In aspects, v₁ is 2. In aspects, v₂ is 0. In aspects, v₂ is 1. In aspects, v₂ is 2. In aspects, v₃ is 0. In aspects, v₃ is 1. In aspects, v₃ is 2.

In embodiments, R¹ is independently hydrogen, halogen, -CF₃, -CN, -CCl₃, -COOH, -CH₂COOH, -CONH₂, -OH, -SH, -SO₂Cl,-SO₃H, -SO₄H, -SO₂NH₂, -NO₂, -NH₂, -NHNH₂, -ONH₂, -NHC=(O)NHNH₂, R^{1A}-substituted or unsubstituted alkyl, R^{1A}- substituted or unsubstituted heteroalkyl, R^{1A}-substituted or unsubstituted cycloalkyl, R^{1A}-substituted or unsubstituted heterocycloalkyl, R^{1A}-substituted or unsubstituted aryl, or R^{1A}-substituted or unsubstituted heteroaryl. R¹ may be R^{1A}-substituted or unsubstituted (e.g., C₁-C₂₀ or C₁-C₆) alkyl, R^{1A}- substituted or unsubstituted (e.g., 2 to 20 membered or 2 to 6 membered) heteroalkyl, R^{1A}-substituted or unsubstituted (e.g., C₃-C₈ or C₅-C₇) cycloalkyl, R^{1A}-substituted or unsubstituted (e.g., 3 to 8 membered or 3 to 6 membered) heterocycloalkyl, R^{1A}-substituted or unsubstituted (e.g., C₅-C₁₀ or C₅-C₆) aryl, or R^{1A}-substituted or unsubstituted (e.g., 5 to 10 membered or 5 to 6 membered) heteroaryl.

In embodiments, R^{1A} is independently hydrogen, halogen, =O, =S, -CF₃, -CN, -CCl₃, -COOH, -CH₂COOH, -CONH₂, -OH, -SH, -SO₂Cl,-SO₃H, -SO₄H, -SO₂NH₂, -NO₂, -NH₂, -NHNH₂, -ONH₂, -NHC=(O)NHNH₂, R^{1B}-substituted or unsubstituted alkyl, R^{1B}-substituted or unsubstituted heteroalkyl, R^{1B}-substituted or unsubstituted cycloalkyl, R^{1B}-substituted or unsubstituted heterocycloalkyl, R^{1B}-substituted or unsubstituted aryl, or R^{1B}-substituted or unsubstituted heteroaryl. R^{1A} may be R^{1B}-substituted or unsubstituted (e.g., C₁-C₂₀ or C₁-C₆) alkyl, R^{1B}- substituted or unsubstituted (e.g., 2 to 20 membered or 2 to 6 membered) heteroalkyl, R^{1B}-substituted or unsubstituted (e.g., C₃-C₈ or C₅-C₇) cycloalkyl, R^{1B}-substituted or unsubstituted (e.g., 3 to 8 membered or 3 to 6 membered) heterocycloalkyl, R^{1B}-substituted or unsubstituted (e.g., C₅-C₁₀ or C₅-C₆) aryl, or R^{1B}-substituted or unsubstituted (e.g., 5 to 10 membered or 5 to 6 membered) heteroaryl.

In embodiments, R^{1B} is independently hydrogen, halogen, =O, =S, -CF₃, -CN, -CCl₃, -COOH, -CH₂COOH, -CONH₂, -OH, -SH, -SO₂Cl,-SO₃H, -SO₄H, -SO₂NH₂, -NO₂, -NH₂, -NHNH₂, -ONH₂, -NHC=(O)NHNH₂, R^{1C}-substituted or unsubstituted alkyl, R^{1C}-substituted or unsubstituted heteroalkyl, R^{1C}-substituted or unsubstituted cycloalkyl, R^{1C}-substituted or unsubstituted heterocycloalkyl, R^{1C}-substituted or unsubstituted aryl, or R^{1C}-substituted or unsubstituted heteroaryl. R^{1B} may be R^{1C}-substituted or unsubstituted (e.g., C₁-C₂₀ or C₁-C₆) alkyl, R^{1C}-substituted or unsubstituted (e.g., 2 to 20 membered or 2 to 6 membered) heteroalkyl, R^{1C}-substituted or unsubstituted (e.g., C₃-C₈ or C₅-C₇) cycloalkyl, R^{1C}-substituted or unsubstituted (e.g., 3 to 8 membered or 3 to 6 membered) heterocycloalkyl, R^{1C}-substituted or unsubstituted (e.g., C₅-C₁₀ or C₅-C₆) aryl, or R^{1C}-substituted or unsubstituted (e.g., 5 to 10 membered or 5 to 6 membered) heteroaryl.

In embodiments, R^{1C} is independently hydrogen, halogen, =O, =S, -CF₃, -CN, -CCl₃, -COOH, -CH₂COOH, -CONH₂, -OH, -SH, -SO₂Cl,-SO₃H, -SO₄H, -SO₂NH₂, -NO₂, -NH₂, -NHNH₂, -ONH₂, -NHC=(O)NHNH₂, unsubstituted alkyl, unsubstituted heteroalkyl, unsubstituted cycloalkyl, unsubstituted heterocycloalkyl, unsubstituted aryl, or unsubstituted heteroaryl. R^{1C} may be independently unsubstituted (e.g., C₁-C₂₀ or C₁-C₆) alkyl, unsubstituted (e.g., 2 to 20 membered or 2 to 6 membered) heteroalkyl, unsubstituted (e.g., C₃-C₈ or C₅-C₇) cycloalkyl, unsubstituted (e.g., 3 to 8 membered or 3 to 6 membered) heterocycloalkyl, unsubstituted (e.g., C₅-C₁₀ or C₅-C₆) aryl, or unsubstituted (e.g., 5 to 10 membered or 5 to 6 membered) heteroaryl.

In embodiments, R¹ is independently R^{1A}-substituted or unsubstituted alkyl, R^{1A}-substituted or unsubstituted heteroalkyl, R^{1A}-substituted or unsubstituted cycloalkyl, R^{1A}-substituted or unsubstituted heterocycloalkyl, R^{1A}-substituted or unsubstituted aryl, or s R^{1A}-substituted or unsubstituted heteroaryl. In aspects, R¹ is R^{1A}-substituted or unsubstituted (e.g., 5 to 10 membered or 5 to 6 membered) heteroaryl. In aspects, R¹ is unsubstituted 5 to 6 membered heteroaryl. In aspects, R¹ is R^{1A}-substituted 5 to 6 membered heteroaryl. In aspects, R¹ is unsubstituted 5 membered heteroaryl. In aspects, R¹ is R^{1A}-substituted 5 membered heteroaryl. In aspects, R¹ is R^{1A}-substituted furanyl.

In embodiments, R^{1A} is R^{1B}-substituted or unsubstituted (e.g., C₁-C₂₀ or C₁-C₆) alkyl. In aspects, R^{1A} is R^{1B}-substituted C₁-C₆ alkyl. In aspects, R^{1A} is unsubstituted C₁-C₆ alkyl. In aspects, R^{1A} is R^{1B}-substituted C₁-C₄ alkyl. In aspects, R^{1A} is unsubstituted C₁-C₄ alkyl. In aspects, R^{1A} is R^{1B}-substituted C₁-C₃ alkyl. In aspects, R^{1A} is unsubstituted C₁-C₃ alkyl. In aspects, R^{1A} is methyl.

In embodiments, R² is independently hydrogen, halogen, -CX^{b}₃, -CN, -SO₂Cl, -SOₙ₂R¹¹, -SOᵥ₂NR¹¹R¹², -NHNH₂, -ONR¹¹R¹², -NHC=(O)NHNH₂, -NHC=(O)NR¹¹R¹², -N(O)ₘ₂, -NR¹¹R¹², -NH-O-R¹¹, -C(O)R¹¹, -C(O)-OR¹¹, -C(O)NR¹¹R¹², or -OR¹¹. In aspects of the methods provided herein, R² is independently hydrogen, halogen, -CF₃, -CN, -CCl₃, -COOH, -CH₂COOH, -CONH₂, -OH,-SH, -SO₂Cl,-SO₃H, -SO₄H, -SO₂NH₂, -NO₂, -NH₂, -NHNH₂, -ONH₂, -NHC=(O)NHNH₂, unsubstituted alkyl, unsubstituted heteroalkyl, unsubstituted cycloalkyl, unsubstituted heterocycloalkyl, unsubstituted aryl, or unsubstituted heteroaryl. In aspects, R² is -NR¹¹R¹². In aspects, R¹¹ and R¹² are independently hydrogen or substituted or unsubstituted (e.g., C₁-C₂₀ or C₁-C₆) alkyl. In aspects, R¹¹ and R¹² are independently substituted or unsubstituted C₁-C₆ alkyl. In aspects, R¹¹ and R¹² are independently substituted or unsubstituted C₁-C₄ alkyl. In aspects, R¹¹ and R¹² are independently substituted or unsubstituted C₁-C₃ alkyl. In aspects, R¹¹ and R¹² are independently unsubstituted C₁-C₆ alkyl. In aspects, R¹¹ and R¹² are independently substituted or unsubstituted C₁-C₄ alkyl. In aspects, R¹¹ and R¹² are independently unsubstituted C₁-C₃ alkyl. In aspects, R¹¹ and R¹² are independently hydrogen.

In embodiments, R³ is independently hydrogen, halogen, -CF₃, -CN, -CCl₃, -COOH, -CH₂COOH, -CONH₂, -OH, -SH, -SO₂Cl,-SO₃H, -SO₄H, -SO₂NH₂, -NO₂, -NH₂, -NHNH₂, -ONH₂, -NHC=(O)NHNH₂, R⁴-substituted or unsubstituted alkyl, R⁴- substituted or unsubstituted heteroalkyl, R⁴-substituted or unsubstituted cycloalkyl, R⁴-substituted or unsubstituted heterocycloalkyl, R⁴-substituted or unsubstituted aryl, or R⁴-substituted or unsubstituted heteroaryl. R³ may be R⁴-substituted or unsubstituted (e.g., C₁-C₂₀ or C₁-C₆) alkyl, R⁴- substituted or unsubstituted (e.g., 2 to 20 membered or 2 to 6 membered) heteroalkyl, R⁴-substituted or unsubstituted (e.g., C₃-C₈ or C₅-C₇) cycloalkyl, R⁴-substituted or unsubstituted (e.g., 3 to 8 membered or 3 to 6 membered) heterocycloalkyl, R⁴-substituted or unsubstituted (e.g., C₅-C₁₀ or C₅-C₆) aryl, or R⁴-substituted or unsubstituted (e.g., 5 to 10 membered or 5 to 6 membered) heteroaryl.

In embodiments, R⁴ is independently hydrogen, halogen, =O, =S, -CF₃, -CN, -CCl₃, -COOH, -CH₂COOH, -CONH₂, -OH, -SH, -SO₂Cl,-SO₃H, -SO₄H, -SO₂NH₂, -NO₂, -NH₂, -NHNH₂, -ONH₂, -NHC=(O)NHNH₂, R⁵-substituted or unsubstituted alkyl, R⁵- substituted or unsubstituted heteroalkyl, R⁵-substituted or unsubstituted cycloalkyl, R⁵-substituted or unsubstituted heterocycloalkyl, R⁵-substituted or unsubstituted aryl, or R⁵-substituted or unsubstituted heteroaryl. R⁴ may be R⁵-substituted or unsubstituted (e.g., C₁-C₂₀ or C₁-C₆) alkyl, R⁵- substituted or unsubstituted (e.g., 2 to 20 membered or 2 to 6 membered) heteroalkyl, R⁵-substituted or unsubstituted(e.g., C₃-C₈ or C₅-C₇) cycloalkyl, R⁵-substituted or unsubstituted (e.g., 3 to 8 membered or 3 to 6 membered) heterocycloalkyl, R⁵-substituted or unsubstituted (e.g., C₅-C₁₀ or C₅-C₆) aryl, or R⁵-substituted or unsubstituted (e.g., 5 to 10 membered or 5 to 6 membered) heteroaryl.

In embodiments, R⁵ is independently hydrogen, halogen, =O, =S, -CF₃, -CN, -CCl₃, -COOH, -CH₂COOH, -CONH₂, -OH, -SH, -SO₂Cl,-SO₃H, -SO₄H, -SO₂NH₂, -NO₂, -NH₂, -NHNH₂, -ONH₂, -NHC=(O)NHNH₂, R⁶-substituted or unsubstituted alkyl, R⁶- substituted or unsubstituted heteroalkyl, R⁶-substituted or unsubstituted cycloalkyl, R⁶-substituted or unsubstituted heterocycloalkyl, R⁶-substituted or unsubstituted aryl, or R⁶-substituted or unsubstituted heteroaryl. R⁵ may be R⁶-substituted or unsubstituted (e.g., C₁-C₂₀ or C₁-C₆) alkyl, R⁶- substituted or unsubstituted (e.g., 2 to 20 membered or 2 to 6 membered) heteroalkyl, R⁶-substituted or unsubstituted (e.g., C₃-C₈ or C₅-C₇) cycloalkyl, R⁶-substituted or unsubstituted (e.g., 3 to 8 membered or 3 to 6 membered) heterocycloalkyl, R⁶-substituted or unsubstituted (e.g., C₅-C₁₀ or C₅-C₆) aryl, or R⁶-substituted or unsubstituted (e.g., 5 to 10 membered or 5 to 6 membered) heteroaryl.

In embodiments, R⁶ is independently hydrogen, halogen, =O, =S, -CF₃, -CN, -CCl₃, -COOH, -CH₂COOH, -CONH₂, -OH, -SH, -SO₂Cl,-SO₃H, -SO₄H, -SO₂NH₂, -NO₂, -NH₂, -NHNH₂, -ONH₂, -NHC=(O)NHNH₂, R⁷-substituted or unsubstituted alkyl, R⁷- substituted or unsubstituted heteroalkyl, R⁷-substituted or unsubstituted cycloalkyl, R⁷-substituted or unsubstituted heterocycloalkyl, R⁷-substituted or unsubstituted aryl, or R⁷-substituted or unsubstituted heteroaryl. R⁶ may be R⁷-substituted or unsubstituted (e.g., C₁-C₂₀ or C₁-C₆) alkyl, R⁷- substituted or unsubstituted (e.g., 2 to 20 membered or 2 to 6 membered) heteroalkyl, R⁷-substituted or unsubstituted (e.g., C₃-C₈ or C₅-C₇) cycloalkyl, R⁷-substituted or unsubstituted (e.g., 3 to 8 membered or 3 to 6 membered) heterocycloalkyl, R⁷-substituted or unsubstituted (e.g., C₅-C₁₀ or C₅-C₆) aryl, or R⁷-substituted or unsubstituted (e.g., 5 to 10 membered or 5 to 6 membered) heteroaryl.

In embodiments, R³ is independently hydrogen, halogen, R⁴-substituted or unsubstituted alkyl, R⁴- substituted or unsubstituted heteroalkyl, R⁴-substituted or unsubstituted cycloalkyl, R⁴-substituted or unsubstituted heterocycloalkyl, R⁴-substituted or unsubstituted aryl, or R⁴-substituted or unsubstituted heteroaryl. In aspects, R³ is independently R⁴-substituted or unsubstituted (e.g., C₁-C₂₀ or C₁-C₆) alkyl. In aspects, R³ is independently R⁴-substituted or unsubstituted C₁-C₆ alkyl. In aspects, R³ is independently R⁴-substituted or unsubstituted C₁-C₅ alkyl. In aspects, R³ is independently R⁴-substituted or unsubstituted C₁-C₄ alkyl. In aspects, R³ is independently R⁴-substituted or unsubstituted C₁-C₃ alkyl. In aspects, R³ is independently unsubstituted C₁-C₆ alkyl. In aspects, R³ is independently unsubstituted C₁-C₅ alkyl. In aspects, R³ is independently R⁴- unsubstituted C₁-C₄ alkyl. In aspects, R³ is independently unsubstituted C₁-C₃ alkyl. In aspects, R³ is independently R⁴-substituted C₁-C₆ alkyl. In aspects, R³ is independently R⁴-substituted C₁-C₅ alkyl. In aspects, R³ is independently R⁴-substituted C₁-C₄ alkyl. In aspects, R³ is independently R⁴-substituted C₁-C₃ alkyl. In aspects, R³ is R⁴-substituted C₁ alkyl.

In embodiments, R⁴ is R⁵-substituted or unsubstituted (e.g., C₁-C₂₀ or C₁-C₆) alkyl, R⁵- substituted or unsubstituted (e.g., 2 to 20 membered or 2 to 6 membered) heteroalkyl, R⁵-substituted or unsubstituted(e.g., C₃-C₈ or C₅-C₇) cycloalkyl, R⁵-substituted or unsubstituted (e.g., 3 to 8 membered or 3 to 6 membered) heterocycloalkyl, R⁵-substituted or unsubstituted (e.g., C₅-C₁₀ or C₅-C₆) aryl, or R⁵-substituted or unsubstituted (e.g., 5 to 10 membered or 5 to 6 membered) heteroaryl. In aspects, R⁴ is R⁵-substituted or unsubstituted 5 to 6 membered heteroaryl. In aspects, R⁴ is R⁵-substituted or unsubstituted 6 membered heteroaryl. In aspects, R⁴ is unsubstituted 6 membered heteroaryl. In aspects, R⁴ is R⁵-substituted 6 membered heteroaryl. In aspects, R⁴ is R⁵-substituted pyridinyl.

In embodiments, R⁵ is R⁶-substituted or unsubstituted (e.g., C₁-C₂₀ or C₁-C₆) alkyl, R⁶- substituted or unsubstituted (e.g., 2 to 20 membered or 2 to 6 membered) heteroalkyl, R⁶-substituted or unsubstituted (e.g., C₃-C₈ or C₅-C₇) cycloalkyl, R⁶-substituted or unsubstituted (e.g., 3 to 8 membered or 3 to 6 membered) heterocycloalkyl, R⁶-substituted or unsubstituted (e.g., C₅-C₁₀ or C₅-C₆) aryl, or R⁶-substituted or unsubstituted (e.g., 5 to 10 membered or 5 to 6 membered) heteroaryl. In aspects, R⁵ is R⁶-substituted or unsubstituted 2 to 6 membered heteroalkyl. In aspects, R⁵ is R⁶-substituted or unsubstituted 2 to 5 membered heteroalkyl. In aspects, R⁵ is R⁶-substituted or unsubstituted 2 to 4 membered heteroalkyl. In aspects, R⁵ is R⁶-substituted or unsubstituted 2 to 3 membered heteroalkyl. In aspects, R⁵ is R⁶-substituted or unsubstituted 2 membered heteroalkyl. In aspects, R⁵ is unsubstituted 2 to 6 membered heteroalkyl. In aspects, R⁵ is unsubstituted 2 to 5 membered heteroalkyl. In aspects, R⁵ is unsubstituted 2 to 4 membered heteroalkyl. In aspects, R⁵ unsubstituted 2 to 3 membered heteroalkyl. In aspects, R⁵ is unsubstituted 2 membered heteroalkyl. In aspects, R⁵ is R⁶-substituted 2 to 6 membered heteroalkyl. In aspects, R⁵ is R⁶-substituted 2 to 5 membered heteroalkyl. In aspects, R⁵ is R⁶-substituted 2 to 4 membered heteroalkyl. In aspects, R⁵ is R⁶-substituted 2 to 3 membered heteroalkyl. In aspects, R⁵ is R⁶-substituted 2 membered heteroalkyl.

In embodiments, R⁶ is R⁷-substituted or unsubstituted (e.g., C₁-C₂₀ or C₁-C₆) alkyl, R⁷- substituted or unsubstituted (e.g., 2 to 20 membered or 2 to 6 membered) heteroalkyl, R⁷-substituted or unsubstituted (e.g., C₃-C₈ or C₅-C₇) cycloalkyl, R⁷-substituted or unsubstituted (e.g., 3 to 8 membered or 3 to 6 membered) heterocycloalkyl, R⁷-substituted or unsubstituted (e.g., C₅-C₁₀ or C₅-C₆) aryl, or R⁷-substituted or unsubstituted (e.g., 5 to 10 membered or 5 to 6 membered) heteroaryl. In aspects, R⁶ is R⁷-substituted or unsubstituted 3 to 6 membered heterocycloalkyl. In aspects, R⁶ is R⁷-substituted or unsubstituted 5 membered heterocycloalkyl. In aspects, R⁶ is R⁷-substituted 5 membered heterocycloalkyl. In aspects, R⁶ is unsubstituted 5 membered heterocycloalkyl. In aspects, R⁶ is unsubstituted tetrahydrofuranyl.

In embodiments of the methods provided herein, R⁹, R¹⁰, R¹¹, R¹², R¹³ and R¹⁴ are independently hydrogen, halogen, =O, =S, -CF₃, -CN, -CCl₃, -COOH, -CH₂COOH, -CONH₂, -OH, -SH, -SO₂Cl,-SO₃H, -SO₄H, -SO₂NH₂, -NO₂, -NH₂, -NHNH₂, -ONH₂, -NHC=(O)NHNH₂, unsubstituted alkyl, unsubstituted heteroalkyl, unsubstituted cycloalkyl, unsubstituted heterocycloalkyl, unsubstituted aryl, or unsubstituted heteroaryl.

In embodiments, R¹ is R^{1A}-substituted furanyl. In aspects, R^{1A} is methyl. In aspects, R² is -NR¹¹R¹². In aspects, R¹¹ and R¹² are independently hydrogen. In aspects, R³ is R⁴-substituted C₁ alkyl. In aspects, R⁴ is R⁵-substituted pyridinyl. In aspects, R⁵ is R⁶-substituted 2 membered heteroalkyl. In aspects, R⁶ is unsubstituted tetrahydrofuranyl.

In embodiments, the adenosine A2A receptor antagonist is a compound of Formula (II) or a pharmaceutically acceptable salt thereof:

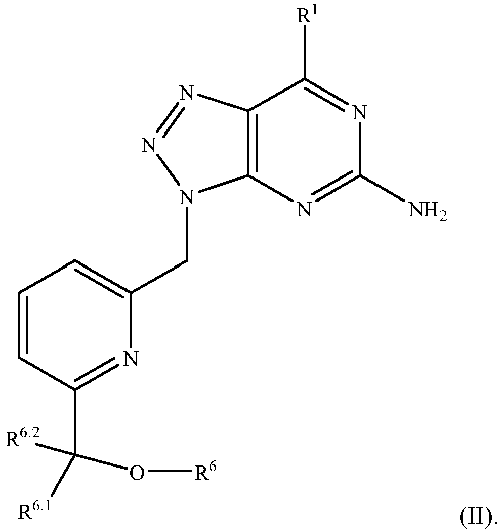

In Formula (II): R⁶, R^{6.1} and R^{6.2} are independently hydrogen, halogen, -CF₃, -CN, -CCl₃, -COOH, -CH₂COOH, -CONH₂, -OH, -SH, -SO₂Cl,-SO₃H, -SO₄H, -SO₂NH₂, -NO₂, -NH₂, -NHNH₂, -ONH₂, -NHC=(O)NHNH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. In aspects, R⁶, R^{6.1} and R^{6.2} are independently hydrogen, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. In aspects, R^{6.1} and R^{6.2} are hydrogen and R⁶ is a substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. In aspects, R^{6.1} and R^{6.2} are hydrogen and R6 is substituted or unsubstituted heterocycloalkyl. In aspects, R^{6.1} and R^{6.2} are hydrogen and R6 is unsubstituted heterocycloalkyl. In aspects, R¹ is substituted (e.g. with an unsubstituted C₁-C₅ alkyl) or unsubstituted heteroaryl. In aspects, R¹ is substituted (e.g. with an unsubstituted C₁-C₅ alkyl) or unsubstituted furanyl. In aspects, R¹ is methyl-substituted furanyl.

In Formula (II): R¹ and R⁶ are as described above (e.g., R⁶ may be R⁷-substituted or unsubstituted 3 to 6 membered heterocycloalkyl and R¹ may be R^{1A}-substituted 5 to 6 membered heteroaryl). Thus, in aspects, R⁶ is unsubstituted tetrahydrofuranyl and R¹ is R^{1A}-substituted furanyl.

In Formula (II): R^{6.1} may be independently hydrogen, halogen, -CF₃, -CN, -CCl₃, -COOH, -CH₂COOH, -CONH₂, -OH, -SH, -SO₂Cl,-SO₃H, -SO₄H, -SO₂NH₂, -NO₂, -NH₂, -NHNH₂, -ONH₂, -NHC=(O)NHNH₂, R^{7.1}-substituted or unsubstituted alkyl, R^{7.1}- substituted or unsubstituted heteroalkyl, R^{7.1}-substituted or unsubstituted cycloalkyl, R^{7.1}-substituted or unsubstituted heterocycloalkyl, R^{7.1}-substituted or unsubstituted aryl, or R^{7.1}-substituted or unsubstituted heteroaryl. R^{6.1} may be R^{7.1}-substituted or unsubstituted (e.g., C₁-C₂₀ or C₁-C₆) alkyl, R^{7.1}-substituted or unsubstituted (e.g., 2 to 20 membered or 2 to 6 membered) heteroalkyl, R^{7.1}-substituted or unsubstituted (e.g., C₃-C₈ or C₅-C₇) cycloalkyl, R^{7.1}-substituted or unsubstituted (e.g., 3 to 8 membered or 3 to 6 membered) heterocycloalkyl, R^{7.1}-substituted or unsubstituted (e.g., C₅-C₁₀ or C₅-C₆) aryl, or R^{7.1}-substituted or unsubstituted (e.g., 5 to 10 membered or 5 to 6 membered) heteroaryl. In aspects, R^{6.1} is R^{7.1}-substituted or unsubstituted C₁-C₆ alkyl. In aspects, R^{6.1} is R^{7.1}-substituted or unsubstituted C₁-C₅ alkyl. In aspects, R^{6.1} is R^{7.1}-substituted or unsubstituted C₁-C₄ alkyl. In aspects, R^{6.1} is R^{7.1}-substituted or unsubstituted C₁-C₃ alkyl. In aspects, R^{6.1} is R^{7.1}-substituted C₁-C₆ alkyl. In aspects, R^{6.1} is R^{7.1}-substituted C₁-C₅ alkyl. In aspects, R^{6.1} is R^{7.1}-substituted C₁-C₄ alkyl. In aspects, R^{6.1} is R^{7.1}-substituted C₁-C₃ alkyl. In aspects, R^{6.1} is unsubstituted C₁-C₆ alkyl. In aspects, R^{6.1} is unsubstituted C₁-C₅ alkyl. In aspects, R^{6.1} is unsubstituted C₁-C₄ alkyl. In aspects, R^{6.1} is unsubstituted C₁-C₃ alkyl. In aspects, R^{6.1} is unsubstituted methyl.

In Formula (II): R^{6.2} is independently hydrogen, halogen, =O, -CF₃, -CN, -CCl₃, -COOH, -CH₂COOH, -CONH₂, -OH, -SH, -SO₂Cl,-SO₃H, -SO₄H, -SO₂NH₂, -NO₂, -NH₂, -NHNH₂, -ONH₂, -NHC=(O)NHNH₂, R^{7.2}-substituted or unsubstituted alkyl, R^{7.2}- substituted or unsubstituted heteroalkyl, R^{7.2}-substituted or unsubstituted cycloalkyl, R^{7.2}-substituted or unsubstituted heterocycloalkyl, R^{7.2}-substituted or unsubstituted aryl, or R⁷-substituted or unsubstituted heteroaryl. R^{6.2} may be R^{7.2}-substituted or unsubstituted (e.g., C₁-C₂₀ or C₁-C₆) alkyl, R^{7.2}-substituted or unsubstituted (e.g., 2 to 20 membered or 2 to 6 membered) heteroalkyl, R^{7.2}-substituted or unsubstituted (e.g., C₃-C₈ or C₅-C₇) cycloalkyl, R^{7.2} -substituted or unsubstituted (e.g., 3 to 8 membered or 3 to 6 membered) heterocycloalkyl, R^{7.2}-substituted or unsubstituted (e.g., C₅-C₁₀ or C₅-C₆) aryl, or R^{7.2}-substituted or unsubstituted (e.g., 5 to 10 membered or 5 to 6 membered) heteroaryl. In aspects, R^{6.2} is R^{7.2}-substituted or unsubstituted C₁-C₆ alkyl. In aspects, R^{6.2} is R^{7.2}-substituted or unsubstituted C₁-C₅ alkyl. In aspects, R^{6.2} is R^{7.2}-substituted or unsubstituted C₁-C₄ alkyl. In aspects, R^{6.2} is R^{7.2}-substituted or unsubstituted C₁-C₃ alkyl. In aspects, R^{6.2} is R^{7.2}-substituted C₁-C₆ alkyl. In aspects, R^{6.2} is R^{7.2}-substituted C₁-C₅ alkyl. In aspects, R^{6.2} is R^{7.2}-substituted C₁-C₄ alkyl. In aspects, R^{6.2} is R^{7.2}-substituted C₁-C₃ alkyl. In aspects, R^{6.2} is unsubstituted C₁-C₆ alkyl. In aspects, R^{6.2} is unsubstituted C₁-C₅ alkyl. In aspects, R^{6.2} is unsubstituted C₁-C₄ alkyl. In aspects, R^{6.2} is unsubstituted C₁-C₃ alkyl. In aspects, R^{6.2} is unsubstituted methyl.

In Formula (II): R⁷, R^{7.1} and R^{7.2} are independently hydrogen, halogen, =O, =S, -CF₃, -CN, -CCl₃, -COOH, -CH₂COOH, -CONH₂, -OH, -SH, -SO₂Cl,-SO₃H, -SO₄H, -SO₂NH₂, -NO₂, -NH₂, -NHNH₂, -ONH₂, -NHC=(O)NHNH₂, unsubstituted alkyl, unsubstituted heteroalkyl, unsubstituted cycloalkyl, unsubstituted heterocycloalkyl, unsubstituted aryl, or unsubstituted heteroaryl. R⁷, R^{7.1} and R^{7.2} may be independently unsubstituted (e.g., C₁-C₂₀ or C₁-C₆) alkyl, unsubstituted (e.g., 2 to 20 membered or 2 to 6 membered) heteroalkyl, unsubstituted (e.g., C₃-C₈ or C₅-C₇) cycloalkyl, unsubstituted (e.g., 3 to 8 membered or 3 to 6 membered) heterocycloalkyl, unsubstituted (e.g., C₅-C₁₀ or C₅-C₆) aryl, or unsubstituted (e.g., 5 to 10 membered or 5 to 6 membered) heteroaryl.

In embodiments, the A2A receptor antagonist is a compound of Formula (III) or a pharmaceutically acceptable salt thereof:

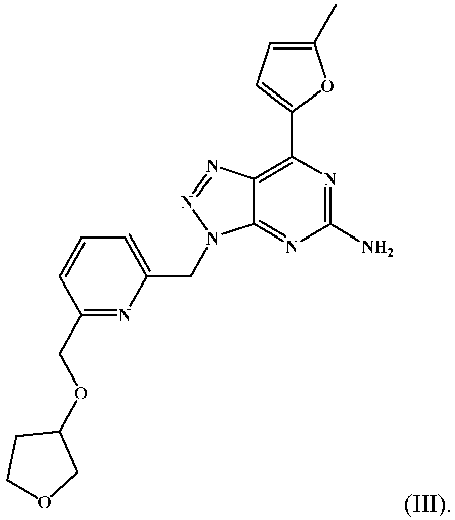

The "compound of Formula (III)" may also be referred to herein as "Formula (III)" or as CPI-444. In aspects, the compound of Formula (III) is a compound of Formula (IIIA). In aspects, the compound of Formula (III) is a compound of Formula (IIIB). In aspects, the compound of Formula (III) is a mixture of the compounds of Formula (IIIA) and (IIIB). In aspects, the compound of Formula (III) is a racemic mixture of the compounds of Formula (IIIA) and (IIIB).

In embodiments, the compound of Formula (III) is the compound of Formula (IIIA) or a pharmaceutically acceptable salt thereof:

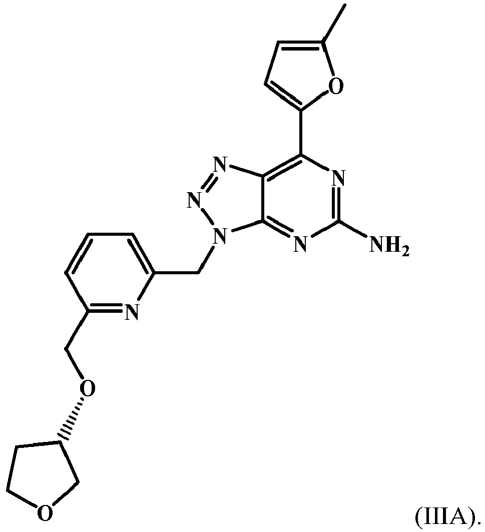

In embodiments, the compound of Formula (III) is the compound of Formula (IIIB) or a pharmaceutically acceptable salt thereof:

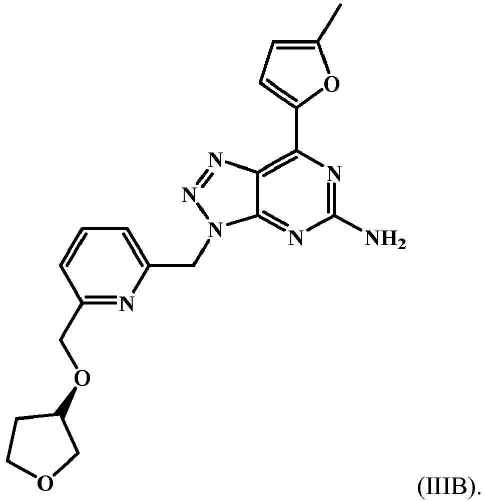

Methods for making adenosine A2A receptor antagonists, including those of Formula (I), Formula (II), Formula (III), Formula (IIIA), and Formula (IIIB), are known in the art and described, for example, in WO 2017/112917, US Patent No. 8,450,328, US Patent No. 8,987,279, US Patent No. 9,376,443, and US Patent No. 9,765,080, the disclosures of which are incorporated by reference herein in their entirety.

The abbreviations used herein have their conventional meaning within the chemical and biological arts. The chemical structures and formulae set forth herein are constructed according to the standard rules of chemical valency known in the chemical arts. Where substituent groups are specified by their conventional chemical formulae, written from left to right, they equally encompass the chemically identical substituents that would result from writing the structure from right to left, e.g., -CH₂O- is equivalent to -OCH₂-.

"Alkyl," by itself or as part of another substituent, means a straight (i.e., unbranched) or branched non-cyclic carbon chain (or carbon), or combination thereof, which may be fully saturated, mono- or polyunsaturated and can include di- and multivalent radicals, having the number of carbon atoms designated (i.e., C₁-C₁₀ means one to ten carbons). Examples of saturated hydrocarbon radicals include, but are not limited to, groups such as methyl, ethyl, n-propyl, isopropyl, n-butyl, t-butyl, isobutyl, sec-butyl, (cyclohexyl)methyl, homologs and isomers of, for example, n-pentyl, n-hexyl, n-heptyl, n-octyl, and the like. An unsaturated alkyl group is one having one or more double bonds or triple bonds. Examples of unsaturated alkyl groups include, but are not limited to, vinyl, 2-propenyl, crotyl, 2-isopentenyl, 2-(butadienyl), 2,4-pentadienyl, 3-(1,4-pentadienyl), ethynyl, 1- and 3-propynyl, 3-butynyl, and the higher homologs and isomers. An alkoxy is an alkyl attached to the remainder of the molecule via an oxygen linker (-O-). An alkyl moiety may be an alkenyl moiety. An alkyl moiety may be an alkynyl moiety. An alkyl moiety may be fully saturated. An alkenyl may include more than one double bond and/or one or more triple bonds in addition to the one or more double bonds. An alkynyl may include more than one triple bond and/or one or more double bonds in addition to the one or more triple bonds.

"Alkylene," by itself or as part of another substituent, means a divalent radical derived from an alkyl, as exemplified, but not limited by, -CH₂CH₂CH₂CH₂-. Typically, an alkyl (or alkylene) group will have from 1 to 24 carbon atoms, with those groups having 10 or fewer carbon atoms being preferred, and with 6 or fewer carbon atoms or 4 or few carbon atoms more preferred. A "lower alkyl" or "lower alkylene" is a shorter chain alkyl or alkylene group, generally having eight or fewer carbon atoms. The term "alkenylene," by itself or as part of another substituent, means, unless otherwise stated, a divalent radical derived from an alkene.

"Heteroalkyl," by itself or in combination with another term, means a stable straight or branched non-cyclic chain, or combinations thereof, including at least one carbon atom and at least one heteroatom (e.g. O, N, P, Si, and S), and wherein the nitrogen and sulfur atoms may optionally be oxidized, and the nitrogen heteroatom may optionally be quaternized. The heteroatom(s) (e.g. O, N, P, S, and Si) may be placed at any interior position of the heteroalkyl group or at the position at which the alkyl group is attached to the remainder of the molecule. Examples include, but are not limited to: -CH₂-CH₂-O-CH₃, -CH₂-CH₂-NH-CH₃, -CH₂-CH₂-N(CH₃)-CH₃, -CH₂-S-CH₂-CH₃,-CH₂-CH₂, -S(O)-CH₃, -CH₂-CH₂-S(O)₂-CH₃, -CH=CH-O-CH₃, -Si(CH₃)₃, -CH₂-CH=N-OCH₃, -CH=CH-N(CH₃)-CH₃, -O-CH₃, -O-CH₂-CH₃, and -CN. Up to two or three heteroatoms may be consecutive, such as, for example, -CH₂-NH-OCH₃ and -CH₂-O-Si(CH₃)₃. A heteroalkyl moiety may include one heteroatom (e.g., O, N, S, Si, or P). A heteroalkyl moiety may include two optionally different heteroatoms (e.g., O, N, S, Si, or P). A heteroalkyl moiety may include three optionally different heteroatoms (e.g., O, N, S, Si, or P). A heteroalkyl moiety may include four optionally different heteroatoms (e.g., O, N, S, Si, or P). A heteroalkyl moiety may include five optionally different heteroatoms (e.g., O, N, S, Si, or P). A heteroalkyl moiety may include up to 8 optionally different heteroatoms (e.g., O, N, S, Si, or P). The term "heteroalkenyl," by itself or in combination with another term, means, unless otherwise stated, a heteroalkyl including at least one double bond. A heteroalkenyl may optionally include more than one double bond and/or one or more triple bonds in additional to the one or more double bonds. The term "heteroalkynyl," by itself or in combination with another term, means, unless otherwise stated, a heteroalkyl including at least one triple bond. A heteroalkynyl may optionally include more than one triple bond and/or one or more double bonds in additional to the one or more triple bonds.

"Heteroalkylene," by itself or as part of another substituent, means a divalent radical derived from heteroalkyl, as exemplified, but not limited by, -CH₂-CH₂-S-CH₂-CH₂- and -CH₂-S-CH₂-CH₂-NH-CH₂-. For heteroalkylene groups, heteroatoms can also occupy either or both of the chain termini (e.g., alkyleneoxy, alkylenedioxy, alkyleneamino, alkylenediamino, and the like). Still further, for alkylene and heteroalkylene linking groups, no orientation of the linking group is implied by the direction in which the formula of the linking group is written. For example, the formula -C(O)₂R'- represents both -C(O)₂R'- and -R'C(O)₂-. As described above, heteroalkyl groups, as used herein, include those groups that are attached to the remainder of the molecule through a heteroatom, such as -C(O)R', -C(O)NR', -NR'R", -OR', -SR', and/or -SO₂R'. Where "heteroalkyl" is recited, followed by recitations of specific heteroalkyl groups, such as -NR'R" or the like, it will be understood that the terms heteroalkyl and -NR'R" are not redundant or mutually exclusive. Rather, the specific heteroalkyl groups are recited to add clarity. Thus, the term "heteroalkyl" should not be interpreted herein as excluding specific heteroalkyl groups, such as -NR'R" or the like.

"Cycloalkyl" and "heterocycloalkyl," by themselves or in combination with other terms, mean non-aromatic cyclic versions of "alkyl" and "heteroalkyl," respectively, wherein the carbons making up the ring or rings do not necessarily need to be bonded to a hydrogen due to all carbon valencies participating in bonds with non-hydrogen atoms. Additionally, for heterocycloalkyl, a heteroatom can occupy the position at which the heterocycle is attached to the remainder of the molecule. Examples of cycloalkyl include, but are not limited to, cyclopropyl, cyclobutyl, cyclopentyl, cyclohexyl, 1-cyclohexenyl, 3-cyclohexenyl, cycloheptyl, 3-hydroxy-cyclobut-3-enyl-1,2, dione, 1H-1,2,4-triazolyl-5(4H)-one, 4H-1,2,4-triazolyl, and the like. Examples of heterocycloalkyl include, but are not limited to, 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1-piperazinyl, 2-piperazinyl, and the like. A "cycloalkylene" and a "heterocycloalkylene," alone or as part of another substituent, means a divalent radical derived from a cycloalkyl and heterocycloalkyl, respectively. A heterocycloalkyl moiety may include one ring heteroatom (e.g., O, N, S, Si, or P). A heterocycloalkyl moiety may include two optionally different ring heteroatoms (e.g., O, N, S, Si, or P). A heterocycloalkyl moiety may include three optionally different ring heteroatoms (e.g., O, N, S, Si, or P). A heterocycloalkyl moiety may include four optionally different ring heteroatoms (e.g., O, N, S, Si, or P). A heterocycloalkyl moiety may include five optionally different ring heteroatoms (e.g., O, N, S, Si, or P). A heterocycloalkyl moiety may include up to 8 optionally different ring heteroatoms (e.g., O, N, S, Si, or P).

"Halo" or "halogen," by themselves or as part of another substituent, mean a fluorine, chlorine, bromine, or iodine atom. Additionally, terms such as "haloalkyl" are meant to include monohaloalkyl and polyhaloalkyl. For example, the term "halo(C₁-C₄)alkyl" includes, but is not limited to, fluoromethyl, difluoromethyl, trifluoromethyl, 2,2,2-trifluoroethyl, 4-chlorobutyl, 3-bromopropyl, and the like.

"Acyl" means, unless otherwise stated, -C(O)R where R is a substituted or unsubstituted alkyl, substituted or unsubstituted cycloalkyl, substituted or unsubstituted heteroalkyl, substituted or unsubstituted heterocycloalkyl, substituted or unsubstituted aryl, or substituted or unsubstituted heteroaryl.

"Aryl" means a polyunsaturated, aromatic, hydrocarbon substituent, which can be a single ring or multiple rings (preferably from 1 to 3 rings) that are fused together (i.e., a fused ring aryl) or linked covalently. A fused ring aryl refers to multiple rings fused together wherein at least one of the fused rings is an aryl ring. The term "heteroaryl" refers to aryl groups (or rings) that contain at least one heteroatom such as N, O, or S, wherein the nitrogen and sulfur atoms are optionally oxidized, and the nitrogen atom(s) are optionally quaternized. Thus, the term "heteroaryl" includes fused ring heteroaryl groups (i.e., multiple rings fused together wherein at least one of the fused rings is a heteroaromatic ring). 5,6-fused ring heteroarylene refers to two rings fused together, wherein one ring has 5 members and the other ring has 6 members, and wherein at least one ring is a heteroaryl ring. Likewise, a 6,6-fused ring heteroarylene refers to two rings fused together, wherein one ring has 6 members and the other ring has 6 members, and wherein at least one ring is a heteroaryl ring. And a 6,5-fused ring heteroarylene refers to two rings fused together, wherein one ring has 6 members and the other ring has 5 members, and wherein at least one ring is a heteroaryl ring. A heteroaryl group can be attached to the remainder of the molecule through a carbon or heteroatom. Non-limiting examples of aryl and heteroaryl groups include phenyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, and 6-quinolyl. Substituents for each of the above noted aryl and heteroaryl ring systems are selected from the group of acceptable substituents described below. An "arylene" and a "heteroarylene," alone or as part of another substituent, mean a divalent radical derived from an aryl and heteroaryl, respectively. Non-limiting examples of aryl and heteroaryl groups include pyridinyl, pyrimidinyl, thiophenyl, thienyl, furanyl, indolyl, benzoxadiazolyl, benzodioxolyl, benzodioxanyl, thianaphthanyl, pyrrolopyridinyl, indazolyl, quinolinyl, quinoxalinyl, pyridopyrazinyl, quinazolinonyl, benzoisoxazolyl, imidazopyridinyl, benzofuranyl, benzothienyl, benzothiophenyl, phenyl, naphthyl, biphenyl, pyrrolyl, pyrazolyl, imidazolyl, pyrazinyl, oxazolyl, isoxazolyl, thiazolyl, furylthienyl, pyridyl, pyrimidyl, benzothiazolyl, purinyl, benzimidazolyl, isoquinolyl, thiadiazolyl, oxadiazolyl, pyrrolyl, diazolyl, triazolyl, tetrazolyl, benzothiadiazolyl, isothiazolyl, pyrazolopyrimidinyl, pyrrolopyrimidinyl, benzotriazolyl, benzoxazolyl, or quinolyl. The examples above may be substituted or unsubstituted and divalent radicals of each heteroaryl example above are non-limiting examples of heteroarylene. A heteroaryl moiety may include one ring heteroatom (e.g., O, N, or S). A heteroaryl moiety may include two optionally different ring heteroatoms (e.g., O, N, or S). A heteroaryl moiety may include three optionally different ring heteroatoms (e.g., O, N, or S). A heteroaryl moiety may include four optionally different ring heteroatoms (e.g., O, N, or S). A heteroaryl moiety may include five optionally different ring heteroatoms (e.g., O, N, or S). An aryl moiety may have a single ring. An aryl moiety may have two optionally different rings. An aryl moiety may have three optionally different rings. An aryl moiety may have four optionally different rings. A heteroaryl moiety may have one ring. A heteroaryl moiety may have two optionally different rings. A heteroaryl moiety may have three optionally different rings. A heteroaryl moiety may have four optionally different rings. A heteroaryl moiety may have five optionally different rings.

A fused ring heterocyloalkyl-aryl is an aryl fused to a heterocycloalkyl. A fused ring heterocycloalkyl-heteroaryl is a heteroaryl fused to a heterocycloalkyl. A fused ring heterocycloalkyl-cycloalkyl is a heterocycloalkyl fused to a cycloalkyl. A fused ring heterocycloalkyl-heterocycloalkyl is a heterocycloalkyl fused to another heterocycloalkyl. Fused ring heterocycloalkyl-aryl, fused ring heterocycloalkyl-heteroaryl, fused ring heterocycloalkyl-cycloalkyl, or fused ring heterocycloalkyl-heterocycloalkyl may each independently be unsubstituted or substituted with one or more of the substituents described herein.

"Oxo" means an oxygen that is double bonded to a carbon atom.

"Alkylsulfonyl" means a moiety having the formula -S(O₂)-R', where R' is a substituted or unsubstituted alkyl group as defined above. R' may have a specified number of carbons (*e.g.,* "C₁-C₄ alkylsulfonyl").

Each of the above terms (*e.g.,* "alkyl", "heteroalkyl", "cycloalkyl", "heterocycloalkyl", "aryl", and "heteroaryl") includes both substituted and unsubstituted forms of the indicated radical. Preferred substituents for each type of radical are provided below.

Substituents for the alkyl and heteroalkyl radicals (including those groups often referred to as alkylene, alkenyl, heteroalkylene, heteroalkenyl, alkynyl, cycloalkyl, heterocycloalkyl, cycloalkenyl, and heterocycloalkenyl) can be one or more of a variety of groups selected from, but not limited to, -OR', =O, =NR', =N-OR', -NR'R", -SR', -halogen, -SiR'R"R"', -OC(O)R', -C(O)R', -CO₂R', -CONR'R", -OC(O)NR'R", -NR"C(O)R', -NR'-C(O)NR"R"', -NR"C(O)₂R', -NR-C(NR'R"R"')=NR"", -NR-C(NR'R")=NR"', -S(O)R', -S(O)₂R', -S(O)₂NR'R", -NRSO₂R', -NR'NR"R"', -ONR'R", -NR'C=(O)NR"NR"'R"", -CN, -NO₂, in a number ranging from zero to (2m'+1), where m' is the total number of carbon atoms in such radical. R, R', R", R'", and R"" each preferably independently refer to hydrogen, substituted or unsubstituted heteroalkyl, substituted or unsubstituted cycloalkyl, substituted or unsubstituted heterocycloalkyl, substituted or unsubstituted aryl (e.g., aryl substituted with 1-3 halogens), substituted or unsubstituted heteroaryl, substituted or unsubstituted alkyl, alkoxy, or thioalkoxy groups, or arylalkyl groups. When a compound includes more than one R group, for example, each of the R groups is independently selected as are each R', R", R'", and R"" group when more than one of these groups is present. When R' and R" are attached to the same nitrogen atom, they can be combined with the nitrogen atom to form a 4-, 5-, 6-, or 7-membered ring. For example, -NR'R" includes, but is not limited to, 1-pyrrolidinyl and 4-morpholinyl. From the above discussion of substituents, one of skill in the art will understand that the term "alkyl" is meant to include groups including carbon atoms bound to groups other than hydrogen groups, such as haloalkyl (e.g., -CF₃ and -CH₂CF₃) and acyl (e.g., -C(O)CH₃, -C(O)CF₃, -C(O)CH₂OCH₃, and the like).

Similar to the substituents described for the alkyl radical, substituents for the aryl and heteroaryl groups are varied and are selected from, for example: -OR', -NR'R", -SR', -halogen, -SiR'R"R"', -OC(O)R', -C(O)R', -CO₂R', -CONR'R", -OC(O)NR'R", -NR"C(O)R', -NR'-C(O)NR"R"', -NR"C(O)₂R', -NR-C(NR'R"R"')=NR"", -NR-C(NR'R")=NR"', -S(O)R', -S(O)₂R', -S(O)₂NR'R", -NRSO₂R', -NR'NR"R"', -ONR'R", -NR'C=(O)NR"NR"'R"", -CN, -NO₂, -R', -N₃, -CH(Ph)₂, fluoro(C₁-C₄)alkoxy, and fluoro(C₁-C₄)alkyl, in a number ranging from zero to the total number of open valences on the aromatic ring system; and where R', R", R"', and R"" are preferably independently selected from hydrogen, substituted or unsubstituted alkyl, substituted or unsubstituted heteroalkyl, substituted or unsubstituted cycloalkyl, substituted or unsubstituted heterocycloalkyl, substituted or unsubstituted aryl, and substituted or unsubstituted heteroaryl. When a compound includes more than one R group, for example, each of the R groups is independently selected as are each R', R", R"', and R"" groups when more than one of these groups is present.

Two or more substituents may optionally be joined to form aryl, heteroaryl, cycloalkyl, or heterocycloalkyl groups. Such so-called ring-forming substituents are typically, though not necessarily, found attached to a cyclic base structure. In aspects, the ring-forming substituents are attached to adjacent members of the base structure. For example, two ring-forming substituents attached to adjacent members of a cyclic base structure create a fused ring structure. In aspects, the ring-forming substituents are attached to a single member of the base structure. For example, two ring-forming substituents attached to a single member of a cyclic base structure create a spirocyclic structure. In aspects, the ring-forming substituents are attached to non-adjacent members of the base structure.

Two of the substituents on adjacent atoms of the aryl or heteroaryl ring may optionally form a ring of the formula -T-C(O)-(CRR')_{q}-U-, wherein T and U are independently -NR-, -O-, -CRR'-, or a single bond, and q is an integer of from 0 to 3. Alternatively, two of the substituents on adjacent atoms of the aryl or heteroaryl ring may optionally be replaced with a substituent of the formula -A-(CH₂)ᵣ-B-, wherein A and B are independently -CRR'-, -O-, -NR-, -S-, -S(O) -, -S(O)₂-, -S(O)₂NR'-, or a single bond, and r is an integer of from 1 to 4. One of the single bonds of the new ring so formed may optionally be replaced with a double bond. Alternatively, two of the substituents on adjacent atoms of the aryl or heteroaryl ring may optionally be replaced with a substituent of the formula -(CRR')ₛ-X'- (C"R"R"')_{d}-, where s and d are independently integers of from 0 to 3, and X' is -O-, -NR'-, -S-, -S(O)-, -S(O)₂-, or -S(O)₂NR'-. The substituents R, R', R", and R'" are preferably independently selected from hydrogen, substituted or unsubstituted alkyl, substituted or unsubstituted heteroalkyl, substituted or unsubstituted cycloalkyl, substituted or unsubstituted heterocycloalkyl, substituted or unsubstituted aryl, and substituted or unsubstituted heteroaryl.

"Heteroatom" or "ring heteroatom" are meant to include, oxygen (O), nitrogen (N), sulfur (S), phosphorus (P), and silicon (Si).

A "substituent group," as used herein, means a group selected from the following moieties: (A) oxo, halogen, -CF₃, -CN, -OH, -NH₂, -COOH, -CONH₂, -NO₂, -SH, -SO₃H, -SO₄H, -SO₂NH₂, -NHNH₂, -ONH₂, -NHC=(O)NHNH₂, -NHC=(O) NH₂, -NHSO₂H, - NHC= (O)H, -NHC(O)-OH, -NHOH, -OCF₃, -OCHF₂, unsubstituted alkyl, unsubstituted heteroalkyl, unsubstituted cycloalkyl, unsubstituted heterocycloalkyl, unsubstituted aryl, unsubstituted heteroaryl, and (B) alkyl, heteroalkyl, cycloalkyl, heterocycloalkyl, aryl, heteroaryl, substituted with at least one substituent selected from: (i) oxo, halogen, -CF₃, -CN, -OH, -NH₂, -COOH, -CONH₂, -NO₂, -SH, -SO₃H, -SO₄H,-SO₂NH₂, -NHNH₂, -ONH₂, -NHC=(O)NHNH₂, -NHC=(O)NH₂, -NHSO₂H, -NHC= (O)H, -NHC(O)OH, -NHOH, -OCF₃, -OCHF₂, unsubstituted alkyl, unsubstituted heteroalkyl, unsubstituted cycloalkyl, unsubstituted heterocycloalkyl, unsubstituted aryl, unsubstituted heteroaryl, and (ii) alkyl, heteroalkyl, cycloalkyl, heterocycloalkyl, aryl, heteroaryl, substituted with at least one substituent selected from: (a) oxo, halogen, -CF₃, -CN, -OH, -NH₂, -COOH, -CONH₂, -NO₂, -SH, -SO₃H, -SO₄H, -SO₂NH₂, -NHNH₂, -ONH₂, -NHC=(O)NHNH₂, -NHC=(O) NH₂, -NHSO₂H, -NHC= (O)H, -NHC(O)-OH, -NHOH, -OCF₃, -OCHF₂, unsubstituted alkyl, unsubstituted heteroalkyl, unsubstituted cycloalkyl, unsubstituted heterocycloalkyl, unsubstituted aryl, unsubstituted heteroaryl, and (b) alkyl, heteroalkyl, cycloalkyl, heterocycloalkyl, aryl, heteroaryl, substituted with at least one substituent selected from: oxo, halogen, -CF₃, -CN, -OH, -NH₂, -COOH, -CONH₂, -NO₂, -SH, -SO₃H, -SO₄H, -SO₂NH₂, -NHNH₂, -ONH₂, -NHC=(O)NHNH₂, -NHC=(O) NH₂, -NHSO₂H, -NHC= (O)H,-NHC(O)OH, -NHOH, -OCF₃, -OCHF₂, unsubstituted alkyl, unsubstituted heteroalkyl, unsubstituted cycloalkyl, unsubstituted heterocycloalkyl, unsubstituted aryl, unsubstituted heteroaryl.

A "size-limited substituent" or " size-limited substituent group," as used herein, means a group selected from all of the substituents described above for a "substituent group," wherein each substituted or unsubstituted alkyl is a substituted or unsubstituted C₁-C₂₀ alkyl, each substituted or unsubstituted heteroalkyl is a substituted or unsubstituted 2 to 20 membered heteroalkyl, each substituted or unsubstituted cycloalkyl is a substituted or unsubstituted C₃-C₈ cycloalkyl, each substituted or unsubstituted heterocycloalkyl is a substituted or unsubstituted 3 to 8 membered heterocycloalkyl, each substituted or unsubstituted aryl is a substituted or unsubstituted C₆-C₁₀ aryl, and each substituted or unsubstituted heteroaryl is a substituted or unsubstituted 5 to 10 membered heteroaryl.

A "lower substituent" or " lower substituent group," as used herein, means a group selected from all of the substituents described above for a "substituent group," wherein each substituted or unsubstituted alkyl is a substituted or unsubstituted C₁-C₈ alkyl, each substituted or unsubstituted heteroalkyl is a substituted or unsubstituted 2 to 8 membered heteroalkyl, each substituted or unsubstituted cycloalkyl is a substituted or unsubstituted C₃-C₇ cycloalkyl, each substituted or unsubstituted heterocycloalkyl is a substituted or unsubstituted 3 to 7 membered heterocycloalkyl, each substituted or unsubstituted aryl is a substituted or unsubstituted C₆-C₁₀ aryl, and each substituted or unsubstituted heteroaryl is a substituted or unsubstituted 5 to 9 membered heteroaryl.

In embodiments, each substituted group described in the compounds herein is substituted with at least one substituent group. More specifically, in some aspects, each substituted alkyl, substituted heteroalkyl, substituted cycloalkyl, substituted heterocycloalkyl, substituted aryl, substituted heteroaryl, substituted alkylene, substituted heteroalkylene, substituted cycloalkylene, substituted heterocycloalkylene, substituted arylene, and/or substituted heteroarylene described in the compounds herein are substituted with at least one substituent group. In aspects, at least one or all of these groups are substituted with at least one size-limited substituent group. In aspects, at least one or all of these groups are substituted with at least one lower substituent group.

In embodiments of the compounds described herein, each substituted or unsubstituted alkyl may be a substituted or unsubstituted C₁-C₂₀ alkyl, each substituted or unsubstituted heteroalkyl is a substituted or unsubstituted 2 to 20 membered heteroalkyl, each substituted or unsubstituted cycloalkyl is a substituted or unsubstituted C₃-C₈ cycloalkyl, each substituted or unsubstituted heterocycloalkyl is a substituted or unsubstituted 3 to 8 membered heterocycloalkyl, each substituted or unsubstituted aryl is a substituted or unsubstituted C₆-C₁₀ aryl, and/or each substituted or unsubstituted heteroaryl is a substituted or unsubstituted 5 to 10 membered heteroaryl. In aspects of the compounds herein, each substituted or unsubstituted alkylene is a substituted or unsubstituted C₁-C₂₀ alkylene, each substituted or unsubstituted heteroalkylene is a substituted or unsubstituted 2 to 20 membered heteroalkylene, each substituted or unsubstituted cycloalkylene is a substituted or unsubstituted C₃-C₈ cycloalkylene, each substituted or unsubstituted heterocycloalkylene is a substituted or unsubstituted 3 to 8 membered heterocycloalkylene, each substituted or unsubstituted arylene is a substituted or unsubstituted C₆-C₁₀ arylene, and/or each substituted or unsubstituted heteroarylene is a substituted or unsubstituted 5 to 10 membered heteroarylene.

In embodiments, each substituted or unsubstituted alkyl is a substituted or unsubstituted C₁-C₈ alkyl, each substituted or unsubstituted heteroalkyl is a substituted or unsubstituted 2 to 8 membered heteroalkyl, each substituted or unsubstituted cycloalkyl is a substituted or unsubstituted C₃-C₇ cycloalkyl, each substituted or unsubstituted heterocycloalkyl is a substituted or unsubstituted 3 to 7 membered heterocycloalkyl, each substituted or unsubstituted aryl is a substituted or unsubstituted C₆-C₁₀ aryl, and/or each substituted or unsubstituted heteroaryl is a substituted or unsubstituted 5 to 9 membered heteroaryl. In some aspects, each substituted or unsubstituted alkylene is a substituted or unsubstituted C₁-C₈ alkylene, each substituted or unsubstituted heteroalkylene is a substituted or unsubstituted 2 to 8 membered heteroalkylene, each substituted or unsubstituted cycloalkylene is a substituted or unsubstituted C₃-C₇ cycloalkylene, each substituted or unsubstituted heterocycloalkylene is a substituted or unsubstituted 3 to 7 membered heterocycloalkylene, each substituted or unsubstituted arylene is a substituted or unsubstituted C₆-C₁₀ arylene, and/or each substituted or unsubstituted heteroarylene is a substituted or unsubstituted 5 to 9 membered heteroarylene.

The term "pharmaceutically acceptable salt" is meant to include salts of the active compounds that are prepared with relatively nontoxic acids or bases, depending on the particular substituents found on the compounds described herein. When the compounds described herein contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When the compounds described herein contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic,monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like. Certain of the compounds described herein contain both basic and acidic functionalities that allow the compounds to be converted into either base or acid addition salts.

Thus, the compounds described herein may exist as salts, such as with pharmaceutically acceptable acids. Examples of such salts include hydrochlorides, hydrobromides, sulfates, methanesulfonates, nitrates, maleates, acetates, citrates, fumarates, tartrates (e.g., (+)-tartrates, (-)-tartrates, or mixtures thereof including racemic mixtures), succinates, benzoates, and salts with amino acids such as glutamic acid. These salts may be prepared by methods known to those skilled in the art.

The neutral forms of the compounds are preferably regenerated by contacting the salt with a base or acid and isolating the parent compound in the conventional manner. The parent form of the compound differs from the various salt forms in certain physical properties, such as solubility in polar solvents.

Compounds described herein can exist in unsolvated forms as well as solvated forms, including hydrated forms. In general, the solvated forms are equivalent to unsolvated forms.

Compounds described herein possess asymmetric carbon atoms (optical or chiral centers) or double bonds; the enantiomers, racemates, diastereomers, tautomers, geometric isomers, stereoisometric forms that may be defined, in terms of absolute stereochemistry, as (R)-or (S)- or, as (D)- or (L)- for amino acids, and individual isomers are encompassed within the scope of the disclosure. The disclosure is meant to include compounds in racemic and optically pure forms. Optically active (R)- and (S)-, or (D)- and (L)-isomers may be prepared using chiral synthons or chiral reagents, or resolved using conventional techniques. Unless otherwise stated, structures depicted herein are also meant to include all stereochemical forms of the structure; i.e., the R and S configurations for each asymmetric center. Therefore, single stereochemical isomers as well as enantiomeric and diastereomeric mixtures of the present compounds are within the scope of the disclosure. When the compounds described herein contain olefinic bonds or other centers of geometric asymmetry, and unless specified otherwise, it is intended that the compounds include both E and Z geometric isomers.

"Tautomer," as used herein, refers to one of two or more structural isomers which exist in equilibrium and which are readily converted from one isomeric form to another. It will be apparent to one skilled in the art that certain compounds may exist in tautomeric forms.

Unless otherwise stated, structures depicted herein are also meant to include compounds which differ only in the presence of one or more isotopically enriched atoms. For example, compounds may have the present structures except for the replacement of a hydrogen by a deuterium or tritium, or the replacement of a carbon by ¹³C- or ¹⁴C-enriched carbon. The compounds described herein may also contain unnatural proportions of atomic isotopes at one or more of the atoms that constitute such compounds. For example, the compounds may be radiolabeled with radioactive isotopes, such as for example tritium (³H), iodine-125 (¹²⁵I), or carbon-14 (¹⁴C).

The symbol "-" denotes the point of attachment of a chemical moiety to the remainder of a molecule or chemical formula.

In embodiments, the compounds described herein may include multiple instances of R² and/or other variables. In aspects, each variable may optional be different and be appropriately labeled to distinguish each group for greater clarity. For example, where each R² is different, they may be referred to, for example, as R^{2.1}, R^{2.2}, R^{2.3}, and/or R^{2.4} respectively, wherein the definition of R² is assumed by R^{2.1}, R^{2.2}, R^{2.3}, and/or R^{2.4}. The variables used within a definition of R² and/or other variables that appear at multiple instances and are different may similarly be appropriately labeled to distinguish each group for greater clarity. In aspects, the compound is a compound described herein (e.g., in an aspect, embodiment, example, claim, table, scheme, drawing, or figure).

The terms "a" or "an," as used in herein means one or more. In addition, the phrase "substituted with a[n]," as used herein, means the specified group may be substituted with one or more of any or all of the named substituents. For example, where a group, such as an alkyl or heteroaryl group, is "substituted with an unsubstituted C₁-C₂₀ alkyl, or unsubstituted 2 to 20 membered heteroalkyl," the group may contain one or more unsubstituted C₁-C₂₀ alkyls, and/or one or more unsubstituted 2 to 20 membered heteroalkyls.

Where a moiety is substituted with an R substituent, the group may be referred to as "R-substituted." Where a moiety is R-substituted, the moiety is substituted with at least one R substituent and each R substituent is optionally different. For example, where a moiety herein is R¹²-substituted or unsubstituted alkyl, a plurality of R¹² substituents may be attached to the alkyl moiety wherein each R¹² substituent is optionally different. Where an R-substituted moiety is substituted with a plurality R substituents, each of the R-substituents may be differentiated herein using a prime symbol (') such as R', R", etc. For example, where a moiety is R¹²-substituted or unsubstituted alkyl, and the moiety is substituted with a plurality of R¹² substituents, the plurality of R¹² substituents may be differentiated as R¹²', R¹²", R¹²"', etc. In aspects, the plurality of R substituents is 3. In aspects, the plurality of R substituents is 2.

In embodiments, a compound as described herein may include multiple instances of R¹, R², R³, R⁴, R⁵, R⁶, R⁷, R⁹, R¹⁰, R¹¹, R¹², R¹³, R¹⁴ and/or other variables. In aspects, each variable may optional be different and be appropriately labeled to distinguish each group for greater clarity. For example, where each R¹, R², R³, R⁴, R⁵, R⁶, R⁷, R⁹, R¹⁰, R¹¹, R¹², R¹³, and/or R¹⁴, is different, they may be referred to, for example, as R¹¹, R^{1.2}, R^{1.3} , R^{1.4}, R^{2.1}, R^{2.2}, R^{2.3}, R^{2.4}, R^{3.1}, R^{3.2}, R^{3.3}, R^{3.4}, R^{4.1}, R^{4.2}, R^{4.3}, R^{4.4}, R^{5.1}, R^{5.2}, R⁵³, R^{5.4}, R^{6.1}, R^{6.2}, R^{6.3}, R^{6.4}, R^{7.1}, R^{7.2}, R^{7.3}, R^{7.4}, R^{9.1}, R^{9.2}, R^{9.3}, R^{9.4}, R^{10.1}, R^{10.2}, R^{10.3}, R^{10.4}, R^{11.1} , R^{11.2}, R^{11.3}, R^{11.4}, R^{12.1}, R^{12.2}, R^{12.3}, R^{12.4}, R^{13.1}, R^{13.2}, R^{13.3}, R^{13.4}, R^{14.1} , R^{14.2}, R^{14.3}, and/or R^{14.4}, respectively, wherein the definition of R¹ is assumed by R^{1.1}, R^{1.2}, R^{1.3}, and/or R¹⁴, the definition of R² is assumed by R^{2.1} , R^{2.2}, R^{2.3}, and/or R^{2.4}, the definition of R³ is assumed by R^{3.1}, R^{3.2}, R^{3.3}, and/or R^{3.4}, the definition of R⁴ is assumed by R^{4.1}, R^{4.2}, R^{4.3}, and/or R^{4.4}, the definition of R⁵ is assumed by R^{5.1} , R^{5.2} , R^{5.3}, and/or R^{5.4}, the definition of R⁶ is assumed by R^{6.1}, R^{6.2}, R^{6.3}, and/or R^{6.4}, the definition of R⁷ is assumed by R^{7.1}, R^{7.2}, R^{7.3}, and/or R^{7.4}, the definition of R⁹ is assumed by R^{9.1}, R^{9.2}, R^{9.3}, and/or R^{9.4}, the definition of R¹⁰ is assumed by R^{10.1}, R^{10.2}, R^{10.3}, and/or R^{10.4}, the definition of R¹¹ is assumed by R^{11.1}, R^{11.2}, R^{11.3}, and/or R^{11.4}, the definition of R¹² is assumed by R^{12.1}, R^{12.2}, R^{12.3}, and/or R^{12.4}, the definition of R¹³ is assumed by R^{13.1}, R^{13.2}, R^{13.3}, and/or R^{13.A}, the definition of R¹⁴ is assumed by R^{14.1}, R^{14.2}, R^{14.3}, and/or R^{14. 4}. The variables used within a definition of R¹, R², R³, R⁴, R⁵, R⁶, R⁷, R⁹, R¹⁰, R¹¹, R¹², R¹³ and/or R¹⁴, and/or other variables that appear at multiple instances and are different may similarly be appropriately labeled to distinguish each group for greater clarity.

Descriptions of compounds herein are limited by principles of chemical bonding known to those skilled in the art. Accordingly, where a group may be substituted by one or more of a number of substituents, such substitutions are selected so as to comply with principles of chemical bonding and to give compounds which are not inherently unstable and/or would be known to one of ordinary skill in the art as likely to be unstable under ambient conditions, such as aqueous, neutral, and several known physiological conditions. For example, a heterocycloalkyl or heteroaryl is attached to the remainder of the molecule via a ring heteroatom in compliance with principles of chemical bonding known to those skilled in the art thereby avoiding inherently unstable compounds.

### Pharmaceutical Compositions of Embodiment H

In embodiments, the disclosure provides amorphous solid dispersions comprising an adenosine A2A receptor antagonist and a thermoplastic polymer. In aspects, the amorphous solid dispersions further comprise one or more pharmaceutically acceptable excipients. In aspects, the amorphous solid dispersions are produced by hot melt extrusion. In embodiments, the disclosure provides compositions comprising an amorphous adenosine A2A receptor antagonist dispersed in a thermoplastic polymer. In aspects, the composition is a solid composition. In aspects, the compositions are produced by hot melt extrusion. In embodiments, the disclosure provides extrudates comprising an adenosine A2A receptor antagonist and a thermoplastic polymer. In embodiments, the disclosure provides oral formulations comprising an adenosine A2A receptor antagonist and a thermoplastic polymer. In aspects, the oral formulation is a tablet, a capsule, a granule, or a powder.

In embodiments of the amorphous solid dispersions, compositions, extrudates, and oral formulations described herein, the adenosine A2A receptor antagonist is a compound of Formula (I) or a pharmaceutically acceptable salt thereof. In aspects, the compound of Formula (I) is in the form of a pharmaceutically acceptable salt. In aspects, the compound of Formula (I) is in free base form. In aspects, the adenosine A2A receptor antagonist is a compound of Formula (II) or a pharmaceutically acceptable salt thereof. In aspects, the compound of Formula (II) is in the form of a pharmaceutically acceptable salt. In aspects, the compound of Formula (II) is in free base form. In aspects, the adenosine A2A receptor antagonist is a compound of Formula (III) or a pharmaceutically acceptable salt thereof. In aspects, the compound of Formula (III) is in the form of a pharmaceutically acceptable salt. In aspects, the compound of Formula (III) is in free base form. In aspects, the adenosine A2A receptor antagonist is a compound of Formula (IIIA) or a pharmaceutically acceptable salt thereof. In aspects, the compound of Formula (IIIA) is in free base form. In aspects, the compound of Formula (IIIA) is in the form of a pharmaceutically acceptable salt. In aspects, the adenosine A2A receptor antagonist is a compound of Formula (IIIB) or a pharmaceutically acceptable salt thereof. In aspects, the compound of Formula (IIIB) is in free base form. In aspects, the compound of Formula (IIIB) is in the form of a pharmaceutically acceptable salt. In aspects, the adenosine A2A receptor antagonist is a mixture of a compound of Formula (IIIA) or a pharmaceutically acceptable salt thereof and a compound of Formula (IIIB) or a pharmaceutically acceptable salt thereof. In aspects, the adenosine A2A receptor antagonist is a racemic mixture of a compound of Formula (IIIA) or a pharmaceutically acceptable salt thereof and a compound of Formula (IIIB) or a pharmaceutically acceptable salt thereof. In aspects, the adenosine A2A receptor antagonist of Formula (I), (II), (III), (IIIA), or (IIIB) is amorphous. In aspects, the adenosine A2A receptor antagonist of Formula (I), (II), (III), (IIIA), or (IIIB) is the sole active ingredient in the amorphous solid dispersions, compositions, extrudates, and oral formulations.

In embodiments of the amorphous solid dispersions, compositions, extrudates, and oral formulations described herein, the thermoplastic polymer is any known in the art. Exemplary thermoplastic polymers include polyethylene oxide; polypropylene oxide; copolymers of polyethylene oxide and polypropylene oxide; polyvinylpyrrolidone; polyvinyl acetate; ethylene vinyl acetate; copolymers of polyvinylpyrrolidone and vinyl acetate; acrylate polymers; methacrylate copolymers; copolymers of acrylate and methacrylate; polyethylene; polyethylene glycol; poly(vinyl alcohol); polycaprolactone; cellulose compounds (e.g., methyl cellulose, hydroxymethyl cellulose, hydroxyethyl cellulose, hydroxypropyl cellulose, hydroxybutyl cellulose, hydroxyethyl methyl cellulose, hydroxypropylmethyl cellulose, carboxymethyl cellulose); starches, pectins; polylactide polymers; polyglycolide polymers; polylactide-co-glycolide copolymers; polyesters; wax (e.g., carnauba wax, beeswax); and the like. In aspects, the thermoplastic polymer is (i) polyvinylpyrrolidone; (ii) vinyl acetate; (iii) a copolymer of polyvinylpyrrolidone and vinyl acetate; (iv) a methacrylic acid polymer; (v) an ethyl acrylate polymer; (vi) a copolymer of methacrylic acid and ethyl acrylate; (vii) a methyl methacrylate polymer; (viii) a copolymer of methacrylic acid and methyl methacrylate; (ix) a cellulose compound; or (x) a combination of two or more of the foregoing. In aspects, the thermoplastic polymer is (i) polyvinylpyrrolidone; (ii) a copolymer of polyvinylpyrrolidone and vinyl acetate; (iii) a copolymer of methacrylic acid and ethyl acrylate; (iv) a copolymer of methacrylic acid and methyl methacrylate; (v) hydroxypropyl methylcellulose; or (vii) a combination of two or more of the foregoing. In aspects, the thermoplastic polymer is (i) a polyvinylpyrrolidone polymer, (ii) a polyvinylpyrrolidone copolymer, (iii) an acrylic polymer, (iv) an acrylic copolymer, (v) hydroxypropyl methylcellulose acetate succinate, or (vi) a combination of two or more of the foregoing. In aspects, the thermoplastic polymer is (i) a copolymer of 1-vinyl-2-pyrrolidone and vinyl acetate at the weight ratio of 1-vinyl-2-pyrrolidone to vinyl acetate of about 3:2; (ii) a copolymer of methacrylic acid and ethyl acrylate at the weight ratio of methacrylic acid to ethyl acrylate of about 1:1; (iii) a copolymer of methacrylic acid and methyl methacrylate at the weight ratio of methacrylic acid to methyl methacrylate of 1:1; (iv) hydroxypropyl methylcellulose acetate succinate; or (v) a combination of two or more of the foregoing. In aspects, the thermoplastic polymer is (i) a copolymer of 1-vinyl-2-pyrrolidone and vinyl acetate at the weight ratio of 1-vinyl-2-pyrrolidone to vinyl acetate of about 3:2; (ii) a copolymer of methacrylic acid and ethyl acrylate at the weight ratio of methacrylic acid to ethyl acrylate of about 1:1; (iii) a copolymer of methacrylic acid and methyl methacrylate at the weight ratio of methacrylic acid to methyl methacrylate of 1:1; or (iv) a combination of two or more of the foregoing. In aspects, the thermoplastic polymer is (i) a copolymer of methacrylic acid and ethyl acrylate at the weight ratio of methacrylic acid to ethyl acrylate of about 1:1; (ii) a copolymer of methacrylic acid and methyl methacrylate at the weight ratio of methacrylic acid to methyl methacrylate of 1:1; or (iii) a combination thereof. In aspects, the thermoplastic polymer is polyvinylpyrrolidone. In aspects, the thermoplastic polymer is a copolymer of polyvinylpyrrolidone and vinyl acetate. In aspects, the thermoplastic polymer is a copolymer of 1-vinyl-2-pyrrolidone and vinyl acetate at the weight ratio of 1-vinyl-2-pyrrolidone to vinyl acetate of about 3:2. In aspects, the thermoplastic polymer is a copolymer of methacrylic acid and ethyl acrylate. In aspects, the thermoplastic polymer is a copolymer of methacrylic acid and ethyl acrylate at the weight ratio of methacrylic acid to ethyl acrylate of about 1:1. In aspects, the thermoplastic polymer is a copolymer of methacrylic acid and methyl methacrylate. In aspects, the thermoplastic polymer is a copolymer of methacrylic acid and methyl methacrylate at the weight ratio of methacrylic acid to methyl methacrylate of 1:1. In aspects, the thermoplastic polymer is an acrylic polymer or an acrylic copolymer. In aspects, the thermoplastic polymer is a methyl methacrylate polymer, an ethyl acrylate polymer, or a copolymer of methyl methacrylate and ethyl acrylate. In aspects, the thermoplastic polymer is hydroxypropyl cellulose. In aspects, the thermoplastic polymer is hydroxypropyl cellulose having a viscosity from about 3.0 mPa•s to about 5.9 mPa•s. In aspects, the thermoplastic polymer is hydroxypropyl methylcellulose. In aspects, the thermoplastic polymer is hydroxypropyl methylcellulose that has an average of about 29% methoxyl groups and about 10% hydroxypropyl groups. In aspects, the thermoplastic polymer is hydroxypropyl methyl cellulose having the following characteristics: (i) an apparent viscosity of about 2.4 mPa•s to about 3.6 mPa•s; (ii) a methyl content from about 28.0% to about 30.0%; and (iii) a hydroxypropyl content from about 7.0% to about 12.0%. In aspects, the thermoplastic polymer is hydroxypropyl methylcellulose acetate succinate. In aspects, the thermoplastic polymer is hydroxypropyl methylcellulose acetate succinate have a mean particle size of about 5 µm and a viscosity of about 3 mPa•s. In aspects, the thermoplastic polymers described herein have a glass transition temperature of about 65°C or higher. In aspects, the glass transition temperature is from about 65°C to about 150°C. In aspects, the glass transition temperature is from about 65°C to about 100°C. In aspects, the glass transition temperature is about 70°C or higher. In aspects, the glass transition temperature is from about 70°C to about 150°C. In aspects, the glass transition temperature is from about 70°C to about 100°C. In aspects, the glass transition temperature is from about 70°C to about 95°C. In aspects, the glass transition temperature is from about 80°C to about 95°C. In aspects, the glass transition temperature is from about 80°C to about 90°C.

In embodiments, the amorphous solid dispersions, compositions, extrudates, and oral formulations described herein have a glass transition temperature of about 65°C or higher. In aspects, the glass transition temperature is from about 65°C to about 150°C. In aspects, the glass transition temperature is from about 65°C to about 100°C. In aspects, the glass transition temperature is about 70°C or higher. In aspects, the glass transition temperature is from about 70°C to about 150°C. In aspects, the glass transition temperature is from about 70°C to about 100°C. In aspects, the glass transition temperature is from about 70°C to about 95°C. In aspects, the glass transition temperature is from about 80°C to about 95°C. In aspects, the glass transition temperature is from about 80°C to about 90°C.

In embodiments of the amorphous solid dispersions, compositions, extrudates, and oral formulations described herein, the adenosine A2A receptor antagonist is present in an amount of about 10 wt% to about 50 wt%. In aspects, the adenosine A2A receptor antagonist is present in an amount of about 15 wt% to about 45 wt%. In aspects, the adenosine A2A receptor antagonist is present in an amount of about 10 wt% to about 40 wt%. In aspects, the adenosine A2A receptor antagonist is present in an amount of about 20 wt% to about 40 wt%. In aspects, the adenosine A2A receptor antagonist is present in an amount of about 25 wt% to about 35 wt%. In aspects, the adenosine A2A receptor antagonist is present in an amount of about 20 wt%. In aspects, the adenosine A2A receptor antagonist is present in an amount of about 25 wt%. In aspects, the adenosine A2A receptor antagonist is present in an amount of about 30 wt%. In aspects, the adenosine A2A receptor antagonist is present in an amount of about 35 wt%.

In embodiments of the amorphous solid dispersions, compositions, extrudates, and oral formulations described herein, the thermoplastic polymer is present in an amount of about 50 wt% to about 90 wt%. In aspects, the thermoplastic polymer is present in an amount of about 55 wt% to about 85 wt%. In aspects, the thermoplastic polymer is present in an amount of about 60 wt% to about 80 wt%. In aspects, the thermoplastic polymer is present in an amount of about 60 wt% to about 90 wt%. In aspects, the thermoplastic polymer is present in an amount of about 65 wt% to about 75 wt%. In aspects, the thermoplastic polymer is present in an amount of about 60 wt%. In aspects, the thermoplastic polymer is present in an amount of about 65 wt%. In aspects, the thermoplastic polymer is present in an amount of about 70 wt%. In aspects, the thermoplastic polymer is present in an amount of about 75 wt%. In aspects, the thermoplastic polymer is present in an amount of about 80 wt%.

In embodiments of the amorphous solid dispersions, compositions, extrudates, and oral formulations described herein, the adenosine A2A receptor antagonist is present in an amount of about 10 wt% to about 50 wt% and the thermoplastic polymer is present in an amount of about 50 wt% to about 90 wt%. In aspects, the adenosine A2A receptor antagonist is present in an amount of about 10 wt% to about 40 wt% and the thermoplastic polymer is present in an amount of about 60 wt% to about 90 wt%. In aspects, the adenosine A2A receptor antagonist is present in an amount of about 15 wt% to about 45 wt% and the thermoplastic polymer is present in an amount of about 55 wt% to about 85 wt%. In aspects, the adenosine A2A receptor antagonist is present in an amount of about 20 wt% to about 40 wt% and the thermoplastic polymer is present in an amount of about 60 wt% to about 80 wt%. In aspects, the adenosine A2A receptor antagonist is present in an amount of about 25 wt% to about 35 wt% and the thermoplastic polymer is present in an amount of about 65 wt% to about 75 wt%. In aspects, the adenosine A2A receptor antagonist is present in an amount of about 30 wt% and the thermoplastic polymer is present in an amount of about 70 wt%.

In embodiments of the amorphous solid dispersions, compositions, extrudates, and oral formulations described herein, the weight ratio of the adenosine A2A receptor antagonist to the thermoplastic polymer is from about 1:10 to about 1:1. In aspects, the weight ratio of the adenosine A2A receptor antagonist to the thermoplastic polymer is from about 1:9 to about 1:1. In aspects, the weight ratio of the adenosine A2A receptor antagonist to the thermoplastic polymer is from about 1:8 to about 1:1. In aspects, the weight ratio of the adenosine A2A receptor antagonist to the thermoplastic polymer is from about 1:7 to about 1:1. In aspects, the weight ratio of the adenosine A2A receptor antagonist to the thermoplastic polymer is from about 1:6 to about 1:1. In aspects, the weight ratio of the adenosine A2A receptor antagonist to the thermoplastic polymer is from about 1:5 to about 1:1. In aspects, the weight ratio of the adenosine A2A receptor antagonist to the thermoplastic polymer is from about 1:4 to about 1:1. In aspects, the weight ratio of the adenosine A2A receptor antagonist to the thermoplastic polymer is from about 1:7 to about 1:1.2. In aspects, the weight ratio of the adenosine A2A receptor antagonist to the thermoplastic polymer is from about 1:4 to about 1:1.5. In aspects, the weight ratio of the adenosine A2A receptor antagonist to the thermoplastic polymer is from about 1:3 to about 1:1.5. In aspects, the weight ratio of the adenosine A2A receptor antagonist to the thermoplastic polymer is from about 1:3 to about 1:2. In aspects, the weight ratio of the adenosine A2A receptor antagonist to the thermoplastic polymer is from about 1:2.5 to about 1:2. In aspects, the weight ratio of the adenosine A2A receptor antagonist to the thermoplastic polymer is about 1:2.3.

In embodiment of the compositions provided herein, the composition includes no other active pharmaceutical ingredients other than one or more A2A receptor antagonists. In aspect of the compositions provided herein, the composition includes only a single type of A2A receptor antagonist. In aspects of the compositions provided herein, the composition include only a single type of A2A receptor antagonist and no other active pharmaceutical ingredient. In aspects of the compositions provided herein, the composition includes only a single type of A2A receptor antagonist and no pharmaceutical excipients or other active pharmaceutical ingredients.

### Pharmaceutical Compositions of Embodiment M

In embodiments, the disclosure provides amorphous solid dispersions comprising an adenosine A2A receptor antagonist and a pharmaceutically acceptable polymer. In aspects, the adenosine A2A receptor antagonist is amorphous. In aspects, the pharmaceutically acceptable polymer is any polymer known in the art, such as an enteric polymer. In aspects, the disclosure provides compositions comprising an amorphous adenosine A2A receptor antagonist and a pharmaceutically acceptable polymer. In aspects, the pharmaceutically acceptable polymer is any polymer known in the art, such as an enteric polymer. In aspects, the disclosure provides microprecipitated bulk powders comprising an adenosine A2A receptor antagonist and a pharmaceutically acceptable polymer. In aspects, the adenosine A2A receptor antagonist is amorphous. In aspects, the pharmaceutically acceptable polymer is any polymer known in the art, such as an enteric polymer. In aspects, the disclosure provides oral formulations comprising an adenosine A2A receptor antagonist and a pharmaceutically acceptable polymer. In aspects, the adenosine A2A receptor antagonist is amorphous. In aspects, the pharmaceutically acceptable polymer is any polymer known in the art, such as an enteric polymer. In aspects, the pharmaceutically acceptable polymer is not an enteric polymer. In aspects, the oral formulation is a tablet, a capsule, granule, or a powder.

In embodiments of the amorphous solid dispersions, compositions, microprecipitated bulk powders, and oral formulations described herein, the adenosine A2A receptor antagonist is a compound of Formula (I) or a pharmaceutically acceptable salt thereof. In aspects, the compound of Formula (I) is in free base form. In aspects, the compound of Formula (I) is in the form of a pharmaceutically acceptable salt. In aspects, the adenosine A2A receptor antagonist is a compound of Formula (II) or a pharmaceutically acceptable salt thereof. In aspects, the compound of Formula (II) is in free base form. In aspects, the compound of Formula (II) is in the form of a pharmaceutically acceptable salt. In aspects, the adenosine A2A receptor antagonist is a compound of Formula (III) or a pharmaceutically acceptable salt thereof. In aspects, the compound of Formula (III) is in the form of a pharmaceutically acceptable salt. In aspects, the compound of Formula (III) is in free base form. In aspects, the adenosine A2A receptor antagonist is a compound of Formula (IIIA) or a pharmaceutically acceptable salt thereof. In aspects, the compound of Formula (IIIA) is in free base form. In aspects, the compound of Formula (IIIA) is in the form of a pharmaceutically acceptable salt. In aspects, the adenosine A2A receptor antagonist is a compound of Formula (IIIB) or a pharmaceutically acceptable salt thereof. In aspects, the compound of Formula (IIIB) is in free base form. In aspects, the compound of Formula (IIIB) is in the form of a pharmaceutically acceptable salt. In aspects, the adenosine A2A receptor antagonist is a mixture of a compound of Formula (IIIA) or a pharmaceutically acceptable salt thereof and a compound of Formula (IIIB) or a pharmaceutically acceptable salt thereof. In aspects, the adenosine A2A receptor antagonist is a racemic mixture of a compound of Formula (IIIA) or a pharmaceutically acceptable salt thereof and a compound of Formula (IIIB) or a pharmaceutically acceptable salt thereof. In aspects, the adenosine A2A receptor antagonist of Formula (I), (II), (III), (IIIA), or (IIIB) is amorphous. In aspects, the adenosine A2A receptor antagonist of Formula (I), (II), (III), (IIIA), or (IIIB) is the sole active ingredient in the amorphous solid dispersions, compositions, extrudates, and oral formulations.

In aspects of the amorphous solid dispersions, compositions, microprecipitated bulk powders, and oral formulations described herein, the pharmaceutically acceptable polymer is any polymer known in the art. In aspects, the pharmaceutically acceptable polymer is an enteric polymer. In aspects, the enteric polymer is a methacrylic acid copolymer. In aspects, the enteric polymer is an acrylic polymer. In aspects, the enteric polymer is an acrylic copolymer. In aspects, the enteric polymer is a methyl methacrylate polymer. In aspects, the enteric polymer is an ethyl acrylate copolymer. In aspects, the enteric polymer is a copolymer of methacrylic acid and ethyl acrylate. In aspects, the enteric polymer is a copolymer of methacrylic acid and ethyl acrylate at the weight ratio of methacrylic acid to ethyl acrylate of about 1:1. In aspects, the enteric polymer is a copolymer of methacrylic acid and methyl methacrylate. In aspects, the enteric polymer is a copolymer of methacrylic acid and methyl methacrylate at the weight ratio of methacrylic acid to methyl methacrylate of 1:1. In aspects, the enteric polymer is an acrylic polymer or an acrylic copolymer. In aspects, the enteric polymer is a methyl methacrylate polymer, an ethyl acrylate polymer, or a copolymer of methyl methacrylate and ethyl acrylate. In aspects, the enteric polymer is a copolymer of 1-vinyl-2-pyrrolidone and vinyl acetate at the weight ratio of 1-vinyl-2-pyrrolidone to vinyl acetate of about 3:2. In aspects, the enteric polymer is a copolymer of methacrylic acid and ethyl acrylate at the weight ratio of methacrylic acid to ethyl acrylate of about 1:1. In aspects, the enteric polymer is a copolymer of methacrylic acid and methyl methacrylate at the weight ratio of methacrylic acid to methyl methacrylate of 1:1. In aspects, the enteric polymer is polymerized gelatin. In aspects, the enteric polymer is shellac. In aspects, the enteric polymer is a cellulose phthalate compound. Exemplary cellulose phthalate compounds include cellulose butyrate phthalate, cellulose hydrogen phthalate, cellulose propionate phthalate, polyvinyl acetate phthalate, cellulose acetate phthalate, hydroxypropyl methylcellulose phthalate, and hydroxypropyl methylcellulose acetate. In aspects, the enteric polymer is cellulose acetate trimellitate. In aspects, the enteric polymer is dioxypropyl methylcellulose succinate. In aspects, the enteric polymer is carboxymethyl ethylcellulose. In aspects, the enteric polymer is hydroxypropyl methylcellulose acetate succinate.

In aspects of the amorphous solid dispersions, compositions, microprecipitated bulk powders, and oral formulations described herein, the adenosine A2A receptor antagonist is present in an amount of about 5 wt% to about 40 wt%. In aspects, the A2A receptor antagonist is present in an amount of about 5 wt% to about 35 wt%. In aspects, the A2A receptor antagonist is present in an amount of about 5 wt% to about 30 wt%. In aspects, the A2A receptor antagonist is present in an amount of about 5 wt% to about 25 wt%. In aspects, the A2A receptor antagonist is present in an amount of about 10 wt% to about 30 wt%. In aspects, the A2A receptor antagonist is present in an amount of about 10 wt% to about 25 wt%. In aspects, the A2A receptor antagonist is present in an amount of about 15 wt% to about 25 wt%. In aspects, the A2A receptor antagonist is present in an amount of about 15 wt%. In aspects, the A2A receptor antagonist is present in an amount of about 20 wt%. In aspects, the A2A receptor antagonist is present in an amount of about 25 wt%.

In aspects of the amorphous solid dispersions, compositions, microprecipitated bulk powders, and oral formulations described herein, the pharmaceutically acceptable polymer is present in an amount of about 50 wt% to about 95 wt%. In aspects, the pharmaceutically acceptable polymer is present in an amount of about 60 wt% to about 95 wt%. In aspects, the pharmaceutically acceptable polymer is present in an amount of about 70 wt% to about 95 wt%. In aspects, the pharmaceutically acceptable polymer is present in an amount of about 75 wt% to about 95 wt%. In aspects, the pharmaceutically acceptable polymer is present in an amount of about 50 wt% to about 90 wt%. In aspects, the pharmaceutically acceptable polymer is present in an amount of about 50 wt% to about 80 wt%. In aspects, the pharmaceutically acceptable polymer is present in an amount of about 60 wt% to about 90 wt%. In aspects, the pharmaceutically acceptable polymer is present in an amount of about 60 wt% to about 80 wt%. In aspects, the pharmaceutically acceptable polymer is present in an amount of about 70 wt% to about 80 wt%. In aspects, the pharmaceutically acceptable polymer is present in an amount of about 75 wt% to about 85 wt%. In aspects, the pharmaceutically acceptable polymer is present in an amount of about 75 wt% to about 90 wt%. In aspects, the pharmaceutically acceptable polymer is present in an amount of about 70 wt%. In aspects, the pharmaceutically acceptable polymer is present in an amount of about 80 wt%.

In aspects of the compositions described herein, the adenosine A2A receptor antagonist is present in an amount of about 5 wt% to about 30 wt% and the pharmaceutically acceptable polymer is present in an amount of about 70 wt% to about 95 wt%. In other aspects, the A2A receptor antagonist is present in an amount of about 5 wt% to about 25 wt% and the pharmaceutically acceptable polymer is present in an amount of about 75 wt% to about 95 wt%. In aspects, the A2A receptor antagonist is present in an amount of about 10 wt% to about 30 wt% and the pharmaceutically acceptable polymer is present in an amount of about 70 wt% to about 90 wt%. In aspects, the A2A receptor antagonist is present in an amount of about 10 wt% to about 25 wt% and the pharmaceutically acceptable polymer is present in an amount of about 75 wt% to about 90 wt%. In aspects, the A2A receptor antagonist is present in an amount of about 15 wt% to about 25 wt% and the pharmaceutically acceptable polymer is present in an amount of about 75 wt% to about 85 wt%. In aspects, the A2A receptor antagonist is present in an amount of about 20 wt% and the pharmaceutically acceptable polymer is present in an amount of about 80 wt%.

In embodiment of the compositions provided herein, the composition includes no other active pharmaceutical ingredients other than one or more A2A receptor antagonists. In aspect of the compositions provided herein, the composition includes only a single type of A2A receptor antagonist. In aspects of the compositions provided herein, the composition include only a single type of A2A receptor antagonist and no other active pharmaceutical ingredient. In aspects of the compositions provided herein, the composition includes only a single type of A2A receptor antagonist and no pharmaceutical excipients or other active pharmaceutical ingredients.

### Pharmaceutical Formulations of Embodiment N

In embodiments, the disclosure provides pharmaceutical compositions comprising (i) drug nanoparticles and (ii) a pharmaceutically acceptable excipient; wherein the drug nanoparticles comprise an adenosine A2A receptor antagonist. In aspects, the disclosure provides pharmaceutical compositions comprising (i) drug nanoparticles and (ii) a pharmaceutically acceptable polymer; wherein the drug nanoparticles comprise an adenosine A2A receptor antagonist. In aspects, the disclosure provides pharmaceutical compositions comprising (i) drug nanoparticles and (ii) a surfactant; wherein the drug nanoparticles comprise an adenosine A2A receptor antagonist. In aspects, the disclosure provides pharmaceutical compositions comprising (i) drug nanoparticles, (ii) a pharmaceutically acceptable polymer, and (iii) a surfactant; wherein the drug nanoparticles comprise an adenosine A2A receptor antagonist. In aspects, the adenosine A2A receptor antagonist is crystalline.

In embodiments, the disclosure provides granules comprising (i) drug nanoparticles and (ii) a pharmaceutically acceptable excipient; wherein the drug nanoparticles comprise an adenosine A2A receptor antagonist. In aspects, the disclosure provides granules comprising (i) drug nanoparticles and (ii) a pharmaceutically acceptable polymer; wherein the drug nanoparticles comprise an adenosine A2A receptor antagonist. In aspects, the disclosure provides granules comprising (i) drug nanoparticles and (ii) a surfactant; wherein the drug nanoparticles comprise an adenosine A2A receptor antagonist. In aspects, the disclosure provides granules comprising (i) drug nanoparticles, (ii) a pharmaceutically acceptable polymer, and (iii) a surfactant; wherein the drug nanoparticles comprise an adenosine A2A receptor antagonist. In aspects, the adenosine A2A receptor antagonist is crystalline.

In embodiments, the disclosure provides powders comprising (i) drug nanoparticles and (ii) a pharmaceutically acceptable excipient; wherein the drug nanoparticles comprise an adenosine A2A receptor antagonist. In aspects, the disclosure provides powders comprising (i) drug nanoparticles and (ii) a pharmaceutically acceptable polymer; wherein the drug nanoparticles comprise an adenosine A2A receptor antagonist. In aspects, the disclosure provides powders comprising (i) drug nanoparticles and (ii) a surfactant; wherein the drug nanoparticles comprise an adenosine A2A receptor antagonist. In aspects, the disclosure provides powders comprising (i) drug nanoparticles, (ii) a pharmaceutically acceptable polymer, and (iii) a surfactant; wherein the drug nanoparticles comprise an adenosine A2A receptor antagonist. In aspects, the adenosine A2A receptor antagonist is crystalline.

In embodiments, the disclosure provides tablets comprising (i) drug nanoparticles and (ii) a pharmaceutically acceptable excipient; wherein the drug nanoparticles comprise an adenosine A2A receptor antagonist. In aspects, the disclosure provides tablets comprising (i) drug nanoparticles and (ii) a pharmaceutically acceptable polymer; wherein the drug nanoparticles comprise an adenosine A2A receptor antagonist. In aspects, the disclosure provides tablets comprising (i) drug nanoparticles and (ii) a surfactant; wherein the drug nanoparticles comprise an adenosine A2A receptor antagonist. In aspects, the disclosure provides tablets comprising (i) drug nanoparticles, (ii) a pharmaceutically acceptable polymer, and (iii) a surfactant; wherein the drug nanoparticles comprise an adenosine A2A receptor antagonist. In aspects, the adenosine A2A receptor antagonist is crystalline.

In embodiments, the disclosure provides beads comprising an inert core and an outer layer; wherein the outer layer comprises: (i) drug nanoparticles and (ii) a pharmaceutically acceptable excipient; wherein the drug nanoparticles comprise an adenosine A2A receptor antagonist. In aspects, the disclosure provides beads comprising an inert core and an outer layer; wherein the outer layer comprises: (i) drug nanoparticles and (ii) a pharmaceutically acceptable polymer; wherein the drug nanoparticles comprise an adenosine A2A receptor antagonist. In aspects, the disclosure provides beads comprising an inert core and an outer layer; wherein the outer layer comprises: (i) drug nanoparticles and (ii) a surfactant; wherein the drug nanoparticles comprise an adenosine A2A receptor antagonist. In aspects, the disclosure provides beads comprising an inert core and an outer layer; wherein the outer layer comprises: (i) drug nanoparticles, (ii) a pharmaceutically acceptable polymer, and (iii) a surfactant; wherein the drug nanoparticles comprise an adenosine A2A receptor antagonist. In aspects, the adenosine A2A receptor antagonist is crystalline.

In embodiments, the disclosure provides oral formulations comprising (i) drug nanoparticles and (ii) a pharmaceutically acceptable excipient; wherein the drug nanoparticles comprise an adenosine A2A receptor antagonist. In aspects, the disclosure provides oral formulations comprising (i) drug nanoparticles and (ii) a pharmaceutically acceptable polymer; wherein the drug nanoparticles comprise an adenosine A2A receptor antagonist. In aspects, the disclosure provides oral formulations comprising (i) drug nanoparticles and (ii) a surfactant; wherein the drug nanoparticles comprise an adenosine A2A receptor antagonist. In aspects, the disclosure provides oral formulations comprising (i) drug nanoparticles, (ii) a pharmaceutically acceptable polymer, and (iii) a surfactant; wherein the drug nanoparticles comprise an adenosine A2A receptor antagonist. In aspects, the adenosine A2A receptor antagonist is crystalline.

In embodiments of the pharmaceutical compositions, granules, tablets, powders, oral formulations, and beads described herein, the adenosine A2A receptor antagonist is a compound of Formula (I) or a pharmaceutically acceptable salt thereof. In aspects, the compound of Formula (I) is in free base form. In aspects, the compound of Formula (I) is in the form of a pharmaceutically acceptable salt. In aspects, the compound of Formula (I) is crystalline. In aspects, the adenosine A2A receptor antagonist is a compound of Formula (II) or a pharmaceutically acceptable salt thereof. In aspects, the compound of Formula (II) is in free base form. In aspects, the compound of Formula (II) is in the form of a pharmaceutically acceptable salt. In aspects, the compound of Formula (II) is crystalline.

In embodiments of the pharmaceutical compositions, granules, tablets, powders, oral formulations, and beads described herein, the adenosine A2A receptor antagonist is a compound of Formula (III) or a pharmaceutically acceptable salt thereof. In aspects, the compound of Formula (III) is in the form of a pharmaceutically acceptable salt. In aspects, the compound of Formula (III) is in free base form. In aspects, the compound of Formula (III) is crystalline. In aspects, the adenosine A2A receptor antagonist is a compound of Formula (IIIA) or a pharmaceutically acceptable salt thereof. In aspects, the compound of Formula (IIIA) is in free base form. In aspects, the compound of Formula (IIIA) is in the form of a pharmaceutically acceptable salt. In aspects, the compound of Formula (IIIA) is crystalline. In aspects, the adenosine A2A receptor antagonist is a compound of Formula (IIIB) or a pharmaceutically acceptable salt thereof. In aspects, the compound of Formula (IIIB) is in free base form. In aspects, the compound of Formula (IIIB) is in the form of a pharmaceutically acceptable salt. In aspects, the compound of Formula (IIIB) is crystalline. In aspects, the adenosine A2A receptor antagonist is a mixture of a compound of Formula (IIIA) or a pharmaceutically acceptable salt thereof and a compound of Formula (IIIB) or a pharmaceutically acceptable salt thereof. In aspects, the adenosine A2A receptor antagonist is a racemic mixture of a compound of Formula (IIIA) or a pharmaceutically acceptable salt thereof and a compound of Formula (IIIB) or a pharmaceutically acceptable salt thereof.

In embodiments of the pharmaceutical compositions, granules, tablets, powders, oral formulations, and beads described herein, the drug nanoparticles have a particle size distribution with a D90 of about 1000 nm or less, as measured by laser diffraction spectroscopy. In aspects, the drug nanoparticles have a particle size distribution with a D90 of about 975 nm or less. In aspects, the particle size distribution with a D90 is about 950 nm or less. In aspects, the particle size distribution with a D90 is about 925 nm or less. In aspects, the particle size distribution with a D90 is about 900 nm or less. In aspects, the particle size distribution with a D90 is about 875 nm or less. In aspects, the particle size distribution with a D90 is about 850 nm or less. In aspects, the particle size distribution with a D90 is about 825 nm or less. In aspects, the particle size distribution with a D90 is about 800 nm or less. In aspects, the particle size distribution with a D90 is about 775 nm or less. In aspects, the particle size distribution with a D90 is about 750 nm or less. In aspects, the particle size distribution with a D90 is about 725 nm or less. In aspects, the particle size distribution with a D90 is about 700 nm or less. In aspects, the particle size distribution with a D90 is about 675 nm or less. In aspects, the particle size distribution with a D90 is about 650 nm or less. In aspects, the particle size distribution with a D90 is about 625 nm or less. In aspects, the particle size distribution with a D90 is about 600 nm or less. In aspects, the particle size distribution with a D90 is about 575 nm or less. In aspects, the particle size distribution with a D90 is about 550 nm or less. In aspects, the particle size distribution with a D90 is about 525 nm or less. In aspects, the particle size distribution with a D90 is about 500 nm or less. In aspects, the particle size distribution with a D90 is about 490 nm or less. In aspects, the particle size distribution with a D90 is about 480 nm or less. In aspects, the particle size distribution with a D90 is about 470 nm or less. In aspects, the particle size distribution with a D90 is about 460 nm or less. In aspects, the particle size distribution with a D90 is about 450 nm or less. In aspects, the particle size distribution with a D90 is about 440 nm or less. In aspects, the particle size distribution with a D90 is about 430 nm or less. In aspects, the particle size distribution with a D90 is about 420 nm or less. In aspects, the particle size distribution with a D90 is about 410 nm or less. In aspects, the particle size distribution with a D90 is about 400 nm or less. In aspects, the particle size distribution with a D90 is about 390 nm or less. In aspects, the particle size distribution with a D90 is about 380 nm or less. In aspects, the particle size distribution with a D90 is about 370 nm or less. In aspects, the particle size distribution with a D90 is about 360 nm or less. In aspects, the particle size distribution with a D90 is about 350 nm or less. In aspects, the particle size distribution with a D90 is about 340 nm or less. In aspects, the particle size distribution with a D90 is about 330 nm or less. In aspects, the particle size distribution with a D90 is about 320 nm or less. In aspects, the particle size distribution with a D90 is about 310 nm or less. In aspects, the particle size distribution with a D90 is about 300 nm or less. In aspects, the particle size distribution with a D90 is about 290 nm or less. In aspects, the particle size distribution with a D90 is about 280 nm or less. In aspects, the particle size distribution with a D90 is about 270 nm or less. In aspects, the particle size distribution with a D90 is about 260 nm or less. In aspects, the particle size distribution with a D90 is about 250 nm or less. In aspects, the particle size distribution with a D90 is about 240 nm or less. In aspects, the particle size distribution with a D90 is about 230 nm or less. In aspects, the particle size distribution with a D90 is about 220 nm or less. In aspects, the particle size distribution with a D90 is about 210 nm or less. In aspects, the particle size distribution with a D90 is about 200 nm or less. In aspects, the particle size distribution with a D90 is about 195 nm or less. In aspects, the particle size distribution with a D90 is about 190 nm or less. In aspects, the particle size distribution with a D90 is about 185 nm or less. In aspects, the particle size distribution with a D90 is about 180 nm or less. In aspects, the particle size distribution with a D90 is about 175 nm or less. In aspects, the particle size distribution with a D90 is about 170 nm or less. In aspects, the particle size distribution with a D90 is about 165 nm or less. In aspects, the particle size distribution with a D90 is about 160 nm or less. In aspects, the particle size distribution with a D90 is about 155 nm or less. In aspects, the particle size distribution with a D90 is about 150 nm or less. In aspects, the particle size distribution with a D90 is about 145 nm or less. In aspects, the particle size distribution with a D90 is about 140 nm or less. In aspects, the particle size distribution with a D90 is about 135 nm or less. In aspects, the particle size distribution with a D90 is about 130 nm or less. In aspects, the particle size distribution with a D90 is about 125 nm or less. In aspects, the particle size distribution with a D90 is about 120 nm or less. In aspects, the particle size distribution with a D90 is about 115 nm or less. In aspects, the particle size distribution with a D90 is about 110 nm or less. In aspects, the particle size distribution with a D90 is about 105 nm or less. In aspects, the particle size distribution with a D90 is about 100 nm or less. In aspects, the particle size distribution with a D90 is about 95 nm or less. In aspects, the particle size distribution with a D90 is about 90 nm or less. In aspects, the particle size distribution with a D90 is about 85 nm or less. In aspects, the particle size distribution with a D90 is about 80 nm or less. In aspects, the particle size distribution with a D90 is about 75 nm or less. The particle size distribution of the drug particles described herein is measured by laser diffraction spectroscopy.

In embodiments of the pharmaceutical compositions, granules, tablets, powders, oral formulations, and beads described herein, the adenosine A2A receptor antagonist is present in an effective amount. In aspects, the adenosine A2A receptor antagonist is present in an amount of about 1 wt% to about 50 wt%. In aspects, the adenosine A2A receptor antagonist is present in an amount of about 5 wt% to about 50 wt%. In aspects, the adenosine A2A receptor antagonist is present in an amount of about 10 wt% to about 50 wt%. In aspects, the adenosine A2A receptor antagonist is present in an amount of about 15 wt% to about 45 wt%. In aspects, the adenosine A2A receptor antagonist is present in an amount of about 20 wt% to about 40 wt%. In aspects, the adenosine A2A receptor antagonist is present in an amount of about 21 wt% to about 39 wt%. In aspects, the adenosine A2A receptor antagonist is present in an amount of about 22 wt% to about 38 wt%. In aspects, the adenosine A2A receptor antagonist is present in an amount of about 23 wt% to about 37 wt%. In aspects, the adenosine A2A receptor antagonist is present in an amount of about 24 wt% to about 36 wt%. In aspects, the adenosine A2A receptor antagonist is present in an amount of about 25 wt% to about 35 wt%. In aspects, the adenosine A2A receptor antagonist is present in an amount of about 26 wt% to about 34 wt%. In aspects, the adenosine A2A receptor antagonist is present in an amount of about 27 wt% to about 33 wt%. In aspects, the adenosine A2A receptor antagonist is present in an amount of about 28 wt% to about 32 wt%. In aspects, the adenosine A2A receptor antagonist is present in an amount of about 29 wt% to about 31 wt%. In aspects, the adenosine A2A receptor antagonist is present in an amount of about 20 wt%. In aspects, the adenosine A2A receptor antagonist is present in an amount of about 21 wt%. In aspects, the adenosine A2A receptor antagonist is present in an amount of about 22 wt%. In aspects, the adenosine A2A receptor antagonist is present in an amount of about 23 wt%. In aspects, the adenosine A2A receptor antagonist is present in an amount of about 24 wt%. In aspects, the adenosine A2A receptor antagonist is present in an amount of about 25 wt%. In aspects, the adenosine A2A receptor antagonist is present in an amount of about 26 wt%. In aspects, the adenosine A2A receptor antagonist is present in an amount of about 27 wt%. In aspects, the adenosine A2A receptor antagonist is present in an amount of about 28 wt%. In aspects, the adenosine A2A receptor antagonist is present in an amount of about 29 wt%. In aspects, the adenosine A2A receptor antagonist is present in an amount of about 30 wt%. In aspects, the adenosine A2A receptor antagonist is present in an amount of about 31 wt%. In aspects, the adenosine A2A receptor antagonist is present in an amount of about 32 wt%. In aspects, the adenosine A2A receptor antagonist is present in an amount of about 33 wt%. In aspects, the adenosine A2A receptor antagonist is present in an amount of about 34 wt%. In aspects, the adenosine A2A receptor antagonist is present in an amount of about 35 wt%. In aspects, the adenosine A2A receptor antagonist is present in an amount of about 36 wt%. In aspects, the adenosine A2A receptor antagonist is present in an amount of about 37 wt%. In aspects, the adenosine A2A receptor antagonist is present in an amount of about 38 wt%. In aspects, the adenosine A2A receptor antagonist is present in an amount of about 39 wt%. In aspects, the adenosine A2A receptor antagonist is present in an amount of about 40 wt%.

In embodiments of the pharmaceutical compositions, granules, tablets, powders, oral formulations, and beads described herein, the drug nanoparticles are present in an effective weight ratio to the pharmaceutically acceptable excipient, where the pharmaceutically acceptable excipient is a pharmaceutically acceptable polymer, a surfactant, or a combination thereof. In aspects, the weight ratio of drug nanoparticles to pharmaceutically acceptable excipient is from about 30:1 to about 1:1. In aspects, the weight ratio of drug nanoparticles to pharmaceutically acceptable excipient is from about 25:1 to about 1:1. In aspects, the weight ratio of drug nanoparticles to pharmaceutically acceptable excipient is from about 20:1 to about 2:1. In aspects, the weight ratio of drug nanoparticles to pharmaceutically acceptable excipient is from about 15:1 to about 2:1. In aspects, the weight ratio of drug nanoparticles to pharmaceutically acceptable excipient is from about 10:1 to about 2:1. In aspects, the weight ratio of drug nanoparticles to pharmaceutically acceptable excipient is from about 5:1 to about 2:1.

In embodiments of the pharmaceutical compositions, granules, tablets, powders, oral formulations, and beads described herein, the surfactant comprises (i) an anionic surfactant, (ii) a cationic surfactant, (iii) a nonionic surfactant, (iv) a zwitterionic surfactant, or (v) a combination of two or more of the foregoing. In aspects, the surfactant comprises an anionic surfactant. In aspects, the surfactant comprises a cationic surfactant. In aspects, the surfactant comprises a nonionic surfactant. In aspects, the nonionic surfactant is a poloxamer. In aspects, the surfactant comprises a zwitterionic surfactant. In aspects, the anionic surfactant is a carboxylate, a sulfate, a sulfonate, a phosphate ester, a bile salt, or a combination of two or more thereof. In aspects, the anionic surfactant is sodium lauryl sulfate, ammonium lauryl sulfate, sodium laureth sulfate, sodium myreth sulfate, sodium stearate, dioctyl sodium sulfosuccinate, or a combination of two or more thereof. In aspects, the anionic surfactant is sodium lauryl sulfate. In aspects, the anionic surfactant is dioctyl sodium sulfosuccinate. In aspects, the anionic surfactant is cholic acid, deoxycholic acid, glycocholic acid, taurocholic acid, glycodeoxycholic acid, taurodeoxycholic acid, ursocholic acid, ursodeoxycholic acid, chenodeoxycholic acid, glycochenodeoxycholic acid, taurochenodeoxycholic acid, oleic acid, or a combination of two or more thereof. In aspects, the anionic surfactant is an aliphatic sulfonate, a C₅-C₂₅ alkane sulfonate, a C₅-C₂₅ alkyl disulfonates, a C₅-C₂₅ alkene sulfonates, a C₅-C₂₅ hydroxyalkane sulfonate, an alkyl glyceryl ether sulfonate, an aromatic sulfonate, a sodium C₁₀-C₂₀ olefin sulfonate, a phosphonic acids, or a combination of two or more thereof.

In embodiments of the pharmaceutical compositions, granules, tablets, powders, oral formulations, and beads described herein, the surfactant is present in an effective amount. In aspects, the surfactant is present in an amount from about 0.005 wt% to about 10.0 wt%. In aspects, the surfactant is present in an amount from about 0.005 wt% to about 9.0 wt%. In aspects, the surfactant is present in an amount from about 0.005 wt% to about 8.0 wt%. In aspects, the surfactant is present in an amount from about 0.005 wt% to about 7.0 wt% In aspects, the surfactant is present in an amount from about 0.005 wt% to about 6.0 wt%. In aspects, the surfactant is present in an amount from about 0.005 wt% to about 5.0 wt%. In aspects, the surfactant is present in an amount from about 0.005 wt% to about 4.5 wt%. In aspects, the surfactant is present in an amount from about 0.005 wt% to about 4.0 wt%. In aspects, the surfactant is present in an amount from about 0.005 wt% to about 3.5 wt%. In aspects, the surfactant is present in an amount from about 0.005 wt% to about 3.0 wt%. In aspects, the surfactant is present in an amount from about 0.005 wt% to about 2.5 wt%. In aspects, the surfactant is present in an amount from about 0.005 wt% to about 2.0 wt%. In aspects, the surfactant is present in an amount from about 0.005 wt% to about 1.9 wt%. In aspects, the surfactant is present in an amount from about 0.005 wt% to about 1.8 wt%. In aspects, the surfactant is present in an amount from about 0.005 wt% to about 1.7 wt%. In aspects, the surfactant is present in an amount from about 0.005 wt% to about 1.6 wt%. In aspects, the surfactant is present in an amount from about 0.005 wt% to about 1.5 wt%. In aspects, the surfactant is present in an amount from about 0.005 wt% to about 1.4 wt%. In aspects, the surfactant is present in an amount from about 0.005 wt% to about 1.3 wt%. In aspects, the surfactant is present in an amount from about 0.005 wt% to about 1.2 wt%. In aspects, the surfactant is present in an amount from about 0.005 wt% to about 1.1 wt%. In aspects, the surfactant is present in an amount from about 0.005 wt% to about 1.0 wt%. In aspects, the surfactant is present in an amount from about 0.005 wt% to about 0.9 wt%. In aspects, the surfactant is present in an amount from about 0.005 wt% to about 0.8 wt%. In aspects, the surfactant is present in an amount from about 0.005 wt% to about 0.7 wt% In aspects, the surfactant is present in an amount from about 0.005 wt% to about 0.6 wt%. In aspects, the surfactant is present in an amount from about 0.005 wt% to about 0.5 wt%. In aspects, the surfactant is present in an amount from about 0.01 wt% to about 5.0 wt%. In aspects, the surfactant is present in an amount from about 0.01 wt% to about 4.5 wt%. In aspects, the surfactant is present in an amount from about 0.01 wt% to about 4.0 wt%. In aspects, the surfactant is present in an amount from about 0.01 wt% to about 3.5 wt%. In aspects, the surfactant is present in an amount from about 0.01 wt% to about 3.0 wt%. In aspects, the surfactant is present in an amount from about 0.01 wt% to about 2.5 wt%. In aspects, the surfactant is present in an amount from about 0.01 wt% to about 2.0 wt%. In aspects, the surfactant is present in an amount from about 0.01 wt% to about 1.9 wt%. In aspects, the surfactant is present in an amount from about 0.01 wt% to about 1.8 wt%. In aspects, the surfactant is present in an amount from about 0.01 wt% to about 1.7 wt%. In aspects, the surfactant is present in an amount from about 0.01 wt% to about 1.6 wt%. In aspects, the surfactant is present in an amount from about 0.01 wt% to about 1.5 wt%. In aspects, the surfactant is present in an amount from about 0.01 wt% to about 1.4 wt%. In aspects, the surfactant is present in an amount from about 0.01 wt% to about 1.3 wt%. In aspects, the surfactant is present in an amount from about 0.01 wt% to about 1.2 wt%. In aspects, the surfactant is present in an amount from about 0.01 wt% to about 1.1 wt%. In aspects, the surfactant is present in an amount from about 0.01 wt% to about 1.0 wt%. In aspects, the surfactant is present in an amount from about 0.01 wt% to about 0.9 wt%. In aspects, the surfactant is present in an amount from about 0.01 wt% to about 0.8 wt%. In aspects, the surfactant is present in an amount from about 0.01 wt% to about 0.7 wt%. In aspects, the surfactant is present in an amount from about 0.01 wt% to about 0.6 wt%. In aspects, the surfactant is present in an amount from about 0.01 wt% to about 0.5 wt%. In aspects, the surfactant is present in an amount from about 0.05 wt% to about 5.0 wt%. In aspects, the surfactant is present in an amount from about 0.05 wt% to about 4.5 wt%. In aspects, the surfactant is present in an amount from about 0.05 wt% to about 4.0 wt%. In aspects, the surfactant is present in an amount from about 0.05 wt% to about 3.5 wt%. In aspects, the surfactant is present in an amount from about 0.05 wt% to about 3.0 wt%. In aspects, the surfactant is present in an amount from about 0.05 wt% to about 2.5 wt%. In aspects, the surfactant is present in an amount from about 0.05 wt% to about 2.0 wt%. In aspects, the surfactant is present in an amount from about 0.05 wt% to about 1.9 wt%. In aspects, the surfactant is present in an amount from about 0.05 wt% to about 1.8 wt%. In aspects, the surfactant is present in an amount from about 0.05 wt% to about 1.7 wt%. In aspects, the surfactant is present in an amount from about 0.05 wt% to about 1.6 wt%. In aspects, the surfactant is present in an amount from about 0.05 wt% to about 1.5 wt%. In aspects, the surfactant is present in an amount from about 0.05 wt% to about 1.4 wt%. In aspects, the surfactant is present in an amount from about 0.05 wt% to about 1.3 wt%. In aspects, the surfactant is present in an amount from about 0.05 wt% to about 1.2 wt%. In aspects, the surfactant is present in an amount from about 0.05 wt% to about 1.1 wt%. In aspects, the surfactant is present in an amount from about 0.05 wt% to about 1.0 wt%. In aspects, the surfactant is present in an amount from about 0.05 wt% to about 0.9 wt%. In aspects, the surfactant is present in an amount from about 0.05 wt% to about 0.8 wt%. In aspects, the surfactant is present in an amount from about 0.05 wt% to about 0.7 wt%. In aspects, the surfactant is present in an amount from about 0.05 wt% to about 0.6 wt%. In aspects, the surfactant is present in an amount from about 0.05 wt% to about 0.5 wt%. In aspects, the surfactant is present in an amount from about 0.1 wt% to about 0.5 wt%.

In embodiments of the pharmaceutical compositions, granules, tablets, powders, oral formulations, and beads described herein, the pharmaceutically acceptable polymer is any known in the art. In aspects, the pharmaceutically acceptable polymer is a non-crosslinked or crosslinked polymer. In aspects, the pharmaceutically acceptable polymer comprises (i) a polyvinylpyrrolidone polymer, (ii) a polyvinylpyrrolidone copolymer, (iii) a cellulose compound, or (iv) a combination of two or more of the thereof. In aspects, the pharmaceutically acceptable polymer comprises (i) a non-crosslinked polyvinylpyrrolidone polymer, (ii) a non-crosslinked polyvinylpyrrolidone copolymer, (iii) a non-crosslinked cellulose compound, or (iv) a combination of two or more of the thereof. In aspects, the pharmaceutically acceptable polymer comprises a polyvinylpyrrolidone polymer. In aspects, the polyvinylpyrrolidone polymer has an average molecular weight from about 1,000 Daltons to about 1,500,000 Daltons; or from about 1,000 Daltons to about 1,000,000 Daltons; or from about 1,000 Daltons to about 500,000 Daltons; or from about 1,000 Daltons to about 200,000. In aspects, the polyvinylpyrrolidone polymer has an average molecular weight from about 1,000 Daltons to about 150,000 Daltons. In aspects, the polyvinylpyrrolidone polymer has an average molecular weight from about 10,000 Daltons to about 150,000 Daltons. In aspects, the polyvinylpyrrolidone polymer has an average molecular weight from about 50,000 Daltons to about 150,000 Daltons. In aspects, the polyvinylpyrrolidone polymer has an average molecular weight from about 60,000 Daltons to about 120,000 Daltons. In aspects, the pharmaceutically acceptable polymer comprises a polyvinylpyrrolidone copolymer. In aspects, the polyvinylpyrrolidone copolymer is a copolymer comprising vinylpyrrolidone and an acrylic monomer. In aspects, the polyvinylpyrrolidone copolymer is a copolymer comprising vinylpyrrolidone and styrene. In aspects, the polyvinylpyrrolidone copolymer is a copolymer comprising vinylpyrrolidone and vinyl acetate. In aspects, the polyvinylpyrrolidone copolymer is a copolymer comprising vinylpyrrolidone and vinyl acetate, where the weight ratio of vinylpyrrolidone to vinyl acetate is from about 30:70 to about 70:30. In aspects, the polyvinylpyrrolidone copolymer is a copolymer comprising vinylpyrrolidone and vinyl acetate, where the weight ratio of vinylpyrrolidone to vinyl acetate is about 3:2. In aspects, the polyvinylpyrrolidone copolymer is copovidone. In aspects, the pharmaceutically acceptable polymer comprises a cellulose compound. In aspects, the cellulose compound is a hydroxyalkyl cellulose compound, an alkyl cellulose compound, or a combination thereof. In aspects, the cellulose compound is hydroxypropyl cellulose, hydroxyethyl cellulose, hydroxyethylmethyl cellulose, hydroxyethyl cellulose, hydroxypropylmethyl cellulose, methyl cellulose, ethylmethyl cellulose, carboxymethyl cellulose, or a combination of two or more thereof. In aspects, the cellulose compound is hydroxypropyl cellulose. In aspects, the cellulose compound is hydroxypropylmethyl cellulose. In aspects, the cellulose compound is hydroxypropyl methylcellulose acetate succinate. In aspects, the polymers are not crosslinked. In aspects the polymer is crosslinked.

In embodiments of the pharmaceutical compositions, granules, tablets, powders, oral formulations, and beads described herein, the pharmaceutically acceptable polymer is present in an effective amount. In aspects, the pharmaceutically acceptable polymer is a crosslinked non-crosslinked polymer. In aspects, the pharmaceutically acceptable polymer is present in an amount from about 0.01 wt% to about 30 wt% of the pharmaceutically acceptable polymer. In aspects, the pharmaceutically acceptable polymer is present in an amount from about 0.1 wt% to about 30 wt% of the pharmaceutically acceptable polymer. In aspects, the pharmaceutically acceptable polymer is present in an amount from about 0.1 wt% to about 25 wt% of the pharmaceutically acceptable polymer. In aspects, the pharmaceutically acceptable polymer is present in an amount from about 0.1 wt% to about 20 wt% of the pharmaceutically acceptable polymer. In aspects, the pharmaceutically acceptable polymer is present in an amount from about 0.1 wt% to about 15 wt% of the pharmaceutically acceptable polymer. In aspects, the pharmaceutically acceptable polymer is present in an amount from about 0.1 wt% to about 14 wt% of the pharmaceutically acceptable polymer. In aspects, the pharmaceutically acceptable polymer is present in an amount from about 0.1 wt% to about 13 wt% of the pharmaceutically acceptable polymer. In aspects, the pharmaceutically acceptable polymer is present in an amount from about 0.1 wt% to about 12 wt% of the pharmaceutically acceptable polymer. In aspects, the pharmaceutically acceptable polymer is present in an amount from about 0.1 wt% to about 11 wt% of the pharmaceutically acceptable polymer. In aspects, the pharmaceutically acceptable polymer is present in an amount from about 0.1 wt% to about 10 wt% of the pharmaceutically acceptable polymer. In aspects, the pharmaceutically acceptable polymer is present in an amount from about 0.1 wt% to about 9 wt% of the pharmaceutically acceptable polymer. In aspects, the pharmaceutically acceptable polymer is present in an amount from about 0.1 wt% to about 8 wt% of the pharmaceutically acceptable polymer. In aspects, the pharmaceutically acceptable polymer is present in an amount from about 0.1 wt% to about 7 wt% of the pharmaceutically acceptable polymer. In aspects, the pharmaceutically acceptable polymer is present in an amount from about 0.1 wt% to about 6 wt% of the pharmaceutically acceptable polymer. In aspects, the pharmaceutically acceptable polymer is present in an amount from about 0.1 wt% to about 5 wt% of the pharmaceutically acceptable polymer. In aspects, the pharmaceutically acceptable polymer is present in an amount from about 0.5 wt% to about 30 wt% of the pharmaceutically acceptable polymer. In aspects, the pharmaceutically acceptable polymer is present in an amount from about 0.5 wt% to about 25 wt% of the pharmaceutically acceptable polymer. In aspects, the pharmaceutically acceptable polymer is present in an amount from about 0.5 wt% to about 20 wt% of the pharmaceutically acceptable polymer. In aspects, the pharmaceutically acceptable polymer is present in an amount from about 0.5 wt% to about 15 wt% of the pharmaceutically acceptable polymer. In aspects, the pharmaceutically acceptable polymer is present in an amount from about 0.5 wt% to about 14 wt% of the pharmaceutically acceptable polymer. In aspects, the pharmaceutically acceptable polymer is present in an amount from about 0.5 wt% to about 13 wt% of the pharmaceutically acceptable polymer. In aspects, the pharmaceutically acceptable polymer is present in an amount from about 0.5 wt% to about 12 wt% of the pharmaceutically acceptable polymer. In aspects, the pharmaceutically acceptable polymer is present in an amount from about 0.5 wt% to about 11 wt% of the pharmaceutically acceptable polymer. In aspects, the pharmaceutically acceptable polymer is present in an amount from about 0.5 wt% to about 10 wt% of the pharmaceutically acceptable polymer. In aspects, the pharmaceutically acceptable polymer is present in an amount from about 0.5 wt% to about 9 wt% of the pharmaceutically acceptable polymer. In aspects, the pharmaceutically acceptable polymer is present in an amount from about 0.5 wt% to about 8 wt% of the pharmaceutically acceptable polymer. In aspects, the pharmaceutically acceptable polymer is present in an amount from about 1 wt% to about 30 wt% of the pharmaceutically acceptable polymer. In aspects, the pharmaceutically acceptable polymer is present in an amount from about 1 wt% to about 25 wt% of the pharmaceutically acceptable polymer. In aspects, the pharmaceutically acceptable polymer is present in an amount from about 1 wt% to about 20 wt% of the pharmaceutically acceptable polymer. In aspects, the pharmaceutically acceptable polymer is present in an amount from about 1 wt% to about 15 wt% of the pharmaceutically acceptable polymer. In aspects, the pharmaceutically acceptable polymer is present in an amount from about 1 wt% to about 14 wt% of the pharmaceutically acceptable polymer. In aspects, the pharmaceutically acceptable polymer is present in an amount from about 1 wt% to about 13 wt% of the pharmaceutically acceptable polymer. In aspects, the pharmaceutically acceptable polymer is present in an amount from about 1 wt% to about 12 wt% of the pharmaceutically acceptable polymer. In aspects, the pharmaceutically acceptable polymer is present in an amount from about 1 wt% to about 11 wt% of the pharmaceutically acceptable polymer. In aspects, the pharmaceutically acceptable polymer is present in an amount from about 1 wt% to about 10 wt% of the pharmaceutically acceptable polymer. In aspects, the pharmaceutically acceptable polymer is present in an amount from about 1 wt% to about 9 wt% of the pharmaceutically acceptable polymer. In aspects, the pharmaceutically acceptable polymer is present in an amount from about 1 wt% to about 8 wt% of the pharmaceutically acceptable polymer.

In embodiment of the compositions provided herein, the composition includes no other active pharmaceutical ingredients other than one or more A2A receptor antagonists. In aspect of the compositions provided herein, the composition includes only a single type of A2A receptor antagonist. In aspects of the compositions provided herein, the composition include only a single type of A2A receptor antagonist and no other active pharmaceutical ingredient. In aspects of the compositions provided herein, the composition includes only a single type of A2A receptor antagonist and no pharmaceutical excipients or other active pharmaceutical ingredients.

### Oral Formulations: Pharmaceutical Compositions of Embodiments H, M, and N

In embodiments, the amorphous solid dispersions, compositions, extrudates, microprecipitated bulk powders, and crystalline nanoparticles described herein are oral formulations. Oral formulations include tablets (including chewable tablets), pills, powder, film, dragees, capsules, wafers, films, liquids, lozenges, sachets, stick packs, gels, syrups, slurries, suspensions, and the like suitable for ingestion by the patient. Solid oral formulations include powders, tablets, granules, beads, capsules, films, wafers, and dispersible granules. Liquid oral formulations include solutions, suspensions, and emulsions. Other oral formulations include oral mucosal formulations, such as sublingual or buccal formulations, such as tablets, films, or wafers.

In aspects, the oral formulation is a tablet comprising the amorphous solid dispersions, compositions, extrudates, microprecipitated bulk powders, and crystalline nanoparticles described herein described herein. In aspects, the tablet is a compressed tablet. In aspects, the tablet is encapsulated within an outer layer. In aspects, the tablet is encapsulated within an outer enteric coating. In aspects, the tablet is a rapidly-disintegrating tablet. In aspects, the tablet is a sublingual tablet or a buccal tablet. In aspects, the tablet is a rapidly-disintegrating sublingual or buccal tablet. In aspects, the tablet is formed by compressing the amorphous solid dispersions, the compositions, or the extrudate described herein.

In embodiments, the oral formulation is a capsule. In aspects, the capsule comprises the amorphous solid dispersions, compositions, extrudates, microprecipitated bulk powders, and crystalline nanoparticles described herein encapsulated within a capsule shell. In aspects, the capsule is encapsulated within an outer layer. In aspects, the capsule is encapsulated within an outer enteric coating. In aspects, the oral formulation is a solid capsule. In aspects, the capsule is a rapidly-disintegrating capsule.

In embodiments, the oral formulation is a powder, where the powder comprises the amorphous solid dispersions, compositions, extrudates, microprecipitated bulk powders, and crystalline nanoparticles described herein. In aspects, the oral formulation is a reconstitutable powder. A reconsititutable powder can be added to a liquid for ingestion, where the powder dissolves in the liquid or the powder forms a suspension in the liquid. In aspects, the powder is sprinkled on or in food for oral administration or administered directly to the patient in a measured amount.

In embodiments, the disclosure provides sachets containing the oral formulations, the amorphous solid dispersions, compositions, extrudates, microprecipitated bulk powders, and crystalline nanoparticles described herein. In aspects, the disclosure provides stick packs containing the oral formulations, amorphous solid dispersions, the compositions, or the microprecipitated bulk powders described herein. A sachet or a stick pack is a useful packaging material where the contents are intended to be sprinkled in food or added to a liquid.

In embodiments, the oral formulations described herein, including the tablets and capsules, comprise a coating. In aspects, the coating is an enteric coating. In aspects, the enteric coating surrounds the formulation, i.e., the enteric coating forms a layer where the formulation is enclosed within (surrounded by) the layer. In aspects, the enteric coating partially surrounds the formulation. In aspects, the oral formulation is a tablet comprising an enteric coating. In aspects, the oral formulation is a compressed tablet comprising an enteric coating. In aspects, the oral formulation is a capsule comprising an enteric coating. In aspects, the enteric coating comprises polymerized gelatin, shellac, methacrylic acid copolymers, cellulose butyrate phthalate, cellulose hydrogen phthalate, cellulose propionate phthalate, polyvinyl acetate phthalate, cellulose acetate phthalate, cellulose acetate trimellitate, hydroxypropyl methylcellulose phthalate, hydroxypropyl methylcellulose acetate, dioxypropyl methylcellulose succinate, carboxymethyl ethylcellulose, hydroxypropyl methylcellulose acetate succinate, and acrylic acid polymers and copolymers, typically formed from methyl acrylate, ethyl acrylate, methyl methacrylate and/or ethyl methacrylate with copolymers of acrylic and methacrylic acid esters (EUDRAGIT® NE, EUDRAGIT® RL, EUDRAGIT® RS). In aspects, the enteric coating comprises hydroxypropyl methylcellulose acetate succinate.

In aspects, the compositions, the amorphous solid dispersions, compositions, extrudates, microprecipitated bulk powders, crystalline nanoparticles, and oral formulations (e.g., tablets, capsules, granules, powders, and beads) described herein comprise a dose of the adenosine A2A receptor antagonist from about 1 mg to about 1,000 mg. In aspects, the dose of the adenosine A2A receptor antagonist is from about 1 mg to about 900 mg. In aspects, the dose of the adenosine A2A receptor antagonist is from about 1 mg to about 800 mg. In aspects, the dose of the adenosine A2A receptor antagonist is from about 1 mg to about 700 mg. In aspects, the dose of the adenosine A2A receptor antagonist is from about 1 mg to about 600 mg. In aspects, the dose of the adenosine A2A receptor antagonist is from about 1 mg to about 500 mg. In aspects, the dose of the adenosine A2A receptor antagonist is from about 1 mg to about 400 mg.

In aspects, the compositions, the amorphous solid dispersions, compositions, extrudates, microprecipitated bulk powders, crystalline nanoparticles, and oral formulations (e.g., tablets, capsules, granules, powders, and beads) described herein comprise a dose of the adenosine A2A receptor antagonist from about 1 mg to about 50 mg. In aspects, the dose of the adenosine A2A receptor antagonist is from about 5 mg to about 40 mg. In aspects, the dose of the adenosine A2A receptor antagonist is from about 5 mg to about 30 mg. In aspects, the dose of the adenosine A2A receptor antagonist is from about 5 mg to about 20 mg. In aspects, the dose of the adenosine A2A receptor antagonist is from about 5 mg to about 10 mg. In aspects, the dose of the adenosine A2A receptor antagonist is about 5 mg. In aspects, the dose of the adenosine A2A receptor antagonist is about 10 mg. In aspects, the dose of the adenosine A2A receptor antagonist is about 20 mg. In aspects, the dose of the adenosine A2A receptor antagonist is about 25 mg.

In aspects, the compositions, the amorphous solid dispersions, compositions, extrudates, microprecipitated bulk powders, crystalline nanoparticles, and oral formulations (e.g., tablets, capsules, granules, powders, and beads) described herein comprise a dose of the adenosine A2A receptor antagonist from about 20 mg to about 100 mg. In aspects, the dose of the adenosine A2A receptor antagonist is from about 25 mg to about 75 mg. In aspects, the dose of the adenosine A2A receptor antagonist is from about 30 mg to about 70 mg. In aspects, the dose of the adenosine A2A receptor antagonist is from about 40 mg to about 60 mg. In aspects, the dose of the adenosine A2A receptor antagonist is from about 45 mg to about 55 mg. In aspects, the dose of the adenosine A2A receptor antagonist is about 50 mg.

In aspects, the compositions, the amorphous solid dispersions, compositions, extrudates, microprecipitated bulk powders, crystalline nanoparticles, and oral formulations (e.g., tablets, capsules, granules, powders, and beads) described herein comprise a dose of the adenosine A2A receptor antagonist is from about 50 mg to about 150 mg. In aspects, the dose of the adenosine A2A receptor antagonist is from about 75 mg to about 125 mg. In aspects, the dose of the adenosine A2A receptor antagonist is from about 80 mg to about 120 mg. In aspects, the dose of the adenosine A2A receptor antagonist is from about 85 mg to about 115 mg. In aspects, the dose of the adenosine A2A receptor antagonist is from about 90 mg to about 110 mg. In aspects, the dose of the adenosine A2A receptor antagonist is from about 95 mg to about 105 mg. In aspects, the dose of the adenosine A2A receptor antagonist is about 100 mg.

In aspects, the compositions, the amorphous solid dispersions, compositions, extrudates, microprecipitated bulk powders, crystalline nanoparticles, and oral formulations (e.g., tablets, capsules, granules, powders, and beads) described herein comprise a dose of the adenosine A2A receptor antagonist from about 100 mg to about 200 mg. In aspects, the dose of the adenosine A2A receptor antagonist is from about 110 mg to about 190 mg. In aspects, the dose of the adenosine A2A receptor antagonist is from about 120 mg to about 180 mg. In aspects, the dose of the adenosine A2A receptor antagonist is from about 130 mg to about 170 mg. In aspects, the dose of the adenosine A2A receptor antagonist is from about 140 mg to about 160 mg. In aspects, the dose of the adenosine A2A receptor antagonist is about 150 mg.

In aspects, the compositions, the amorphous solid dispersions, compositions, extrudates, microprecipitated bulk powders, crystalline nanoparticles, and oral formulations (e.g., tablets, capsules, granules, powders, and beads) described herein comprise a dose of the adenosine A2A receptor antagonist from about 150 mg to about 250 mg. In aspects, the dose of the adenosine A2A receptor antagonist is from about 170 mg to about 230 mg. In aspects, the dose of the adenosine A2A receptor antagonist is from about 180 mg to about 220 mg. In aspects, the dose of the adenosine A2A receptor antagonist is from about 185 mg to about 215 mg. In aspects, the dose of the adenosine A2A receptor antagonist is from about 190 mg to about 210 mg. In aspects, the dose of the adenosine A2A receptor antagonist is from about 195 mg to about 205 mg. In aspects, the dose of the adenosine A2A receptor antagonist is about 200 mg.

In aspects, the compositions, the amorphous solid dispersions, compositions, extrudates, microprecipitated bulk powders, crystalline nanoparticles, and oral formulations (e.g., tablets, capsules, granules, powders, and beads) described herein comprise a dose of the adenosine A2A receptor antagonist from about 200 mg to about 300 mg. In aspects, the dose of the adenosine A2A receptor antagonist is from about 210 mg to about 290 mg. In aspects, the dose of the adenosine A2A receptor antagonist is from about 220 mg to about 280 mg. In aspects, the dose of the adenosine A2A receptor antagonist is from about 230 mg to about 270 mg. In aspects, the dose of the adenosine A2A receptor antagonist is from about 240 mg to about 260 mg. In aspects, the dose of the adenosine A2A receptor antagonist is about 250 mg.

In aspects, the compositions, the amorphous solid dispersions, compositions, extrudates, microprecipitated bulk powders, crystalline nanoparticles, and oral formulations (e.g., tablets, capsules, granules, powders, and beads) described herein comprise a dose of the adenosine A2A receptor antagonist from about 250 mg to about 350 mg. In aspects, the dose of the adenosine A2A receptor antagonist is from about 270 mg to about 330 mg. In aspects, the dose of the adenosine A2A receptor antagonist is from about 280 mg to about 320 mg. In aspects, the dose of the adenosine A2A receptor antagonist is from about 285 mg to about 315 mg. In aspects, the dose of the adenosine A2A receptor antagonist is from about 290 mg to about 310 mg. In aspects, the dose of the adenosine A2A receptor antagonist is from about 295 mg to about 305 mg. In aspects, the dose of the adenosine A2A receptor antagonist is about 300 mg.

In aspects, the compositions, the amorphous solid dispersions, compositions, extrudates, microprecipitated bulk powders, crystalline nanoparticles, and oral formulations (e.g., tablets, capsules, granules, powders, and beads) described herein comprise a dose of the adenosine A2A receptor antagonist from about 300 mg to about 400 mg. In aspects, the dose of the adenosine A2A receptor antagonist is from about 310 mg to about 390 mg. In aspects, the dose of the adenosine A2A receptor antagonist is from about 320 mg to about 380 mg. In aspects, the dose of the adenosine A2A receptor antagonist is from about 330 mg to about 370 mg. In aspects, the dose of the adenosine A2A receptor antagonist is from about 340 mg to about 360 mg. In aspects, the dose of the adenosine A2A receptor antagonist is about 350 mg.

In aspects, the compositions, the amorphous solid dispersions, compositions, extrudates, microprecipitated bulk powders, crystalline nanoparticles, and oral formulations (e.g., tablets, capsules, granules, powders, and beads) described herein comprise a dose of the adenosine A2A receptor antagonist from about 350 mg to about 450 mg. In aspects, the dose of the adenosine A2A receptor antagonist is from about 370 mg to about 430 mg. In aspects, the dose of the adenosine A2A receptor antagonist is from about 380 mg to about 420 mg. In aspects, the dose of the adenosine A2A receptor antagonist is from about 385 mg to about 415 mg. In aspects, the dose of the adenosine A2A receptor antagonist is from about 390 mg to about 410 mg. In aspects, the dose of the adenosine A2A receptor antagonist is from about 395 mg to about 405 mg. In aspects, the dose of the adenosine A2A receptor antagonist is about 400 mg.

In aspects, the compositions, the amorphous solid dispersions, compositions, extrudates, microprecipitated bulk powders, crystalline nanoparticles, and oral formulations (e.g., tablets, capsules, granules, powders, and beads) described herein comprise a dose of the adenosine A2A receptor antagonist from about 400 mg to about 500 mg. In aspects, the dose of the adenosine A2A receptor antagonist is from about 410 mg to about 490 mg. In aspects, the dose of the adenosine A2A receptor antagonist is from about 420 mg to about 480 mg. In aspects, the dose of the adenosine A2A receptor antagonist is from about 430 mg to about 470 mg. In aspects, the dose of the adenosine A2A receptor antagonist is from about 440 mg to about 460 mg. In aspects, the dose of the adenosine A2A receptor antagonist is about 450 mg.

In aspects, the compositions, the amorphous solid dispersions, compositions, extrudates, microprecipitated bulk powders, crystalline nanoparticles, and oral formulations (e.g., tablets, capsules, granules, powders, and beads) described herein comprise a dose of the adenosine A2A receptor antagonist from about 450 mg to about 550 mg. In aspects, the dose of the adenosine A2A receptor antagonist is from about 470 mg to about 530 mg. In aspects, the dose of the adenosine A2A receptor antagonist is from about 480 mg to about 520 mg. In aspects, the dose of the adenosine A2A receptor antagonist is from about 485 mg to about 515 mg. In aspects, the dose of the adenosine A2A receptor antagonist is from about 490 mg to about 510 mg. In aspects, the dose of the adenosine A2A receptor antagonist is from about 495 mg to about 505 mg. In aspects, the dose of the adenosine A2A receptor antagonist is about 500 mg.

In aspects, the compositions, the amorphous solid dispersions, compositions, extrudates, microprecipitated bulk powders, crystalline nanoparticles, and oral formulations (e.g., tablets, capsules, granules, powders, and beads) described herein comprise a dose of the adenosine A2A receptor antagonist from about 500 mg to about 600 mg. In aspects, the dose of the adenosine A2A receptor antagonist is from about 510 mg to about 590 mg. In aspects, the dose of the adenosine A2A receptor antagonist is from about 520 mg to about 580 mg. In aspects, the dose of the adenosine A2A receptor antagonist is from about 530 mg to about 570 mg. In aspects, the dose of the adenosine A2A receptor antagonist is from about 540 mg to about 560 mg. In aspects, the dose of the adenosine A2A receptor antagonist is about 550 mg.

In aspects, the compositions, the amorphous solid dispersions, compositions, extrudates, microprecipitated bulk powders, crystalline nanoparticles, and oral formulations (e.g., tablets, capsules, granules, powders, and beads) described herein comprise a dose of the adenosine A2A receptor antagonist from about 550 mg to about 650 mg. In aspects, the dose of the adenosine A2A receptor antagonist is from about 570 mg to about 630 mg. In aspects, the dose of the adenosine A2A receptor antagonist is from about 580 mg to about 620 mg. In aspects, the dose of the adenosine A2A receptor antagonist is from about 585 mg to about 615 mg. In aspects, the dose of the adenosine A2A receptor antagonist is from about 590 mg to about 610 mg. In aspects, the dose of the adenosine A2A receptor antagonist is from about 595 mg to about 605 mg. In aspects, the dose of the adenosine A2A receptor antagonist is about 600 mg.

### Properties: Pharmaceutical Compositions of Embodiments H, M, and N

In embodiments, the pharmaceutical compositions (e.g., amorphous solid dispersions, compositions, extrudates, microprecipitated bulk powders, crystalline nanoparticles, and oral formulations) described herein are rapidly-dissolving, as measured by US Pharmacopeia (USP), Chapter <711>, Type II (Paddle) Dissolution Apparatus in pH 6.8, 50 mM phosphate buffer. In aspects, the dissolution is at least 50% in 20 minutes. In aspects, the dissolution is at least 55% in 20 minutes. In aspects, the dissolution is at least 60% in 20 minutes. In aspects, the dissolution is at least 65% in 20 minutes. In aspects, the dissolution is at least 70% in 20 minutes. In aspects, the dissolution is at least 75% in 20 minutes. In aspects, the dissolution is at least 80% in 20 minutes. In aspects, the dissolution is at least 85% in 20 minutes. In aspects, the dissolution is at least 90% in 20 minutes. In aspects, the dissolution is at least 95% in 20 minutes. In aspects, the dissolution is 100% in 20 minutes. In aspects, the dissolution is at least 55% in 60 minutes. In aspects, the dissolution is at least 60% in 60 minutes. In aspects, the dissolution is at least 65% in 60 minutes. In aspects, the dissolution is at least 70% in 60 minutes. In aspects, the dissolution is at least 75% in 60 minutes. In aspects, the dissolution is at least 80% in 60 minutes. In aspects, the dissolution is at least 81% in 60 minutes. In aspects, the dissolution is at least 82% in 60 minutes. In aspects, the dissolution is at least 83% in 60 minutes. In aspects, the dissolution is at least 84% in 60 minutes. In aspects, the dissolution is at least 85% in 60 minutes. In aspects, the dissolution is at least 86% in 60 minutes. In aspects, the dissolution is at least 87% in 60 minutes. In aspects, the dissolution is at least 88% in 60 minutes. In aspects, the dissolution is at least 90% in 60 minutes. In aspects, the dissolution is at least 95% in 60 minutes.

In embodiments, the dissolution is at least 55% in 30 minutes, as measured by US Pharmacopeia (USP), Chapter <711>, Type II (Paddle) Dissolution Apparatus. In aspects, the dissolution is at least 60% in 30 minutes. In aspects, the dissolution is at least 65% in 30 minutes. In aspects, the dissolution is at least 70% in 30 minutes. In aspects, the dissolution is at least 75% in 30 minutes. In aspects, the dissolution is at least 80% in 30 minutes. In aspects, the dissolution is at least 85% in 30 minutes. In aspects, the dissolution is at least 90% in 30 minutes. In aspects, the dissolution is at least 95% in 30 minutes. "Dissolution" or "dissolution rate" described herein is measured by US Pharmacopeia (USP), Chapter <711>, Type II (Paddle) Dissolution Apparatus.

In embodiments, the dissolution is at least 50% in 10 minutes, as measured by US Pharmacopeia (USP), Chapter <711>, Type II (Paddle) Dissolution Apparatus. In aspects, the dissolution is at least 55% in 10 minutes. In aspects, the dissolution is at least 60% in 10 minutes. In aspects, the dissolution is at least 65% in 10 minutes. In aspects, the dissolution is at least 70% in 10 minutes. In aspects, the dissolution is at least 75% in 10 minutes. In aspects, the dissolution is at least 80% in 10 minutes. In aspects, the dissolution is at least 85% in 10 minutes. In aspects, the dissolution is at least 90% in 10 minutes. "Dissolution" or "dissolution rate" described herein is measured by US Pharmacopeia (USP), Chapter <711>, Type II (Paddle) Dissolution Apparatus.

In embodiments, the pharmaceutical compositions (e.g., amorphous solid dispersions, compositions, extrudates, microprecipitated bulk powders, crystalline nanoparticles, and oral formulations) described herein are rapidly-disintegrating, as measured by US Pharmacopeia (USP), Chapter <701>, Disintegration Test. In aspects, the pharmaceutical compositions have a disintegration time of about 20 minutes or less. In aspects, the pharmaceutical compositions have a disintegration time of about 19 minutes or less. In aspects, the disintegration time is about 18 minutes or less. In aspects, the disintegration time is about 17 minutes or less. In aspects, the disintegration time is about 16 minutes or less. In aspects, the disintegration time is about 15 minutes or less. In aspects, the disintegration time is about 14 minutes or less. In aspects, the disintegration time is about 13 minutes or less. In aspects, the disintegration time is about 12 minutes or less. In aspects, the disintegration time is about 11 minutes or less. In aspects, the disintegration time is about 10 minutes or less. In aspects, the disintegration time is about 9 minutes or less. In aspects, the disintegration time is about 8 minutes or less. In aspects, the disintegration time is about 7 minutes or less. In aspects, the disintegration time is about 6 minutes or less. In aspects, the disintegration time is about 5 minutes or less. In aspects, the disintegration time is about 4 minutes or less. In aspects, the disintegration time is about 3 minutes or less. In aspects, the disintegration time is about 2 minutes or less. In aspects, the disintegration time is about 1 minute or less.

In embodiments, the pharmaceutical compositions (e.g., amorphous solid dispersions, compositions, extrudates, microprecipitated bulk powders, crystalline nanoparticles, and oral formulations) described herein have a total impurity level of less than 3.0% under one of the following conditions (i) 3 months of storage at 40°C and 75% relative humidity, as measured by high performance liquid chromatography followed by ultraviolet spectroscopy (HPLC-UV); or (ii) 6 months of storage at 40°C and 75% relative humidity, as measured by HPLC-UV. In aspects, the total impurity level is less than 2.5%. In aspects, the pharmaceutical compositions have a total impurity level of less than 2.0%. In aspects, the total impurity level is less than 1.5%. In aspects, the total impurity level is less than 1.4%. In aspects, the total impurity level is less than 1.3%. In aspects, the total impurity level is less than 1.2%. In aspects, the total impurity level is less than 1.1%. In aspects, the total impurity level is less than 1.0%. In aspects, the total impurity level is less than 0.9%. In aspects, the total impurity level is from about 0.5% to less than 2.5%. In aspects, the total impurity level is from about 0.5% to less than 2.0%. In aspects, the total impurity level is from about 0.5% to less than 1.5%. In aspects, the total impurity level is from about 0.5% to less than 1.4%. In aspects, the total impurity level is from about 0.5% to less than 1.3%. In aspects, the total impurity level is from about 0.5% to less than 1.2%. In aspects, the total impurity level is from about 0.5% to less than 1.1%. In aspects, the total impurity level is from about 0.5% to less than 1.0%. In aspects, the total impurity level is from about 0.5% to less than 0.9%. In aspects, the total impurity level is measured after 3 months of storage at 40°C and 75% relative humidity. In aspects, the total impurity level is measured after 6 months of storage at 40°C and 75% relative humidity.

In embodiments, the pharmaceutical compositions (e.g., amorphous solid dispersions, compositions, extrudates, microprecipitated bulk powders, crystalline nanoparticles, and oral formulations) described herein have an assay % label claim (LC) from about 90% to about 110%. In aspects, the %LC is from about 91% to about 109%. In aspects, the %LC is from about 92% to about 108%. In aspects, the %LC is from about 93% to about 107%. In aspects, the %LC is from about 94% to about 106%. In aspects, the %LC is from about 95% to about 105%. In aspects, the %LC is from about 96% to about 104%. In aspects, the %LC is from about 97% to about 103%. In aspects, the %LC is from about 98% to about 102%. In aspects, the %LC is from about 99% to about 101%.

In embodiments, pharmaceutical compositions (e.g., amorphous solid dispersions, compositions, extrudates, microprecipitated bulk powders, crystalline nanoparticles, and oral formulations) described herein have a kinetic solubility of at least 40 µg/mL. In aspects, the kinetic solubility is at least 45 µg/mL. In aspects, the kinetic solubility is at least 50 µg/mL. In aspects, the kinetic solubility is at least 55 µg/mL. In aspects, the kinetic solubility is at least 60 µg/mL. In aspects, the kinetic solubility is at least 65 µg/mL. In aspects, the kinetic solubility is at least 70 µg/mL. In aspects, the kinetic solubility is at least 75 µg/mL. In aspects, the kinetic solubility is at least 80 µg/mL. In aspects, the kinetic solubility is at least 85 µg/mL. In aspects, the kinetic solubility is at least 90 µg/mL. In aspects, the kinetic solubility is at least 95 µg/mL. In aspects, the kinetic solubility is at least 100 µg/mL. In aspects, the kinetic solubility is at least 105 µg/mL. In aspects, the kinetic solubility is from about 50 µg/mL to about 150 µg/mL. In aspects, the kinetic solubility is from about 60 µg/mL to about 150 µg/mL. In aspects, the kinetic solubility is from about 70 µg/mL to about 150 µg/mL. In aspects, the kinetic solubility is from about 75 µg/mL to about 150 µg/mL. In aspects, the kinetic solubility is from about 80 µg/mL to about 150 µg/mL. In aspects, the kinetic solubility is from about 85 µg/mL to about 150 µg/mL. In aspects, the kinetic solubility is from about 90 µg/mL to about 150 µg/mL. In aspects, the kinetic solubility is from about 95 µg/mL to about 150 µg/mL. In aspects, the kinetic solubility is from about 100 µg/mL to about 150 µg/mL. In aspects, the kinetic solubility is from about 50 µg/mL to about 125 µg/mL. In aspects, the kinetic solubility is from about 60 µg/mL to about 125 µg/mL. In aspects, the kinetic solubility is from about 70 µg/mL to about 125 µg/mL. In aspects, the kinetic solubility is from about 75 µg/mL to about 125 µg/mL. In aspects, the kinetic solubility is from about 80 µg/mL to about 125 µg/mL. In aspects, the kinetic solubility is from about 85 µg/mL to about 125 µg/mL. In aspects, the kinetic solubility is from about 90 µg/mL to about 125 µg/mL. In aspects, the kinetic solubility is from about 95 µg/mL to about 125 µg/mL. In aspects, the kinetic solubility is from about 100 µg/mL to about 125 µg/mL. In aspects, the kinetic solubility is from about 50 µg/mL to about 120 µg/mL. In aspects, the kinetic solubility is from about 60 µg/mL to about 120 µg/mL. In aspects, the kinetic solubility is from about 70 µg/mL to about 120 µg/mL. In aspects, the kinetic solubility is from about 75 µg/mL to about 120 µg/mL. In aspects, the kinetic solubility is from about 80 µg/mL to about 120 µg/mL. In aspects, the kinetic solubility is from about 85 µg/mL to about 120 µg/mL. In aspects, the kinetic solubility is from about 90 µg/mL to about 120 µg/mL. In aspects, the kinetic solubility is from about 95 µg/mL to about 120 µg/mL. In aspects, the kinetic solubility is from about 100 µg/mL to about 120 µg/mL.

### Methods of Treatment: Pharmaceutical Compositions of Embodiments H, M, and N

In embodiments, the disclosure provides methods of treating cancer in patients by administering to the patients therapeutically effective amounts of the amorphous solid dispersions, compositions, extrudates, microprecipitated bulk powders, crystalline nanoparticles, or oral formulations described herein. The methods are effective to treat any cancer, particularly cancers that are modulated by the adenosine A2A receptor. In aspects, the methods are for treating lung cancer, melanoma, renal cell cancer, breast cancer, colorectal cancer, bladder cancer, prostate cancer, or a head and neck cancer. In aspects, the methods are for treating non-small cell lung cancer, malignant melanoma, renal cell cancer, triple negative breast cancer, colorectal cancer, or bladder cancer. In aspects, the methods are for treating lung cancer. In aspects, the methods are for treating non-small cell lung cancer. In aspects, the methods are for treating melanoma. In aspects, the methods are for treating malignant melanoma. In aspects, the methods are for treating renal cell cancer. In aspects, the methods are for treating breast cancer. In aspects, the methods are for treating triple negative breast cancer. In aspects, the methods are for treating colorectal cancer. In aspects, the methods are for treating microsatellite instable colorectal cancer. In aspects, the methods are for treating bladder cancer. In embodiment, the methods are for treating head and neck cancers. In aspects, the methods are for treating prostate cancer. In aspects, the methods are for treating castration-resistant prostate cancer. In aspects, the methods are for treating metastatic castration-resistant prostate cancer. The methods of treating cancer encompass methods of treating metastatic cancer, methods of treating cancer tumors, and methods of treating metastatic cancer tumors.

The amorphous solid dispersions, compositions, extrudates, microprecipitated bulk powders, crystalline nanoparticles, and oral formulations described herein contain the adenosine A2A receptor antagonist in a therapeutically effective amount, i.e., in an amount effective to treat cancer. When administered in methods to treat cancer, such compositions will contain an amount of active ingredient effective to achieve the desired results, which include one or more of (i) slowing the progression of the cancer; (ii) enhancing anti-tumor immune memory; (iii) increasing one or more of CD8+ cell infiltration, T cell activation, interferon-gamma pathway gene expression, and T cell clone expansion over baseline; (iv) preventing a tumor from growing in size or volume over time; (v) decreasing a cancerous tumor in size or volume over time; and (vi) preventing the cancerous tumor from metastasizing. Determination of a therapeutically effective amount of the adenosine A2A receptor antagonist is within the capabilities of the skilled artisan, especially in light of the detailed disclosure herein.

In embodiments of the methods of treating cancer with the pharmaceutical compositions, oral formulations (e.g., granules, beads, tablets, capsules, powders), amorphous solid dispersions, extrudates, crystalline nanoparticles, and microprecipitated bulk powders described herein, the therapeutically effective amount of the adenosine A2A receptor antagonist is from about 1 mg to about 1,000 mg. In aspects, the therapeutically effective amount is from about 1 mg to about 9000 mg. In aspects, the therapeutically effective amount is from about 1 mg to about 800 mg. In aspects, the therapeutically effective amount is from about 1 mg to about 700 mg. In aspects, the therapeutically effective amount is from about 1 mg to about 600 mg. In aspects, the therapeutically effective amount is from about 1 mg to about 500 mg. In aspects, the therapeutically effective amount is from about 1 mg to about 400 mg.

In embodiments of the methods of treating cancer with the pharmaceutical compositions, oral formulations (e.g., granules, beads, tablets, capsules, powders), amorphous solid dispersions, extrudates, crystalline nanoparticles, and microprecipitated bulk powders described herein, the therapeutically effective amount of the adenosine A2A receptor antagonist is from about 100 mg to about 1,000 mg. In aspects, the therapeutically effective amount is from about 100 mg to about 900 mg. In aspects, the therapeutically effective amount is from about 100 mg to about 850 mg. In aspects, the therapeutically effective amount is from about 100 mg to about 800 mg. In aspects, the therapeutically effective amount is from about 100 mg to about 700 mg. In aspects, the therapeutically effective amount is from about 100 mg to about 600 mg. In aspects, the therapeutically effective amount is from about 100 mg to about 500 mg. In aspects, the therapeutically effective amount is from about 100 mg to about 450 mg. In aspects, the therapeutically effective amount is from about 100 mg to about 400 mg. In aspects, the therapeutically effective amount is from about 100 mg to about 300 mg. In aspects, the therapeutically effective amount is from about 150 mg to about 250 mg. In aspects, the therapeutically effective amount is from about 200 mg to about 400 mg. In aspects, the therapeutically effective amount is from about 250 mg to about 350 mg. In aspects, the therapeutically effective amount is from about 300 mg to about 500 mg. In aspects, the therapeutically effective amount is from about 350 mg to about 450 mg. In aspects, the administration of this therapeutically effective amount is QD. In aspects, the administration of this therapeutically effective amount is BID.

In embodiments of the methods of treating cancer with the pharmaceutical compositions, oral formulations (e.g., granules, beads, tablets, capsules, powders), amorphous solid dispersions, extrudates, crystalline nanoparticles, and microprecipitated bulk powders described herein, the therapeutically effective amount of the adenosine A2A receptor antagonist is about 100 mg. In aspects, the therapeutically effective amount of the adenosine A2A receptor antagonist is about 125 mg. In aspects, the therapeutically effective amount of the adenosine A2A receptor antagonist is about 150 mg. In aspects, the therapeutically effective amount of the adenosine A2A receptor antagonist is about 175 mg. In aspects, the therapeutically effective amount of the adenosine A2A receptor antagonist is about 200 mg. In aspects, the therapeutically effective amount of the adenosine A2A receptor antagonist is about 225 mg. In aspects, the therapeutically effective amount of the adenosine A2A receptor antagonist is about 250 mg. In aspects, the therapeutically effective amount of the adenosine A2A receptor antagonist is about 275 mg. In aspects, the therapeutically effective amount of the adenosine A2A receptor antagonist is about 300 mg. In aspects, the therapeutically effective amount of the adenosine A2A receptor antagonist is about 325 mg. In aspects, the therapeutically effective amount of the adenosine A2A receptor antagonist is about 350 mg. In aspects, the therapeutically effective amount of the adenosine A2A receptor antagonist is about 375 mg. In aspects, the therapeutically effective amount of the adenosine A2A receptor antagonist is about 400 mg. In aspects, the therapeutically effective amount of the adenosine A2A receptor antagonist is about 425 mg. In aspects, the therapeutically effective amount of the adenosine A2A receptor antagonist is about 450 mg. In aspects, the therapeutically effective amount of the adenosine A2A receptor antagonist is about 475 mg. In aspects, the therapeutically effective amount of the adenosine A2A receptor antagonist is about 500 mg. In aspects, the therapeutically effective amount of the adenosine A2A receptor antagonist is about 525 mg. In aspects, the therapeutically effective amount of the adenosine A2A receptor antagonist is about 550 mg. In aspects, the therapeutically effective amount of the adenosine A2A receptor antagonist is about 575 mg. In aspects, the therapeutically effective amount of the adenosine A2A receptor antagonist is about 600 mg. In aspects, the therapeutically effective amount of the adenosine A2A receptor antagonist is about 625 mg. In aspects, the therapeutically effective amount of the adenosine A2A receptor antagonist is about 650 mg. In aspects, the therapeutically effective amount of the adenosine A2A receptor antagonist is about 675 mg. In aspects, the therapeutically effective amount of the adenosine A2A receptor antagonist is about 700 mg. In aspects, the therapeutically effective amount of the adenosine A2A receptor antagonist is about 725 mg. In aspects, the therapeutically effective amount of the adenosine A2A receptor antagonist is about 750 mg. In aspects, the therapeutically effective amount of the adenosine A2A receptor antagonist is about 775 mg. In aspects, the therapeutically effective amount of the adenosine A2A receptor antagonist is about 800 mg. In aspects, the therapeutically effective amount of the adenosine A2A receptor antagonist is about 825 mg. In aspects, the therapeutically effective amount of the adenosine A2A receptor antagonist is about 850 mg. In aspects, the therapeutically effective amount of the adenosine A2A receptor antagonist is about 875 mg. In aspects, the therapeutically effective amount of the adenosine A2A receptor antagonist is about 900 mg. In aspects, the therapeutically effective amount of the adenosine A2A receptor antagonist is about 950 mg. In aspects, the therapeutically effective amount of the adenosine A2A receptor antagonist is about 1,000 mg. In aspects, the administration of this therapeutically effective amount is QD. In aspects, the administration of this therapeutically effective amount is BID.

The frequency (e.g., once per day, twice per day, thrice per day) and duration (e.g., one week, two weeks, one month, two months, six months, 1 year, 5 to 10 years, or until disease progression) of administration of the pharmaceutical compositions described herein can vary depending upon a variety of factors, for example, whether the patient suffers from another disease, and the route of administration; size, age, sex, health, body weight; nature and extent of symptoms of the disease being treated; whether there is concurrent treatment (e.g., with chemotherapeutic agents, radiation, or immunomodulatory compounds), complications from the cancer being treated or other health-related problems. Adjustment and manipulation of the frequency and duration of treatment are within the ability of one skilled in the art. In aspects, the pharmaceutical compositions described herein are administered to the patient once per day (QD). In aspects, the pharmaceutical compositions are administered twice per day (BID). In aspects, the pharmaceutical compositions are administered thrice per day. In aspects, the pharmaceutical compositions are administered once per day for about 7 days. In aspects, the pharmaceutical compositions are administered once per day for about 14 days. In aspects, the pharmaceutical compositions are administered once per day for about 21 days. In aspects, the pharmaceutical compositions are administered once per day for about one month. In aspects, the pharmaceutical compositions are administered once per day until disease progression. In aspects, the pharmaceutical compositions are administered twice per day for about 7 days. In aspects, the pharmaceutical compositions are administered twice per day for about 14 days. In aspects, the pharmaceutical compositions are administered twice per day for about 21 days. In aspects, the pharmaceutical compositions are administered twice per day for one month. In aspects, the pharmaceutical compositions are administered twice per day until disease progression. After administration for 14 days, another cycle of administration can immediately begin or there can be a discontinuation of treatment for 14 days or one month, followed by another cycle of administration. After administration for a month, another cycle of administration can immediately begin or there can be a discontinuation of treatment for 14 days or one month, followed by another cycle of administration. Thus, after administration for one cycle (e.g., once/twice/thrice per day for 7 days, 14 days, 21 days, 1 month), another cycle of administration can immediately begin or there can be a discontinuation of treatment for a period of time (e.g., 7 days, 14, days, 21 days, 1 month), followed by another cycle of administration. The cycles of administration and/or discontinuation can be the same period of time (e.g., 14 days) or different periods of time (e.g., one cycle is 14 days and another cycle is 21 days).

### Processes of Manufacture: Pharmaceutical Compositions of Embodiment H

The amorphous solid dispersions, compositions, extrudates, and oral formulations described herein, can be prepared by methods know in the art, including those described herein. In embodiments, the disclosure provides processes of forming the amorphous solid dispersions, compositions, extrudates, and oral formulations described herein by the steps of: (i) adding the crystalline adenosine A2A receptor antagonist and the thermoplastic polymer to a hot melt extrusion device; (ii) melting crystalline adenosine A2A receptor antagonist and the thermoplastic polymer by heating to a temperature close to or above the melting point of the drug but substantially lower than the decomposition temperature of the drug; (iii) mixing the adenosine A2A receptor antagonist and the thermoplastic polymer to form a homogenous blend; (iv) extruding the homogenous blend to form the amorphous solid dispersion or the solid composition. In embodiments, steps (ii) and (iii) can be performed in the opposite order. In embodiments, steps (ii) and (iii) can be performed concurrently.

Hot melt extrusion devices and methods of hot melt extrusion are well known in the art. Generally, an extruder equipped with a screw in the barrel (cylinder) (e.g., single screw extruder, twin screw extruder, etc.) can be used. Among these extruders, a twin screw extruder is generally used. An extruder is composed of five main parts, i.e., hopper (input structure), motor (controlling the rotation of screw), screw (primary source for shearing a material and moving it), barrel (housing a screw and controlling the temperature), and die (outlet) (controlling the shape and size of extrudate). Therein, the adenosine A2A receptor antagonist, the thermoplastic polymer, and optional pharmaceutically acceptable excipients are added into the machine via the hopper, in which the temperature of the hot-melt is suitably retained, and then the solid mixture is melted by the rotation of the screw to be kneaded uniformly. Alternatively, it is possible to preliminarily mix the ingredients before adding them into the machine, as appropriate. Once in a molten state, the homogenized blend is transported to the orifice through another heating zone that maintains the molten state of the blend. At the orifice, the molten blend can be formed into strands, cylinders or films. The extrudate that exits is then solidified typically by an air-cooling cooling process. Once solidified, the extrudate may then be further processed (e.g., cut, milled) to form pellets, spheres, fine powders, tablets, beads, and the like.

For hot melt extrusion, it is necessary to heat the adenosine A2A receptor antagonist and the thermoplastic polymer to a temperature greater than both the glass-transition temperature of the polymer and close to or and usually above the melting point of the crystalline drug. On the other hand, the temperature should not be so high too cause decomposition or denaturation of the adenosine A2A receptor antagonist and the thermoplastic polymer. Thus, the temperature for hot melt extrusion should generally be about 200°C or lower, or about 190°C or lower, or about 180°C or lower, depending on the melting point of the crystalline drug and substantially lower than the decomposition temperature of the drug.

In addition to temperature, the setting conditions in the hot melt extrusion process, such as pressure, speed of supplying material, diameter of die, shape of screw, and rotary speed of screw can vary, but the skilled artisan could readily identify the setting conditions based on the general knowledge in the art and based on the examples herein. For example, the setting conditions for the hot melt extrusion process can be those conditions shown in the examples herein. In view of this information, the skilled artisan could optionally vary one or more of the setting conditions to further optimize the setting conditions to produce the compositions described herein. In aspects where the drug is particularly sensitive to degradation by heat, the drug may be added in a later zone than the flowing polymer to decrease the residence time with adroit design of the screws to provide sufficient mixing in the shorter time.

In hot melt extrusion, other pharmaceutically acceptable excipients, in addition to the thermoplastic polymer, may be used. Such pharmaceutically acceptable excipients are not limited as long as they are what are generally used as a material for pharmaceutical formulation and additionally they do not damage the function of the solid amorphous dispersions.

### Processes of Manufacture: Pharmaceutical Compositions of Embodiment M

The amorphous solid dispersions, compositions, microprecipitated bulk powders, and oral formulations described herein, can be prepared by methods know in the art, including those described herein. In aspects, the amorphous solid dispersions, compositions, microprecipitated bulk powders, and oral formulations described herein can be prepared by a co-precipitation process. Briefly, an organic solvent is used to dissolve the adenosine A2A receptor antagonist and the pharmaceutically acceptable polymer; thereafter, an anti-solvent is added to co-precipitate the adenosine A2A receptor antagonist and pharmaceutically acceptable polymer. The solvent is then extracted by washing, and the amorphous coprecipitated materials are filtered and dried. In aspects, the organic solvent is a polar aprotic solvent. In aspects, the anti-solvent is maintained at a temperature below room temperature, such as a temperature from about 2°C to about 8°C. In aspects, the antisolvent is cold (2°C to 8°C) water at pH 5 or less.

In aspects, the method for manufacturing the compositions described herein can include the following steps. The adenosine A2A receptor antagonist and the pharmaceutically acceptable polymer are dissolved in an organic solvent. In aspects, the adenosine A2A receptor antagonist is dissolved in an organic solvent, and thereafter, the pharmaceutically acceptable polymer is added and dissolved therein. In aspects, the pharmaceutically acceptable polymer is dissolved in an organic solvent, and thereafter, the adenosine A2A receptor antagonist is added and dissolved therein. In aspects, the adenosine A2A receptor antagonist is dissolved in a first organic solvent to form a first solution, and the pharmaceutically acceptable polymer is dissolved in a second organic solvent to form a second solution, and thereafter, the first solution is combined with the second solution to form a third solution containing the adenosine A2A receptor antagonist and the pharmaceutically acceptable polymer. In aspects, the first organic solvent and the second organic solvent are the same organic solvent. In aspects, the first organic solvent and the second organic solvent are different organic solvents.

After preparation of the solution containing the adenosine A2A receptor antagonist, the pharmaceutically acceptable polymer and the organic solvent, the solution can be added (e.g., continuously, batch-wise) into an aqueous phase (e.g., water) in a mixing chamber that is equipped with a high shear mixing unit and two additional openings which connect the mixing chamber to a closed loop where the aqueous phase circulates and passes through the mixing chamber to form an aqueous suspension containing an amorphous formulation containing the adenosine A2A receptor antagonist and the pharmaceutically acceptable polymer, which amorphous formulation precipitates out of the aqueous phase. Thereafter the amorphous formulation is isolated and washed with an acidic solution and/or water to remove the organic solvent, and then dried to provide the amorphous formulation.

As discussed herein, the dried amorphous formulation can be further processed into any type of solid pharmaceutical preparations or dosage forms, which are known to the person of skill in the art, such as oral formulations, including tablets, capsules, powders, and the like.

### Process of Manufacture: Pharmaceutical Compositions of Embodiment N

In embodiments, the adenosine A2A receptor antagonist (e.g., compound of Formula (III)) is milled or precipitated, by any process known in the art, into nanoparticles with a particle size distribution as described herein. In aspects, the adenosine A2A receptor antagonist is formulated into nanoparticles by dry milling, either alone or with one or more pharmaceutically acceptable excipients. An exemplary dry milling device is a jet milling. In aspects, the adenosine A2A receptor antagonist is formed into nanoparticles by wet milling, either alone or with one or more pharmaceutically acceptable excipients. Exemplary wet milling devices include planetary mill, ball mill, or agitator bead mill. Nanoparticle formulations may also be formed by spray drying or by antisolvent precipitation techniques.

After the adenosine A2A receptor antagonist is formed into nanoparticles, it is screened and blended with the pharmaceutically acceptable excipients described herein. The screening step can be performed with any milling device or sieving device known in the art, such as with a gyratory screening machine, a tumbler screening machine, a vibration screening machine, a tumbler-vibration screening machine, or a comil. Any appropriate sieve size may be used in this step of the process. In aspects, the sieve size is a # 16 mesh US standard sieve; or a # 18 mesh US standard sieve; or a # 20 mesh US standard sieve. In aspects, the sieve size is a # 20 mesh US standard sieve; or a # 30 mesh US standard sieve; or a # 35 mesh US standard sieve.

After screening and blending the adenosine A2A receptor antagonist and pharmaceutically acceptable excipients, the blended formulation is granulated to form agglomerates of the adenosine A2A receptor antagonist and pharmaceutically acceptable excipients. Granulation techniques include wet granulation (e.g., low shear, high shear, fluid bed); dry granulation (e.g., roller compaction), and the like. Thereafter, the granules, if not produced from a dry process, are dried (e.g., oven tray drying, fluid bed drying) to remove any moisture.

Following granulation, the formulation is milled with any milling device known in the art, such as with a gyratory screening machine, a tumbler screening machine, a vibration screening machine, or a tumbler-vibration screening machine. In aspects, a FitzMill® Comminutor (The Fitzpatrick Company) may be used. For certain dry granulation processes, the milling step may be connected to and immediately following the granulation process. Thereafter, the formulation may be further blended with pharmaceutically acceptable excipients, compressed into a tablet, and optionally coated with a coating layer, such as OPADRY®.

These methods of forming the compositions described herein are described in more detail in the Examples.

Embodiments H, M, and N are further described, but not limited to, the following descriptions.

### Embodiments H1-H118

Embodiment H1. An amorphous solid dispersion comprising an adenosine A2A receptor antagonist and a thermoplastic polymer.

Embodiment H2. The amorphous solid dispersion of Embodiment H1, wherein the adenosine A2A receptor antagonist is a compound of Formula (I).

Embodiment H3. The amorphous solid dispersion of Embodiment H1, wherein the adenosine A2A receptor antagonist is a compound of Formula (II).

Embodiment H4. The amorphous solid dispersion of Embodiment H1, wherein the adenosine A2A receptor antagonist is a compound of Formula (III).

Embodiment H5. The amorphous solid dispersion of Embodiment H1, wherein the adenosine A2A receptor antagonist is a compound of Formula (IIIA), a compound of Formula (IIIB), or a mixture thereof.

Embodiment H6. The amorphous solid dispersion of any one of Embodiments H1 to H5 having a glass transition temperature of at least 60°C.

Embodiment H7. The amorphous solid dispersion of any one of Embodiments H1 to H5 having a glass transition temperature between about 65°C and about 150°C.

Embodiment H8. The amorphous solid dispersion of any one of Embodiments H1 to H5 having a glass transition temperature between about 65°C and about 100°C.

Embodiment H9. The amorphous solid dispersion of any one of Embodiments H1 to H5 having a glass transition temperature of at least 70°C.

Embodiment H10. The amorphous solid dispersion of any one of Embodiments H1 to H5 having a glass transition temperature between about 70°C and about 95°C.

Embodiment H11. The amorphous solid dispersion of any one of Embodiments H1 to H5 having a glass transition temperature between 80°C and about 90°C.

Embodiment H12. The amorphous solid dispersion of one of Embodiments H1 to H11, wherein the thermoplastic polymer is: (i) a polyvinylpyrrolidone polymer, (ii) a polyvinylpyrrolidone copolymer, (iii) an acrylic polymer, (iv) an acrylic copolymer, (v) hydroxypropyl methylcellulose acetate succinate, or (vi) a combination of two or more of the foregoing.

Embodiment H13. The amorphous solid dispersion of one of Embodiments H1 to H11, wherein the thermoplastic polymer is a copolymer of polyvinylpyrrolidone and vinyl acetate.

Embodiment H14. The amorphous solid dispersion of Embodiment H13, wherein the copolymer of polyvinylpyrrolidone and vinyl acetate has a mass ratio of polyvinylpyrrolidone to vinyl acetate of about 3:2.

Embodiment H15. The amorphous solid dispersion of one of Embodiments H1 to H11, wherein the thermoplastic polymer is a methyl methacrylate polymer, an ethyl acrylate polymer, or a copolymer of methyl methacrylate and ethyl methacrylate.

Embodiment H16. The amorphous solid dispersion of one of Embodiments H1 to H11, wherein the thermoplastic polymer is a copolymer of methacrylic acid and methyl methacrylate.

Embodiment H17. The amorphous solid dispersion of one of Embodiments H1 to H11, wherein the thermoplastic polymer is a copolymer of methacrylic acid and ethyl acrylate.

Embodiment H18. The amorphous solid dispersion of Embodiment H17, wherein the copolymer of methacrylic acid and ethyl acrylate has a mass ratio of methacrylic acid to ethyl acrylate of about 1:1.

Embodiment H19. The amorphous solid dispersion of one of Embodiments H1 to H18, comprising about 5 wt% to about 40 wt% of the adenosine A2A receptor antagonist.

Embodiment H20. The amorphous solid dispersion of one of Embodiments H1 to H18, comprising 20 wt% to 40 wt% of the adenosine A2A receptor antagonist.

Embodiment H21. The amorphous solid dispersion of one of Embodiments H1 to H18, comprising about 30 wt% of the adenosine A2A receptor antagonist.

Embodiment H22. The amorphous solid dispersion of one of Embodiments H1 to H19, comprising 60 wt% to about 90 wt% of the thermoplastic polymer.

Embodiment H23. The amorphous solid dispersion of one of Embodiments H1 to H20, comprising 60 wt% to 80 wt% of the thermoplastic polymer.

Embodiment H24. The amorphous solid dispersion of one of Embodiments H1 to H21, comprising about 70 wt% of the thermoplastic polymer.

Embodiment H25. The amorphous solid dispersion of any one of Embodiments H1 to H18, comprising about 20 wt% to about 40 wt% of the adenosine A2A receptor antagonist and about 60 wt% to about 80% of the thermoplastic polymer.

Embodiment H26. The amorphous solid dispersion of any one of Embodiments H1 to H18, comprising about 25 wt% to about 35 wt% of the adenosine A2A receptor antagonist and about 65 wt% to about 75% of the thermoplastic polymer.

Embodiment H27. The amorphous solid dispersion of any one of Embodiments H1 to H18, comprising about 30 wt% of the adenosine A2A receptor antagonist and about 70 wt% of the thermoplastic polymer.

Embodiment H28. The amorphous solid dispersion of one of Embodiments H1 to H27, wherein the weight ratio of the adenosine A2A receptor antagonist to the thermoplastic polymer is from about 1:4 to about 1:1.

Embodiment H29. The amorphous solid dispersion of one of Embodiments H1 to H27, wherein the weight ratio of the adenosine A2A receptor antagonist to the thermoplastic polymer is about 1:2.3.

Embodiment H30. The amorphous solid dispersion of any one of Embodiments H1 to H29, comprising from about 1 mg to about 1,000 mg of the adenosine A2A receptor antagonist.

Embodiment H31. The amorphous solid dispersion of any one of Embodiments H1 to H29, comprising from about 10 mg to about 800 mg of the adenosine A2A receptor antagonist.

Embodiment H32. The amorphous solid dispersion of any one of Embodiments H1 to H29, comprising 50 mg to 600 mg of the adenosine A2A receptor antagonist

Embodiment H33. The amorphous solid dispersion of any one of Embodiments H1 to H29, comprising from about 100 mg to about 400 mg of the adenosine A2A receptor antagonist.

Embodiment H34. The amorphous solid dispersion of any one of Embodiments H1 to H33 formed by hot melt extrusion.

Embodiment H35. A pharmaceutical formulation comprising the amorphous solid dispersion of any one of Embodiments H1 to H34.

Embodiment H36. The pharmaceutical composition of Embodiment H35, wherein the pharmaceutical formulation is an oral formulation.

Embodiment H37. The pharmaceutical composition of Embodiment H36, wherein the oral formulation is a capsule or a tablet.

Embodiment H38. A method of treating cancer in a patient in need thereof, the method comprising administering an effective amount of the amorphous solid dispersion of any one of Embodiments H1 to H34 or the pharmaceutical formulation of any one of Embodiments H35 to H37 to the patient to treat the cancer.

Embodiment H39. The method of Embodiment H38, wherein the cancer is non-small cell lung cancer, melanoma, renal cell cancer, breast cancer, colorectal cancer, bladder cancer, prostate cancer, or a head and neck cancer.

Embodiment H40. An composition comprising an amorphous adenosine A2A receptor antagonist or a pharmaceutically acceptable salt thereof dispersed in a thermoplastic polymer.

Embodiment H41. The composition of Embodiment H40, wherein the adenosine A2A receptor antagonist is a compound of Formula (I).

Embodiment H42. The composition of Embodiment H40, wherein the adenosine A2A receptor antagonist is a compound of Formula (II).

Embodiment H43. The composition of Embodiment H40, wherein the adenosine A2A receptor antagonist is a compound of Formula (III).

Embodiment H44. The composition of Embodiment H40, wherein the adenosine A2A receptor antagonist is a compound of Formula (IIIA), a compound of Formula (IIIB), or a mixture thereof.

Embodiment H45. The composition of any one of Embodiments H40 to H44 having a glass transition temperature of at least 60°C.

Embodiment H46. The composition of any one of Embodiments H40 to H44 having a glass transition temperature between about 65°C and about 150°C.

Embodiment H47. The composition of any one of Embodiments H40 to H44 having a glass transition temperature between about 65°C and about 100°C.

Embodiment H48. The composition of any one of Embodiments H40 to H44 having a glass transition temperature of at least 70°C.

Embodiment H49. The composition of any one of Embodiments H40 to H44 having a glass transition temperature between about 70°C and about 95°C.

Embodiment H50. The composition of any one of Embodiments H40 to H44 having a glass transition temperature between about 80°C and about 90°C.

Embodiment H51. The composition of one of Embodiments H40 to H50, wherein the thermoplastic polymer is: (i) a polyvinylpyrrolidone polymer, (ii) a polyvinylpyrrolidone copolymer, (iii) an acrylic polymer, (iv) an acrylic copolymer, (v) hydroxypropyl methylcellulose acetate succinate, or (vi) a combination of two or more of the foregoing.

Embodiment H52. The composition of one of Embodiments H40 to H50, wherein the thermoplastic polymer is a copolymer of polyvinylpyrrolidone and vinyl acetate.

Embodiment H53. The composition of Embodiment H52, wherein the copolymer of polyvinylpyrrolidone and vinyl acetate has a mass ratio of polyvinylpyrrolidone to vinyl acetate of about 3:2.

Embodiment H54. The composition of one of Embodiments H40 to H50, wherein the thermoplastic polymer is a methyl methacrylate polymer, an ethyl acrylate polymer, or a copolymer of methyl methacrylate and ethyl methacrylate.

Embodiment H55. The composition of one of Embodiments H40 to H50, wherein the thermoplastic polymer is a copolymer of methacrylic acid and methyl methacrylate.

Embodiment H56. The composition of one of Embodiments H40 to H50, wherein the thermoplastic polymer is a copolymer of methacrylic acid and ethyl acrylate.

Embodiment H57. The composition of Embodiment H56, wherein the copolymer of methacrylic acid and ethyl acrylate has a mass ratio of methacrylic acid to ethyl acrylate of about 1:1.

Embodiment H58. The composition of one of Embodiments H40 to H57, comprising about 5 wt% to about 40 wt% of the adenosine A2A receptor antagonist.

Embodiment H59. The composition of one of Embodiments H40 to H57, comprising 20 wt% to 40 wt% of the adenosine A2A receptor antagonist.

Embodiment H60. The composition of one of Embodiments H40 to H57, comprising about 30 wt% of the adenosine A2A receptor antagonist.

Embodiment H61. The composition of one of Embodiments H40 to H58, comprising 60 wt% to about 90 wt% of the thermoplastic polymer.

Embodiment H62. The composition of one of Embodiments H40 to H59, comprising 60 wt% to 80 wt% of the thermoplastic polymer.

Embodiment H63. The composition of one of Embodiments H40 to H60, comprising about 70 wt% of the thermoplastic polymer.

Embodiment H64. The composition of any one of Embodiments H40 to H57, comprising about 20 wt% to about 40 wt% of the adenosine A2A receptor antagonist and about 60 wt% to about 80% of the thermoplastic polymer.

Embodiment H65. The composition of any one of Embodiments H40 to H57, comprising about 25 wt% to about 35 wt% of the adenosine A2A receptor antagonist and about 65 wt% to about 75% of the thermoplastic polymer.

Embodiment H66. The composition of any one of Embodiments H40 to H57, comprising about 30 wt% of the adenosine A2A receptor antagonist and about 70 wt% of the thermoplastic polymer.

Embodiment H67. The composition of one of Embodiments H40 to H66, wherein the weight ratio of the adenosine A2A receptor antagonist to the thermoplastic polymer is from about 1:4 to about 1:1.

Embodiment H68. The composition of one of Embodiments H40 to H66, wherein the weight ratio of the adenosine A2A receptor antagonist to the thermoplastic polymer is about 1:2.3.

Embodiment H69. The composition of any one of Embodiments H40 to H68, comprising from about 1 mg to about 1,000 mg of the adenosine A2A receptor antagonist.

Embodiment H70. The composition of any one of Embodiments H40 to H68, comprising from about 10 mg to about 800 mg of the adenosine A2A receptor antagonist.

Embodiment H71. The composition of any one of Embodiments H40 to H68, comprising 50 mg to 600 mg of the adenosine A2A receptor antagonist

Embodiment H72. The composition of any one of Embodiments H40 to H68, comprising from about 100 mg to about 400 mg of the adenosine A2A receptor antagonist.

Embodiment H73. The composition of any one of Embodiments H40 to H72 formed by hot melt extrusion.

Embodiment H74. A pharmaceutical formulation comprising the composition of any one of Embodiments H40 to H73.

Embodiment H75. The pharmaceutical composition of Embodiment H74, wherein the pharmaceutical formulation is an oral formulation.

Embodiment H76. The pharmaceutical composition of Embodiment H75, wherein the oral formulation is a capsule or a tablet.

Embodiment H77. A method of treating cancer in a patient in need thereof, the method comprising administering an effective amount of the composition of any one of Embodiments H40 to H73 or the pharmaceutical formulation of any one of Embodiments H74 to H76 to the patient to treat the cancer.

Embodiment H78. The method of Embodiment H77, wherein the cancer is non-small cell lung cancer, melanoma, renal cell cancer, breast cancer, colorectal cancer, bladder cancer, prostate cancer, or a head and neck cancer.

Embodiment H79. A process of forming the amorphous solid dispersion of any one of claims H1 to H34 or the composition of any one of claims H40 to H73, the process comprising the steps of: (i) adding the adenosine A2A receptor antagonist and the thermoplastic polymer to a device; (ii) melting the adenosine A2A receptor antagonist and the thermoplastic polymer; (iii) mixing the adenosine A2A receptor antagonist and the thermoplastic polymer to form a homogenous blend; (iv) extruding the homogenous blend to form the amorphous solid dispersion or the composition, respectively.

Embodiment H80. An extrudate comprising an amorphous adenosine A2A receptor antagonist or a pharmaceutically acceptable salt thereof dispersed in a thermoplastic polymer.

Embodiment H81. The extrudate of Embodiment H80, wherein the adenosine A2A receptor antagonist is a compound of Formula (I).

Embodiment H82. The extrudate of Embodiment H80, wherein the adenosine A2A receptor antagonist is a compound of Formula (II).

Embodiment H83. The extrudate of Embodiment H80, wherein the adenosine A2A receptor antagonist is a compound of Formula (III).

Embodiment H84. The extrudate of Embodiment H80, wherein the adenosine A2A receptor antagonist is a compound of Formula (IIIA), a compound of Formula (IIIB), or a mixture thereof.

Embodiment H85. The extrudate of any one of Embodiments H80 to H84 having a glass transition temperature of at least 60°C.

Embodiment H86. The extrudate of any one of Embodiments H80 to H84 having a glass transition temperature between about 65°C and about 150°C.

Embodiment H87. The extrudate of any one of Embodiments H80 to H84 having a glass transition temperature between 65°C and 100°C.

Embodiment H88. The extrudate of any one of Embodiments H80 to H84 having a glass transition temperature of at least 70°C.

Embodiment H89. The extrudate of any one of Embodiments H80 to H84 having a glass transition temperature between about 70°C and about 95°C.

Embodiment H90. The extrudate of any one of Embodiments H80 to H84 having a glass transition temperature between 80°C and 90°C.

Embodiment H91. The extrudate of one of Embodiments H80 to H90, wherein the thermoplastic polymer is: (i) a polyvinylpyrrolidone polymer, (ii) a polyvinylpyrrolidone copolymer, (iii) an acrylic polymer, (iv) an acrylic copolymer, (v) hydroxypropyl methylcellulose acetate succinate, or (vi) a combination of two or more of the foregoing.

Embodiment H92. The extrudate of one of Embodiments H80 to H90, wherein the thermoplastic polymer is a copolymer of polyvinylpyrrolidone and vinyl acetate.

Embodiment H93. The extrudate of Embodiment H92, wherein the copolymer of polyvinylpyrrolidone and vinyl acetate has a mass ratio of polyvinylpyrrolidone to vinyl acetate of about 3:2.

Embodiment H94. The extrudate of one of Embodiments H80 to H90, wherein the thermoplastic polymer is a methyl methacrylate polymer, an ethyl acrylate polymer, or a copolymer of methyl methacrylate and ethyl methacrylate.

Embodiment H95. The extrudate of one of Embodiments H80 to H90, wherein the thermoplastic polymer is a copolymer of methacrylic acid and methyl methacrylate.

Embodiment H96. The extrudate of one of Embodiments H80 to H90, wherein the thermoplastic polymer is a copolymer of methacrylic acid and ethyl acrylate.

Embodiment H97. The extrudate of Embodiment H96, wherein the copolymer of methacrylic acid and ethyl acrylate has a mass ratio of methacrylic acid to ethyl acrylate of about 1:1.

Embodiment H98. The extrudate of one of Embodiments H80 to H97, comprising about 5 wt% to about 40 wt% of the adenosine A2A receptor antagonist.

Embodiment H99. The extrudate of one of Embodiments H80 to H97, comprising 20 wt% to 40 wt% of the adenosine A2A receptor antagonist.

Embodiment H100. The extrudate of one of Embodiments H80 to H97, comprising about 30 wt% of the adenosine A2A receptor antagonist.

Embodiment H101. The extrudate of one of Embodiments H80 to H98, comprising 60 wt% to about 90 wt% of the thermoplastic polymer.

Embodiment H102. The extrudate of one of Embodiments H80 to H99, comprising 60 wt% to 80 wt% of the thermoplastic polymer.

Embodiment H103. The extrudate of one of Embodiments H80 to H100, comprising about 70 wt% of the thermoplastic polymer.

Embodiment H104. The extrudate of any one of Embodiments H80 to H97, comprising about 20 wt% to about 40 wt% of the adenosine A2A receptor antagonist and about 60 wt% to about 80% of the thermoplastic polymer.

Embodiment H105. The extrudate of any one of Embodiments 80 to 97, comprising about 25 wt% to about 35 wt% of the adenosine A2A receptor antagonist and about 65 wt% to about 75% of the thermoplastic polymer.

Embodiment H106. The extrudate of any one of Embodiments H80 to H97, comprising about 30 wt% of the adenosine A2A receptor antagonist and about 70 wt% of the thermoplastic polymer.

Embodiment H107. The extrudate of one of Embodiments H80 to H106, wherein the weight ratio of the adenosine A2A receptor antagonist to the thermoplastic polymer is from about 1:4 to about 1:1.

Embodiment H108. The extrudate of one of Embodiments H80 to H106, wherein the weight ratio of the adenosine A2A receptor antagonist to the thermoplastic polymer is about 1:2.3.

Embodiment H109. The extrudate of any one of Embodiments H80 to H108, comprising from about 1 mg to about 1,000 mg of the adenosine A2A receptor antagonist.

Embodiment H110. The extrudate of any one of Embodiments H80 to H108, comprising from about 10 mg to about 800 mg of the adenosine A2A receptor antagonist.

Embodiment H111. The extrudate of any one of Embodiments H80 to H108, comprising 50 mg to 600 mg of the adenosine A2A receptor antagonist

Embodiment H112. The extrudate of any one of Embodiments H80 to H108, comprising from about 100 mg to about 400 mg of the adenosine A2A receptor antagonist.

Embodiment H113. The extrudate of any one of Embodiments H80 to H112 formed by hot melt extrusion.

Embodiment H114. A pharmaceutical formulation comprising the extrudate of any one of Embodiments H80 to H113.

Embodiment H115. The pharmaceutical composition of Embodiment H114, wherein the pharmaceutical formulation is an oral formulation.

Embodiment H116. The pharmaceutical composition of Embodiment H115, wherein the oral formulation is a capsule or a tablet.

Embodiment H117. A method of treating cancer in a patient in need thereof, the method comprising administering an effective amount of the extrudate of any one of Embodiments H80 to H113 or the pharmaceutical formulation of any one of Embodiments H114 to H116 to the patient to treat the cancer.

Embodiment H118. The method of Embodiment H117, wherein the cancer is non-small cell lung cancer, melanoma, renal cell cancer, breast cancer, colorectal cancer, bladder cancer, prostate cancer, or a head and neck cancer.

Embodiment H119. The method of Embodiment H38, H39, H77, H78, H117, or H118, wherein the therapeutically effective amount is from about 1 mg to about 1,000 mg per day.

Embodiment H119. The method of Embodiment H38, H39, H77, H78, H117, or H118, wherein the therapeutically effective amount is from about 10 mg to about 600 mg per day.

Embodiment H119. The method of Embodiment H38, H39, H77, H78, H117, or H118, wherein the therapeutically effective amount is from about 100 mg to about 400 mg per day.

### Embodiments M1-M147

Embodiment M1. A pharmaceutical composition comprising an adenosine A2A receptor antagonist and a pharmaceutically acceptable polymer.

Embodiment M2. The pharmaceutical composition of Embodiment M1, wherein the adenosine A2A receptor antagonist is amorphous.

Embodiment M3. The pharmaceutical composition of Embodiment M1 or M2, wherein the adenosine A2A receptor antagonist is dispersed in the pharmaceutically acceptable polymer.

Embodiment M4. The pharmaceutical composition of any one of Embodiments M1 to M3, wherein the adenosine A2A receptor antagonist is a compound of Formula (I).

Embodiment M5. The pharmaceutical composition of any one of Embodiments M1 to M3, wherein the adenosine A2A receptor antagonist is a compound of Formula (II).

Embodiment M6. The pharmaceutical composition of any one of Embodiments M1 to M3, wherein the adenosine A2A receptor antagonist is a compound of Formula (III).

Embodiment M7. The pharmaceutical composition of any one of Embodiments M1 to M3, wherein the adenosine A2A receptor antagonist is a compound of Formula (IIIA), a compound of Formula (IIIB), or a mixture thereof.

Embodiment M8. The amorphous solid dispersion of any one of Embodiments M1 to M7, wherein the pharmaceutically acceptable polymer is an enteric polymer.

Embodiment M9. The pharmaceutical composition of any one of Embodiments M1 to M8, wherein the pharmaceutically acceptable polymer is hydroxypropyl methylcellulose acetate succinate.

Embodiment M10. The pharmaceutical composition of any one of Embodiments M1 to M8, wherein the pharmaceutically acceptable polymer is an acrylic polymer or an acrylic copolymer.

Embodiment M11. The pharmaceutical composition of any one of Embodiments M1 to M8, wherein the pharmaceutically acceptable polymer is a methyl methacrylate polymer, an ethyl acrylate polymer, or a copolymer of methyl methacrylate and ethyl acrylate.

Embodiment M12. The pharmaceutical composition of any one of Embodiments M1 to M8, wherein the pharmaceutically acceptable polymer is a copolymer of methacrylic acid and methyl methacrylate.

Embodiment M13. The pharmaceutical composition of Embodiment M12, wherein the copolymer of methacrylic acid and methyl methacrylate has a mass ratio of methacrylic acid to methyl methacrylate from about 1:1.

Embodiment M14. The pharmaceutical composition of any one of Embodiments M1 to M8, wherein the pharmaceutically acceptable polymer is a copolymer of methacrylic acid and ethyl acrylate.

Embodiment M15. The pharmaceutical composition of Embodiment M14, wherein the copolymer of methacrylic acid and ethyl acrylate has a mass ratio of methacrylic acid to ethyl acrylate of about 1:1.

Embodiment M16. The pharmaceutical composition of any one of Embodiments M1 to M15 comprising about 5 wt% to about 30 wt% of the adenosine A2A receptor antagonist.

Embodiment M17. The pharmaceutical composition of any one of Embodiments M1 to M15 comprising 5 wt% to 25 wt% of the adenosine A2A receptor antagonist.

Embodiment M18. The pharmaceutical composition of any one of Embodiments M1 to M15 comprising 10 wt% to 25 wt% of the adenosine A2A receptor antagonist.

Embodiment M19. The pharmaceutical composition of any one of Embodiments M1 to M15 comprising 15 wt% to 25 wt% of the adenosine A2A receptor antagonist.

Embodiment M20. The pharmaceutical composition of any one of Embodiments M1 to M15 comprising about 20 wt% of the adenosine A2A receptor antagonist.

Embodiment M21. The pharmaceutical composition of any one of Embodiments M1 to M16, comprising 70 wt% to 95 wt% of the pharmaceutically acceptable polymer.

Embodiment M22. The pharmaceutical composition of any one of Embodiments M1 to M17, comprising 75 wt% to 95 wt% of the pharmaceutically acceptable polymer.

Embodiment M23. The pharmaceutical composition of any one of Embodiments M1 to M18, comprising 75 wt% to 90 wt% of the pharmaceutically acceptable polymer.

Embodiment M24. The pharmaceutical composition of any one of Embodiments M1 to M19, comprising 75 wt% to 85 wt% of the pharmaceutically acceptable polymer.

Embodiment M25. The pharmaceutical composition of any one of Embodiments M1 to M20, comprising about 80 wt% of the pharmaceutically acceptable polymer.

Embodiment M26. The pharmaceutical composition of any one of Embodiments M1 to M15, comprising about 5 wt% to about 30 wt% of the adenosine A2A receptor antagonist and about 70 wt% to about 95% of the pharmaceutically acceptable polymer.

Embodiment M27. The pharmaceutical composition of any one of Embodiments M1 to M15, comprising about 5 wt% to about 25 wt% of the adenosine A2A receptor antagonist and about 75 wt% to about 95% of the pharmaceutically acceptable polymer.

Embodiment M28. The pharmaceutical composition of any one of Embodiments M1 to M15, comprising about 10 wt% to about 25 wt% of the adenosine A2A receptor antagonist and about 75 wt% to about 90% of the pharmaceutically acceptable polymer.

Embodiment M29. The pharmaceutical composition of any one of Embodiments M1 to M15, comprising about 15 wt% to about 25 wt% of the adenosine A2A receptor antagonist and about 75 wt% to about 85% of the pharmaceutically acceptable polymer.

Embodiment M30. The pharmaceutical composition of any one of Embodiments M1 to M15, comprising about 20 wt% of the adenosine A2A receptor antagonist and about 80 wt% of the pharmaceutically acceptable polymer.

Embodiment M31. The pharmaceutical composition of any one of Embodiments M1 to M15, wherein the weight ratio of the adenosine A2A receptor antagonist to the pharmaceutically acceptable polymer is from about 1:19 to about 1:3.

Embodiment M32. The pharmaceutical composition of any one of Embodiments M1 to M15, wherein the weight ratio of the adenosine A2A receptor antagonist to the pharmaceutically acceptable polymer is from about 1:6 to about 1:3.

Embodiment M33. The pharmaceutical composition of any one of Embodiments M1 to M15, wherein the weight ratio of the adenosine A2A receptor antagonist to the pharmaceutically acceptable polymer is about 1:4.

Embodiment M34. The pharmaceutical composition of any one of Embodiments M1 to M33, comprising from about 1 mg to about 1,000 mg of the adenosine A2A receptor antagonist.

Embodiment M35. The pharmaceutical composition of any one of Embodiments M1 to M33, comprising from about 10 mg to about 800 mg of the adenosine A2A receptor antagonist.

Embodiment M36. The pharmaceutical composition of any one of Embodiments M1 to M33, comprising about 50 mg to about 600 mg of the adenosine A2A receptor antagonist

Embodiment M37. The pharmaceutical composition of any one of Embodiments M1 to M33, comprising from about 100 mg to about 400 mg of the adenosine A2A receptor antagonist.

Embodiment M38. The pharmaceutical composition of any one of Embodiments M1 to M37, prepared by the process comprising the steps of: (i) dissolving the adenosine A2A receptor antagonist and the pharmaceutically acceptable polymer in a polar aprotic solvent; and (ii) co-precipitating the adenosine A2A receptor antagonist and the pharmaceutically acceptable polymer with an anti-solvent, thereby forming the pharmaceutical composition.

Embodiment M39. The pharmaceutical composition of Embodiment M38, wherein the anti-solvent is acidified water.

Embodiment M40. The pharmaceutical composition of Embodiment M38 or M39, wherein the anti-solvent has a pH less than 5.

Embodiment M41. The pharmaceutical composition of any one of Embodiments M38 to M40, wherein the anti-solvent has a pH less than 4.

Embodiment M42. The pharmaceutical composition of any one of Embodiments M38 to M41, wherein the temperature of the anti-solvent is less than room temperature.

Embodiment M43. The pharmaceutical composition of any one of Embodiments M38 to M41, wherein the temperature of the anti-solvent is from about 2°C to about 8°C.

Embodiment M44. A formulation comprising the pharmaceutical composition of any one of Embodiments M1 to M43.

Embodiment M45. The formulation of Embodiment M44, wherein the formulation is an oral formulation.

Embodiment M46. The formulation of Embodiment M45, wherein the oral formulation is a capsule, a tablet, or a powder.

Embodiment M47. The formulation of any one of Embodiments M44 to M46, further comprising an outer layer.

Embodiment M48. A method of treating cancer in a patient in need thereof, the method comprising administering to the patient an effective amount of the pharmaceutical composition of any one of Embodiments M1 to M43 or the formulation of any one of Embodiments M44 to M47 to treat the cancer.

Embodiment M49. The method of Embodiment M48, wherein the cancer is non-small cell lung cancer, melanoma, renal cell cancer, breast cancer, colorectal cancer, bladder cancer, prostate cancer, or a head and neck cancer.

Embodiment M50. An amorphous solid dispersion comprising an adenosine A2A receptor antagonist and a pharmaceutically acceptable polymer.

Embodiment M51. The amorphous solid dispersion of Embodiment M50, wherein the adenosine A2A receptor antagonist is amorphous.

Embodiment M52. The amorphous solid dispersion of Embodiment M50 or M51, wherein the adenosine A2A receptor antagonist is dispersed in the pharmaceutically acceptable polymer.

Embodiment M53. The amorphous solid dispersion of any one of Embodiments M50 to M52, wherein the adenosine A2A receptor antagonist is a compound of Formula (I).

Embodiment M54. The amorphous solid dispersion of any one of Embodiments M50 to M52, wherein the adenosine A2A receptor antagonist is a compound of Formula (II).

Embodiment M55. The amorphous solid dispersion of any one of Embodiments M50 to M52, wherein the adenosine A2A receptor antagonist is a compound of Formula (III).

Embodiment M56. The amorphous solid dispersion of any one of Embodiments M50 to M52, wherein the adenosine A2A receptor antagonist is a compound of Formula (IIIA), a compound of Formula (IIIB), or a mixture thereof.

Embodiment M57. The amorphous solid dispersion of any one of Embodiments M50 to M56, wherein the pharmaceutically acceptable polymer is an enteric polymer.

Embodiment M58. The amorphous solid dispersion of any one of Embodiments M50 to M57, wherein the pharmaceutically acceptable polymer is hydroxypropyl methylcellulose acetate succinate.

Embodiment M59. The amorphous solid dispersion of any one of Embodiments M50 to M57, wherein the pharmaceutically acceptable polymer is an acrylic polymer or an acrylic copolymer.

Embodiment M60. The amorphous solid dispersion of any one of Embodiments M50 to M57, wherein the pharmaceutically acceptable polymer is a methyl methacrylate polymer, an ethyl acrylate polymer, or a copolymer of methyl methacrylate and ethyl acrylate.

Embodiment M61. The amorphous solid dispersion of any one of Embodiments M50 to M57, wherein the pharmaceutically acceptable polymer is a copolymer of methacrylic acid and methyl methacrylate.

Embodiment M62. The amorphous solid dispersion of Embodiment M61, wherein the copolymer of methacrylic acid and methyl methacrylate has a mass ratio of methacrylic acid to methyl methacrylate from about 1:1.

Embodiment M63. The amorphous solid dispersion of any one of Embodiments M50 to M57, wherein the pharmaceutically acceptable polymer is a copolymer of methacrylic acid and ethyl acrylate.

Embodiment M64. The amorphous solid dispersion of Embodiment M63, wherein the copolymer of methacrylic acid and ethyl acrylate has a mass ratio of methacrylic acid to ethyl acrylate of about 1:1.

Embodiment M65. The amorphous solid dispersion of any one of Embodiments M50 to M64, comprising about 5 wt% to about 30 wt% of the adenosine A2A receptor antagonist.

Embodiment M66. The amorphous solid dispersion of any one of Embodiments M50 to M64, comprising 5 wt% to 25 wt% of the adenosine A2A receptor antagonist.

Embodiment M67. The amorphous solid dispersion of any one of Embodiments M50 to M64, comprising 10 wt% to 25 wt% of the adenosine A2A receptor antagonist.

Embodiment M68. The amorphous solid dispersion of any one of Embodiments M50 to M64, comprising 15 wt% to 25 wt% of the adenosine A2A receptor antagonist.

Embodiment M69. The amorphous solid dispersion of any one of Embodiments M50 to M64, comprising about 20 wt% of the adenosine A2A receptor antagonist.

Embodiment M70. The amorphous solid dispersion of any one of Embodiments M50 to M65, comprising 70 wt% to 95 wt% of the pharmaceutically acceptable polymer.

Embodiment M71. The amorphous solid dispersion of any one of Embodiments M50 to M66, comprising 75 wt% to 95 wt% of the pharmaceutically acceptable polymer.

Embodiment M72. The amorphous solid dispersion of any one of Embodiments M50 to M67, comprising 75 wt% to 90 wt% of the pharmaceutically acceptable polymer.

Embodiment M73. The amorphous solid dispersion of any one of Embodiments M50 to M68, comprising 75 wt% to 85 wt% of the pharmaceutically acceptable polymer.

Embodiment M74. The amorphous solid dispersion of any one of Embodiments M50 to M69, comprising about 80 wt% of the pharmaceutically acceptable polymer.

Embodiment M75. The amorphous solid dispersion of any one of Embodiments M50 to M64, comprising about 5 wt% to about 30 wt% of the adenosine A2A receptor antagonist and about 70 wt% to about 95% of the pharmaceutically acceptable polymer.

Embodiment M76. The amorphous solid dispersion of any one of Embodiments M50 to M64, comprising about 5 wt% to about 25 wt% of the adenosine A2A receptor antagonist and about 75 wt% to about 95% of the pharmaceutically acceptable polymer.

Embodiment M77. The amorphous solid dispersion of any one of Embodiments M50 to M64, comprising about 10 wt% to about 25 wt% of the adenosine A2A receptor antagonist and about 75 wt% to about 90% of the pharmaceutically acceptable polymer.

Embodiment M78. The amorphous solid dispersion of any one of Embodiments M50 to M64, comprising about 15 wt% to about 25 wt% of the adenosine A2A receptor antagonist and about 75 wt% to about 85% of the pharmaceutically acceptable polymer.

Embodiment M79. The amorphous solid dispersion of any one of Embodiments M50 to M64, comprising about 20 wt% of the adenosine A2A receptor antagonist and about 80 wt% of the pharmaceutically acceptable polymer.

Embodiment M80. The amorphous solid dispersion of any one of Embodiments M50 to M64, wherein the weight ratio of the adenosine A2A receptor antagonist to the pharmaceutically acceptable polymer is from about 1:19 to about 1:3.

Embodiment M81. The amorphous solid dispersion of any one of Embodiments M50 to M64, wherein the weight ratio of the adenosine A2A receptor antagonist to the pharmaceutically acceptable polymer is from about 1:6 to about 1:3.

Embodiment M82. The amorphous solid dispersion of any one of Embodiments M50 to M64, wherein the weight ratio of the adenosine A2A receptor antagonist to the pharmaceutically acceptable polymer is about 1:4.

Embodiment M83. The amorphous solid dispersion of any one of Embodiments M50 to M82, comprising from about 1 mg to about 1,000 mg of the adenosine A2A receptor antagonist.

Embodiment M84. The amorphous solid dispersion of any one of Embodiments M50 to M82, comprising from about 10 mg to about 800 mg of the adenosine A2A receptor antagonist.

Embodiment M85. The amorphous solid dispersion of any one of Embodiments M50 to M82, comprising about 50 mg to about 600 mg of the adenosine A2A receptor antagonist

Embodiment M86. The amorphous solid dispersion of any one of Embodiments M50 to M82, comprising from about 100 mg to about 400 mg of the adenosine A2A receptor antagonist.

Embodiment M87. The amorphous solid dispersion of any one of Embodiments M50 to M86, prepared by the process comprising the steps of: (i) dissolving the adenosine A2A receptor antagonist and the pharmaceutically acceptable polymer in a polar aprotic solvent; and (ii) co-precipitating the adenosine A2A receptor antagonist and the pharmaceutically acceptable polymer with an anti-solvent, thereby forming the amorphous solid dispersion.

Embodiment M88. The amorphous solid dispersion of Embodiment M87, wherein the anti-solvent is acidified water.

Embodiment M89. The amorphous solid dispersion of Embodiment M87 or M88, wherein the anti-solvent has a pH less than 5.

Embodiment M90. The amorphous solid dispersion of any one of Embodiments M87 to M89, wherein the anti-solvent has a pH less than 4.

Embodiment M91. The amorphous solid dispersion of any one of Embodiments M87 to M90, wherein the temperature of the anti-solvent is less than room temperature.

Embodiment M92. The amorphous solid dispersion of any one of Embodiments M87 to M91, wherein the temperature of the anti-solvent is from about 2°C to about 8°C.

Embodiment M93. A formulation comprising the amorphous solid dispersion of any one of Embodiments M50 to M92.

Embodiment M94. The formulation of Embodiment M93, wherein the formulation is an oral formulation.

Embodiment M95. The formulation of Embodiment M94, wherein the oral formulation is a capsule, a tablet, or a powder.

Embodiment M96. The formulation of any one of Embodiments M93 to M95, further comprising an outer layer.

Embodiment M97. A method of treating cancer in a patient in need thereof, the method comprising administering to the patient an effective amount of the amorphous solid dispersion of any one of Embodiments M50 to M92 or the formulation of any one of Embodiments M93 to M96 to treat the cancer.

Embodiment M98. The method of Embodiment M97, wherein the cancer is non-small cell lung cancer, melanoma, renal cell cancer, breast cancer, colorectal cancer, bladder cancer, prostate cancer, or a head and neck cancer.

Embodiment M99. A microprecipitated bulk powder comprising an adenosine A2A receptor antagonist and a pharmaceutically acceptable polymer.

Embodiment M100. The microprecipitated bulk powder of Embodiment M99, wherein the adenosine A2A receptor antagonist is amorphous.

Embodiment M101. The microprecipitated bulk powder of Embodiment M99 or M100, wherein the adenosine A2A receptor antagonist is dispersed in the pharmaceutically acceptable polymer.

Embodiment M102. The microprecipitated bulk powder of any one of Embodiments M99 to M101, wherein the adenosine A2A receptor antagonist is a compound of Formula (I).

Embodiment M103. The microprecipitated bulk powder of any one of Embodiments M99 to M101, wherein the adenosine A2A receptor antagonist is a compound of Formula (II).

Embodiment M104. The microprecipitated bulk powder of any one of Embodiments M99 to M101, wherein the adenosine A2A receptor antagonist is a compound of Formula (III).

Embodiment M105. The microprecipitated bulk powder of any one of Embodiments M99 to M101, wherein the adenosine A2A receptor antagonist is a compound of Formula (IIIA), a compound of Formula (IIIB), or a mixture thereof.

Embodiment M106. The amorphous solid dispersion of any one of Embodiments M99 to M105, wherein the pharmaceutically acceptable polymer is an enteric polymer.

Embodiment M107. The microprecipitated bulk powder of any one of Embodiments M99 to M106, wherein the pharmaceutically acceptable polymer is hydroxypropyl methylcellulose acetate succinate.

Embodiment M108. The microprecipitated bulk powder of any one of Embodiments M99 to M106, wherein the pharmaceutically acceptable polymer is an acrylic polymer or an acrylic copolymer.

Embodiment M109. The microprecipitated bulk powder of any one of Embodiments M99 to M106, wherein the pharmaceutically acceptable polymer is a methyl methacrylate polymer, an ethyl acrylate polymer, or a copolymer of methyl methacrylate and ethyl acrylate.

Embodiment M110. The microprecipitated bulk powder of any one of Embodiments M99 to M106, wherein the pharmaceutically acceptable polymer is a copolymer of methacrylic acid and methyl methacrylate.

Embodiment M111.The microprecipitated bulk powder of Embodiment M110, wherein the copolymer of methacrylic acid and methyl methacrylate has a mass ratio of methacrylic acid to methyl methacrylate from about 1:1.

Embodiment M112. The microprecipitated bulk powder of any one of Embodiments M99 to M106, wherein the pharmaceutically acceptable polymer is a copolymer of methacrylic acid and ethyl acrylate.

Embodiment M113. The microprecipitated bulk powder of Embodiment M112, wherein the copolymer of methacrylic acid and ethyl acrylate has a mass ratio of methacrylic acid to ethyl acrylate of about 1:1.

Embodiment M114. The microprecipitated bulk powder of any one of Embodiments M99 to M113, comprising about 5 wt% to about 30 wt% of the adenosine A2A receptor antagonist.

Embodiment M115. The microprecipitated bulk powder of any one of Embodiments M99 to M113, comprising 5 wt% to 25 wt% of the adenosine A2A receptor antagonist.

Embodiment M116. The microprecipitated bulk powder of any one of Embodiments M99 to M113, comprising 10 wt% to 25 wt% of the adenosine A2A receptor antagonist.

Embodiment M117. The microprecipitated bulk powder of any one of Embodiments M99 to M113, comprising 15 wt% to 25 wt% of the adenosine A2A receptor antagonist.

Embodiment M118. The microprecipitated bulk powder of any one of Embodiments M99 to M113, comprising about 20 wt% of the adenosine A2A receptor antagonist.

Embodiment M119. The microprecipitated bulk powder of any one of Embodiments M99 to M114, comprising 70 wt% to 95 wt% of the pharmaceutically acceptable polymer.

Embodiment M120. The microprecipitated bulk powder of any one of Embodiments M99 to M115, comprising 75 wt% to 95 wt% of the pharmaceutically acceptable polymer.

Embodiment M121. The microprecipitated bulk powder of any one of Embodiments M99 to M116, comprising 75 wt% to 90 wt% of the pharmaceutically acceptable polymer.

Embodiment M122. The microprecipitated bulk powder of any one of Embodiments M99 to M117, comprising 75 wt% to 85 wt% of the pharmaceutically acceptable polymer.

Embodiment M123. The microprecipitated bulk powder of any one of Embodiments M99 to M118, comprising about 80 wt% of the pharmaceutically acceptable polymer.

Embodiment M124. The microprecipitated bulk powder of any one of Embodiments M99 to M113, comprising about 5 wt% to about 30 wt% of the adenosine A2A receptor antagonist and about 70 wt% to about 95% of the pharmaceutically acceptable polymer.

Embodiment M125. The microprecipitated bulk powder of any one of Embodiments M99 to M113, comprising about 5 wt% to about 25 wt% of the adenosine A2A receptor antagonist and about 75 wt% to about 95% of the pharmaceutically acceptable polymer.

Embodiment M126. The microprecipitated bulk powder of any one of Embodiments M99 to M113, comprising about 10 wt% to about 25 wt% of the adenosine A2A receptor antagonist and about 75 wt% to about 90% of the pharmaceutically acceptable polymer.

Embodiment M127. The microprecipitated bulk powder of any one of Embodiments M99 to M113, comprising about 15 wt% to about 25 wt% of the adenosine A2A receptor antagonist and about 75 wt% to about 85% of the pharmaceutically acceptable polymer.

Embodiment M128. The microprecipitated bulk powder of any one of Embodiments M99 to M113, comprising about 20 wt% of the adenosine A2A receptor antagonist and about 80 wt% of the pharmaceutically acceptable polymer.

Embodiment M129. The microprecipitated bulk powder of any one of Embodiments M99 to M113, wherein the weight ratio of the adenosine A2A receptor antagonist to the pharmaceutically acceptable polymer is from about 1:19 to about 1:3.

Embodiment M130. The microprecipitated bulk powder of any one of Embodiments M99 to M113, wherein the weight ratio of the adenosine A2A receptor antagonist to the pharmaceutically acceptable polymer is from about 1:6 to about 1:3.

Embodiment M131. The microprecipitated bulk powder of any one of Embodiments M99 to M113, wherein the weight ratio of the adenosine A2A receptor antagonist to the pharmaceutically acceptable polymer is about 1:4.

Embodiment M132. The microprecipitated bulk powder of any one of Embodiments M99 to M131, comprising from about 1 mg to about 1,000 mg of the adenosine A2A receptor antagonist.

Embodiment M133. The microprecipitated bulk powder of any one of Embodiments M99 to M131, comprising from about 10 mg to about 800 mg of the adenosine A2A receptor antagonist.

Embodiment M134. The microprecipitated bulk powder of any one of Embodiments M99 to M131, comprising about 50 mg to about 600 mg of the adenosine A2A receptor antagonist

Embodiment M135. The microprecipitated bulk powder of any one of Embodiments M99 to M131, comprising from about 100 mg to about 400 mg of the adenosine A2A receptor antagonist.

Embodiment M136. The microprecipitated bulk powder of any one of Embodiments M99 to M135, prepared by the process comprising the steps of: (i) dissolving the adenosine A2A receptor antagonist and the pharmaceutically acceptable polymer in a polar aprotic solvent; and (ii) co-precipitating the adenosine A2A receptor antagonist and the pharmaceutically acceptable polymer with an anti-solvent, thereby forming the microprecipitated bulk powder.

Embodiment M137. The microprecipitated bulk powder of Embodiment M136, wherein the anti-solvent is acidified water.

Embodiment M138. The microprecipitated bulk powder of Embodiment M136 or M137, wherein the anti-solvent has a pH less than 5.

Embodiment M139. The microprecipitated bulk powder of any one of Embodiments M136 to M138, wherein the anti-solvent has a pH less than 4.

Embodiment M140. The microprecipitated bulk powder of any one of Embodiments M136 to M139, wherein the temperature of the anti-solvent is less than room temperature.

Embodiment M141. The microprecipitated bulk powder of any one of Embodiments M136 to M140, wherein the temperature of the anti-solvent is from about 2°C to about 8°C.

Embodiment M142. A formulation comprising the microprecipitated bulk powder of any one of Embodiments M99 to M141.

Embodiment M143. The formulation of Embodiment M142, wherein the formulation is an oral formulation.

Embodiment M144. The formulation of Embodiment M143, wherein the oral formulation is a capsule, a tablet, or a powder.

Embodiment M145. The formulation of any one of Embodiments M142 to M144, further comprising an outer layer.

Embodiment M146. A method of treating cancer in a patient in need thereof, the method comprising administering to the patient an effective amount of the microprecipitated bulk powder of any one of Embodiments M99 to M141 or the formulation of any one of Embodiments M142 to M145 to treat the cancer.

Embodiment M147. The method of Embodiment M146, wherein the cancer is non-small cell lung cancer, melanoma, renal cell cancer, breast cancer, colorectal cancer, bladder cancer, prostate cancer, or a head and neck cancer.

Embodiment M148. The method of Embodiment M48, M49, M97, M98, M146, or M147, wherein the therapeutically effective amount is from about 1 mg to about 1,000 mg per day.

Embodiment M148. The method of Embodiment M48, M49, M97, M98, M146, or M147, wherein the therapeutically effective amount is from about 10 mg to about 600 mg per day.

Embodiment M148. The method of Embodiment M48, M49, M97, M98, M146, or M147, wherein the therapeutically effective amount is from about 100 mg to about 400 mg per day.

### Embodiments N1-N55

Embodiment N1. A pharmaceutical composition comprising (i) adenosine A2A receptor antagonist or a pharmaceutically acceptable salt thereof having a particle size distribution with a D90 of about 1000 nm or less, as measured by laser diffraction spectroscopy; and (ii) a pharmaceutically acceptable polymer, a surfactant, or a combination thereof.

Embodiment N2. The composition of Embodiment N1, wherein the adenosine A2A receptor antagonist is a compound of Formula (I) or a pharmaceutically acceptable salt thereof.

Embodiment N3. The composition of Embodiment N1, wherein the adenosine A2A receptor antagonist is a compound of Formula (II) or a pharmaceutically acceptable salt thereof.

Embodiment N4. The composition of Embodiment N, wherein the adenosine A2A receptor antagonist is a compound of Formula (III) or a pharmaceutically acceptable salt thereof.

Embodiment N5. The composition of Embodiment N1, wherein the adenosine A2A receptor antagonist is a compound of Formula (IIIA) or a pharmaceutically acceptable salt thereof.

Embodiment N6. The composition of Embodiment N1, wherein the adenosine A2A receptor antagonist is a compound of Formula (IIIB) or a pharmaceutically acceptable salt thereof.

Embodiment N7. The composition of any one of Embodiments N1 to N6, wherein the adenosine A2A receptor antagonist is crystalline.

Embodiment N8. The composition of any one of Embodiments N1 to N7, wherein the drug nanoparticles have a particle size distribution with a D90 of about 500 nm or less, as measured by laser diffraction spectroscopy.

Embodiment N9. The composition of any one of Embodiments N1 to N7, wherein the drug nanoparticles have a particle size distribution with a D90 of about 250 nm or less, as measured by laser diffraction spectroscopy.

Embodiment N10. The composition of any one of Embodiments N1 to N7, wherein the drug nanoparticles have a particle size distribution with a D90 of about 150 nm or less, as measured by laser diffraction spectroscopy.

Embodiment N11. The composition of any one of Embodiments N1 to N7, wherein the drug nanoparticles have a particle size distribution with a D90 of about 100 nm or less, as measured by laser diffraction spectroscopy.

Embodiment N12. The composition of any one of Embodiments N1 to N11, comprising from about 5 wt% to about 50 wt% of the adenosine A2A receptor antagonist.

Embodiment N13. The composition of any one of Embodiments N1 to N11, comprising from about 10 wt% to about 45 wt% of the adenosine A2A receptor antagonist.

Embodiment N14. The composition of any one of Embodiments N1 to N11, comprising from about 15 wt% to about 40 wt% of the adenosine A2A receptor antagonist.

Embodiment N15. The composition of any one of Embodiments N1 to N11, comprising from about 20 wt% to about 35 wt% of the adenosine A2A receptor antagonist.

Embodiment N16. The composition of any one of Embodiments N1 to N11, comprising from about 25 wt% to about 35 wt% of the adenosine A2A receptor antagonist.

Embodiment N17. The composition of any one of Embodiments N1 to N11, comprising about 30 wt% of the adenosine A2A receptor antagonist.

Embodiment N18. The composition of any one of Embodiments N1 to N17, wherein (ii) comprises the pharmaceutically acceptable polymer and the surfactant.

Embodiment N19. The composition of any one of Embodiments N1 to N18, wherein the weight ratio of (i) to (ii) is from about 30:1 to about 2:1.

Embodiment N20. The composition of any one of Embodiments N1 to N18, wherein the weight ratio of (i) to (ii) is from about 20:1 to about 2:1.

Embodiment N21. The composition of any one of Embodiments N1 to N18, wherein the weight ratio of (i) to (ii) is from about 10:1 to about 2:1.

Embodiment N22. The composition of any one of Embodiments N1 to N18, wherein the weight ratio of (i) to (ii) is from about 5:1 to about 2:1.

Embodiment N23. The composition of any one of Embodiments N18 to N22, wherein the surfactant is an anionic surfactant.

Embodiment N24. The composition of Embodiment N23, wherein the anionic surfactant or its salt is a carboxylate, a sulfate, a sulfonate, a phosphate ester, a bile salt, or a combination of two or more thereof.

Embodiment N25. The composition of Embodiment N23, wherein the anionic surfactant or its salt is sodium lauryl sulfate, ammonium lauryl sulfate, sodium laureth sulfate, sodium myreth sulfate, sodium stearate, dioctyl sodium sulfosuccinate, or a combination of two or more thereof.

Embodiment N26. The composition of Embodiment N23, wherein the anionic surfactant is dioctyl sodium sulfosuccinate.

Embodiment N27. The composition of any one of Embodiments N18 to N26, wherein the surfactant is present in an amount from about 0.005 wt% to about 2.0 wt%.

Embodiment N28. The composition of any one of Embodiments N18 to N26, wherein the surfactant is present in an amount from about 0.005 wt% to about 1.0 wt%.

Embodiment N29. The composition of any one of Embodiments N18 to N26, wherein the surfactant is present in an amount from about 0.05 wt% to about 0.5 wt%.

Embodiment N30. The composition of any one of Embodiments N18 to N29, wherein the pharmaceutically acceptable polymer comprises a polyvinylpyrrolidone polymer, a polyvinylpyrrolidone copolymer, a cellulose compound, or a combination of two or more of the foregoing.

Embodiment N31. The composition of Embodiment N30, wherein the cellulose compound is hydroxypropyl cellulose, hydroxyethyl cellulose, hydroxyethylmethyl cellulose, hydroxyethyl cellulose, hydroxypropylmethyl cellulose, methyl cellulose, ethylmethyl cellulose, carboxymethyl cellulose, or a combination of two or more thereof.

Embodiment N32. The composition of Embodiment N30, wherein the cellulose compound comprises hydroxypropyl cellulose.

Embodiment N33. The composition of any one of Embodiments N30 to N32, wherein the polyvinylpyrrolidone copolymer comprises a copolymer of pyrrolidone and vinyl acetate.

Embodiment N34. The composition Embodiment N33, wherein the weight ratio of pyrrolidone to vinyl acetate is about 3:2.

Embodiment N35. The composition of any one of Embodiments N18 to N34, wherein the pharmaceutically acceptable polymer has an average molecular weight from about 100 Daltons to about 140,000 Daltons.

Embodiment N36. The composition of any one of Embodiments N18 to N34, wherein the pharmaceutically acceptable polymer has an average molecular weight from about 60,000 Daltons to about 120,000 Daltons.

Embodiment N37. The composition of any one of Embodiments N18 to N36, comprising from about 0.1 wt% to about 15 wt% of the pharmaceutically acceptable polymer.

Embodiment N38. The composition of any one of Embodiments N18 to N36, comprising from about 0.5 wt% to about 10 wt% of the pharmaceutically acceptable polymer.

Embodiment N39. The composition of any one of Embodiments N1 to N38, wherein the pharmaceutically acceptable polymer is a non-crosslinked polymer.

Embodiment N40. The composition of any one of Embodiments N1 to N39, comprising from about 1 mg to about 1,000 mg of the adenosine A2A receptor antagonist or the pharmaceutically acceptable salt thereof.

Embodiment N41. The composition of any one of Embodiments N1 to N39, comprising from about 10 mg to about 800 mg of the adenosine A2A receptor antagonist or the pharmaceutically acceptable salt thereof.

Embodiment N42. The composition of any one of Embodiments N1 to N39, comprising from about 50 mg to about 600 mg of the adenosine A2A receptor antagonist or the pharmaceutically acceptable salt thereof.

Embodiment N43. The composition of any one of Embodiments N1 to N39, comprising from about 100 mg to about 400 mg of the adenosine A2A receptor antagonist or the pharmaceutically acceptable salt thereof.

Embodiment N44. The composition of any one of Embodiments N1 to N39, comprising about 50 mg, about 100 mg, about 150 mg, about 200 mg, about 250 mg, about 300 mg, about 350 mg, about 400 mg, about 450 mg, about 500 mg, about 550 mg, or about 600 mg of the adenosine A2A receptor antagonist or the pharmaceutically acceptable salt thereof.

Embodiment N45. An oral pharmaceutical formulation comprising the composition of any one of Embodiments N1 to N44.

Embodiment N46. A tablet comprising the pharmaceutical composition of any one of Embodiments N1 to N44.

Embodiment N47. The tablet of Embodiment N46, further comprising an outer coating selected from a protective coating, an aesthetic coating, an enteric coating, or a combination thereof, in one layer or multiple layers.

Embodiment N48. A powder comprising the pharmaceutical composition of any one of Embodiments N1 to N44.

Embodiment N49. A sachet or a stick pack comprising the powder of Embodiment N48.

Embodiment N50. A granule comprising the pharmaceutical composition of any one of Embodiments N1 to N44.

Embodiment N51. The granule of Embodiment N50, further comprising an outer coating.

Embodiment N52. A bead comprising an inert core and a drug layer, wherein the drug layer comprise the pharmaceutical composition of any one of Embodiments N1 to N44.

Embodiment N53. The bead of Embodiment N52, further comprising an outer coating.

Embodiment N54. A capsule comprising the pharmaceutical composition of any one of Embodiments N1 to N44, the powder of Embodiment N48, the granule of Embodiment N50 or N51, or the bead of Embodiment N52 or N53, encapsulated within a capsule shell.

Embodiment N55. The capsule of Embodiment N54, further comprising an outer coating selected from a protective coating, an aesthetic coating, an enteric coating, or a combination thereof, in one layer or multiple layers.

Embodiment N55. A method of treating cancer in a subject in need thereof, the method comprising administering to the subject a therapeutically effective amount of the pharmaceutical composition of one of Embodiments N1 to N44; the oral pharmaceutical formulation of Embodiment N45; the tablet of Embodiment N46 or N47; the powder of Embodiment N48; the granule of Embodiment N50 or N51; the bead of Embodiment N52 or N53; or the capsule of Embodiment N54 or N55; to treat the cancer.

Embodiment N56. The method of Embodiment N55, wherein the cancer is lung cancer, melanoma, renal cell cancer, breast cancer, colorectal cancer, bladder cancer, prostate cancer, or a head and neck cancer.

Embodiment N57. The method of Embodiment N55 or N56, wherein the therapeutically effective amount is from about 1 mg to about 1,000 mg per day.

Embodiment N57. The method of Embodiment N55 or N56, wherein the therapeutically effective amount is from about 10 mg to about 600 mg per day.

Embodiment N57. The method of Embodiment N55 or N56, wherein the therapeutically effective amount is from about 100 mg to about 400 mg per day.

### EXAMPLES

Abbreviations used herein: API: Active Pharmaceutical Ingredient, which is Formula (III) in the examples; ASD: Amorphous Solid Dispersion; RRT: Relative retention time (in HPLC); SGF: Simulated gastric fluid; XRPD: X-ray powder diffraction; FaSSGF: fasted state simulated gastric fluid; FeSSGF: fed state simulated gastric fluid; FeSSIF: fed state simulated intestinal fluid; FaSSIF: fasted state simulated intestinal fluid. YTZ: yttria-stabilized zirconia.

### Example 1: Pharmaceutical Compositions of Embodiment H

To understand the feasibility for use of amorphous solid dispersions of Formula (III), polymers were selected for initial formulation screening. Compositions of Formula (III) and polymer mixtures are presented in Table 1A. The polymers and Formula (III) were weighed and mixed thoroughly using a mortar and pestle, and the resulting blends were tested by Differential Scanning Calorimetry (DSC).

"Heat-cool-heat cycle." In an exemplary screening procedure, the sample of hand mixed drug and polymer in a range of 20 to 40 wt % Formula (III) can be heated to 180°C at 5°C/minute above the melting point of the drug (Tₘ), kept isothermal for 1 minute (heat cycle), cooled to 0°C using a scan rate of 10°C/minute, kept isothermal for 1 minute (cool cycle), and finally re-heated to 180°C at a scan rate 10°C/minute (heat cycle). This DSC procedure (i.e., "heat-cool-heat cycle") was used for all samples disclosed herein unless otherwise noted. The results of DSC testing of these samples using the heat-cool-heat cycle, and the DSC showed sharp peaks at about 166°C during the first heat cycle, representing the melting point of Formula (III). There was a small transition (inflection point) during the second heating cycle which represented the glass transition temperature of the mixture. The results of the Tg from the second heat cycle are shown in Table 1A.

Without intending to be bound by theory, it is believed that the ratio of Tₘ(Drug)/T_{g}(dispersion) (based on Kelvin), which is related to the driving force for crystallization, should be less than or about equal to 1.42 to ensure the physical stability of the amorphous API in the resulting dispersion. A single Tg was found in all pairs indicating good miscibility. The Tg of the Formula (III) polymer binary mixtures varied between 45°C to 86°C. The two polymers with lowest binary Tg (Formulations B and C) were eliminated because their Tg's were too close to the desired storage temperature (i.e., 25°C), and the Tm/Tg ratios are the highest among all the formulations studied, which may lead to recrystallization of Formula (III).

**Table 1A**

| Formulations | 1A | 1B | 1C | 1D | 1E |
|---|---|---|---|---|---|
| Formula (III) | 30% | 30% | 30% | 30% | 30% |
| Copovidone | 70% | -- | -- | -- | -- |
| HPMC E3 | -- | 70% | -- | -- | -- |
| Affinisol HPMC 6cP | -- | -- | 70% | -- | -- |
| L100-55 | -- | -- | -- | 70% | -- |
| HPMCAS-LF | -- | -- | -- | -- | 70% |
| Total | 100% | 100% | 100% | 100% | 100% |
| T_{g}(°C) | 86 | 64 | 46 | 81 | 69 |
| Tm(Drug)/Tg(dispersion) (in Kelvin) | 1.23 | 1.31 | 1.39 | 1.25 | 1.30 |

Effect of drug loading on Tg in presence of polymer. In order to understand the effect of drug loading on the formulation, different levels of drug loading (from 20% to 40%) in the formulations were evaluated using Copovidone and L100-55. The formulation compositions are shown in Table 1B. Individual Tg values for each composition are presented in Table 1C.

**Table 1B**

| Components (wt%) | #2A | #2B | #2C | #2D |
|---|---|---|---|---|
| Formula (III) | 20 | 40 | 20 | 40 |
| Copovidone | 80 | 60 | 0 | 0 |
| L100-55 | 0 | 0 | 80 | 60 |
| Total | 100 | 100 | 100 | 100 |

**Table 1C**

| Drug Loading (% w/w) | Tg (°C) | |
|---|---|---|
| | Stabilizing Polymer | |
| | Copovidone | L100-55 |
| 20 | 91.5 | 85.6 |
| 30 | 86.0 | 81.3 |
| 40 | 78.1 | 78.3 |

As can be seen from Table 1C, the higher the percentage of Formula (III) in the mixture, the lower the Tg of the mixture. Therefore, without wishing to be bound by any theory of lower driving force to crystallize at lower T_{g} and fixed Tₘ, the drug loading for Formula (III) would generally be in an amount of 40 wt% or less of the amorphous solid dispersion.

### Example 2: Pharmaceutical Compositions of Embodiment H

One of the challenges for hot melt extrusion (HME) is that the high processing temperature can lead to thermal degradation of drug. The melting temperature of Formula (III) is around 170°C and the decomposition temperature is well beyond 200°C. This makes Formula (III) a candidate for HME. The selection of polymer to rapidly disperse the molten drug near its melting temperature and preserve its amorphous state upon cooling is critical to the success of HME.

A twin screw hot melt extrusion process was used to manufacture the solid dispersion of Formula (III) using different polymers and drug loadings. Based on the initial screening results, seven formulation blends with 15 to 30% drug loading (DL) using Copovidone (KOLLIDON® VA64), hypromellose acetate succinate (AQUACOAT® AS-LF), and a methacrylic acid-ethyl acrylate copolymer (EUDRAGIT® L100-55) were prepared for the melt extrusion process. All batches were prepared using a 16 mm twin screw extruder at 100 gram scale with product temperature 160-175°C. One percent colloidal silicon dioxide (fumed silica) was added to all of the blends as a flow enhancer. These blends were processed using a Leistritz 16 mm twin screw extruder, and the resulting extrudates were ground using a coffee grinder and screened through a US standard 60 mesh. X-ray powder diffraction (XRPD) was used to confirm that the extrudates were amorphous. The formulations are shown in Table 2A. The process conditions and parameters are presented in Table 2B.

**Table 2A**

| Components | 001A | 002A | 003A | 004A | 005A | 006A | 007A |
|---|---|---|---|---|---|---|---|
| Formula (III) | 15 | 20 | 30 | 20 | 30 | 20 | 30 |
| Copovidone | 84 | 79 | 69 | -- | -- | -- | -- |
| L100-55 | -- | -- | -- | 79 | 69 | -- | -- |
| HPMCAS | -- | -- | -- | -- | -- | 79 | 69 |
| Fumed Silica | 1 | 1 | 1 | 1 | 1 | 1 | 1 |
| Total | 100 | 100 | 100 | 100 | 100 | 100 | 100 |
| Outcome | Good stability up to 2 weeks | | | | | Some degradation | |
| | All amorphous per XRPD | | | | | | |

**Table 2B**

| Process step | Process Parameter | Process Parameter Setting | | | | | | |
|---|---|---|---|---|---|---|---|---|
| Extrusion (Leistritz 16 mm) | Diameter Screws (mm) | 16 | | | | | | |
| | Screw Speed (rpm) | 150 | | | | | | |
| | Feed Rate (g/min) | Manual Feeding (∼ 5) | | | | | | |
| | Process Temperature (4 zones) (°C) | 1A | 2A | 3A | 4A | 5A | 6A | 7A |
| | | 93 | 98 | 93 | 93 | 92 | 96 | 97 |
| | | 175 | 175 | 175 | 170 | 170 | 170 | 170 |
| | | 175 | 175 | 175 | 170 | 170 | 175 | 170 |
| | | 160 | 160 | 160 | 155 | 155 | 160 | 160 |
| Product pressure (psi) | | 125 | 119 | 109 | 500 | 503 | 242 | 209 |
| Product temperature (°C) | | 171 | 172 | 171 | 165 | 163 | 170 | 173 |
| Milling (Coffee Grinder) | Screen | US Mesh # 60 (250 µm) | | | | | | |

Photomicrographs of the resultant extrudates showed that all extrudates had a clear glassy appearance, indicating miscibility of Formula (III) in the stabilizing polymer after the HME process. The extrudates were milled using a coffee grinder and sized before physical characterization to confirm the amorphous nature of Formula (III) in the solid dispersions of the stabilizing polymers. Once the amorphous form was confirmed, the samples were submitted for chemical characterization to confirm the chemical stability of Formula (III) in the solid dispersion.

Physical State characterization by XRPD. The crystallography of Formula (III) solid dispersions was performed using an X-Ray Powder Diffraction (XRPD) technique, Bruker D8 Advance, Scan range 2.0 - 40.0° 2θ at 0.3s/Step, 30 rpm sample rotation. XRPD plots of all initial samples for Copovidone, L100-55 and HPMC-AS LF based dispersions did not show any structural features of crystallinity of Formula (III) in the melt extrudate, thus confirming the conversion of crystalline API into an amorphous state in the melt-extruded dispersions.

Stability Studies. Amorphous solid dispersions can have a proclivity to chemically degrade faster than their crystalline solids and can also crystallize (physical stability) during storage which can cause slower dissolution. The milled extrudate was monitored for physical solid state and chemical stability for up to 3 months of storage at the accelerated storage condition of 40°C and 75% relative humidity.

Chemical stability. One challenge of HME process is to prevent chemical degradation as a result of high temperature and residence time. The impurity profile of the formulations was compared to the crystalline Formula (III) used in the HME extrudate (Table 2C). Based on chemical characterization results, Copovidone and L100-55 showed acceptable results (total impurity less than 1.5%) at the initial time.

**Table 2C**

| Formulation | API | Copovidone | | | L100-55 | | HPMCAS-LF | |
|---|---|---|---|---|---|---|---|---|
| | | 001A | 002A | 003A | 004A | 005A | 006A | 007A |
| Assay, % LC | NA | 94.4 | 92.5 | 91.7 | 97.9 | 99.1 | 98.9 | 96.0 |
| Total Specified Impurities % | 0.46 | 0.60 | 0.51 | 0.52 | 0.71 | 0.62 | 0.52 | 0.89 |
| Total Impurities % | 0.82 | 1.04 | 0.95 | 0.91 | 1.13 | 0.99 | 1.68 | 1.99 |

Purity data were collected for all HME formulations stored for up to three months at 40°C/75% RH. The degradation for the formulations is shown in Table 2D. The HPMCAS formulation had a higher starting impurity level due to one individual process induced degradation product at RRT=0.98. The three HME formulations were within the specified total impurity (<3.0%) after 3 months of storage at 40°C/75%.

**Table 2D - Stability by HPLC, % Total Impurity**

| Polymer | Lot # | loading | T=0 | 1wk | 2wks | 4wks | 13wks |
|---|---|---|---|---|---|---|---|
| No Polymer | N/A | N/A | 0.82 | | | | |
| Copovidone | 1A | 15% | 1.04 | 0.89 | 1.06 | 0.85 | 0.80 |
| Copovidone | 2A | 20% | 0.95 | 0.75 | 0.99 | 0.79 | 0.73 |
| Copovidone | 3A | 30% | 0.91 | 0.81 | 0.98 | 0.77 | 0.76 |
| L100-55 | 4A | 20% | 1.13 | 1.21 | 1.33 | 1.44 | 1.17 |
| L100-55 | 5A | 30% | 0.99 | 0.87 | 1.07 | 1.39 | 1.18 |
| HPMCAS | 6A | 20% | 1.80 | 1.81 | 1.98 | 2.18 | 2.03 |
| HPMCAS | 7A | 30% | 1.33 | 1.33 | 1.49 | 1.68 | 1.51 |

Solid state stability. All HME formulations, with Copovidone, L100-55, or HPMCAS-LF at different drug loadings, maintained amorphous state for up to 3 months at 40°C/75%RH as confirmed by XRPD.

Thermal degradation was closely monitored due to the high processing temperature. Various degrees of process related chemical degradation were observed when the milled extrudate samples were characterizations by HPLC. The Copovidone based formulation showed negligible process related degradation while the HPMCAS formulation degraded the most during the formulation process. Selected major impurities in the extrudate that could be attributed to the processing condition were summarized based on the relative retention time (RRT) and shown in Table 2E.

**Table 2E**

| Polymer | Sample | API loading | Process Temperature (°C) | Process Related Degradation Product |
|---|---|---|---|---|
| Copovidone | 01A | 15% | 171 | all < 0.1% |
| | 02A | 20% | 172 | all < 0.1% |
| | 03A | 30% | 171 | all < 0.1% |
| L100-55 | 04A | 20% | 165 | RRT 0.73: 0.22%; |
| | 05A | 30% | 163 | RRT 0.73: 0.07% |
| HPMCAS | 06A | 20% | 170 | RRT 0.98: 0.65; RRT 1.02: 0.15; |
| | 07A | 30% | 173 | RRT 0.98: 0.40 |

In general, impurities decreased with increasing drug loading. Based on chemical characterization results, Copovidone and L100-55 showed acceptable results (total impurity less than 1.5%) at the initial time. However, the HPMCAS based solid dispersions showed higher unspecified impurities.

Stability Studies. All milled extrudates were stored in closed 75 mL HDPE bottles and monitored at accelerated conditions (40°C/75%RH). They were tested for chemical stability up to 6 months. The physical state of melt-extruded amorphous solid dispersions was reported by XRPD. The samples of ASDs used for the XRPD plots from Copovidone, L100-55, and HPMCAS were stored at accelerated ICH conditions for 3 months.

Chemical Characterization of stability samples. In order to select the best formulation, the assay and impurity of all melt-extruded dispersions were monitored upon storage at 40°C/75%RH in closed conditions up to 6 months. Additionally, assay and impurity of solid dispersions containing L100-55 or HPMCAS were also tested after 2 weeks exposure to 40°C/75%RH open conditions, that is, the extreme accelerated condition of an open container allowing direct exposure to the high relative humidity. The results are presented in Tables 2F-2L. Based on the XRPD results and chemical characterization data, the formulations with Copovidone and L100-55 demonstrated superior chemical stability compared to HPMCAS based melt extruded dispersions.

**Table 2F: Impurities of Formulation Containing 15 wt% Formula (III) with Copovidone**

| | API | Initial | 4 Week | 3 Months | 6 Months |
|---|---|---|---|---|---|
| Total Specified Impurities % | 0.46 | 0.60 | 0.64 | 0.57 | 0.60 |
| Total Impurities % | 0.82 | 1.04 | 0.85 | 0.80 | 1.03 |

**Table 2G: Impurities of Formulation Containing 20 wt% Formula (III) with Copovidone**

| | API | Initial | 4 Week | 3 Months | 6 Months |
|---|---|---|---|---|---|
| Total Specified Impurities % | 0.46 | 0.51 | 0.56 | 0.55 | 0.53 |
| Total Impurities % | 0.82 | 0.95 | 0.79 | 0.73 | 0.89 |

**Table 2H: Impurities of Formulation Containing 30 wt% Formula (III) with Copovidone**

| | API | Initial | 4 Week | 3 Months | 6 Months |
|---|---|---|---|---|---|
| Total Specified Impurities % | 0.46 | 0.52 | 0.59 | 0.56 | 0.60 |
| Total Impurities % | 0.82 | 0.91 | 0.77 | 0.76 | 1.00 |

**Table 2I: Impurities of Formulation Containing 20 wt% Formula (III) with L100-55**

| | API | Initial | 4 Week | 3 Months | 6 Months |
|---|---|---|---|---|---|
| Total Specified Impurities % | 0.46 | 0.71 | 1.00 | 0.96 | 0.83 |
| Total Impurities % | 0.82 | 1.13 | 1.44 | 1.17 | 1.40 |

**Table 2J: Impurities of Formulation Containing 30 wt% Formula (III) with L100-55**

| | API | Initial | 4 Week | 3 Months | 6 Months |
|---|---|---|---|---|---|
| Total Specified Impurities % | 0.46 | 0.62 | 0.81 | 0.80 | 0.67 |
| Total Impurities % | 0.82 | 0.99 | 1.39 | 0.93 | 1.18 |

**Table 2K: Impurities of Formulation Containing 20 wt% Formula (III) with HPMCAS**

| | API | Initial | 4 Week | 3 Months | 6 Months |
|---|---|---|---|---|---|
| Total Specified Impurities % | 0.462 | 0.52 | 0.88 | 0.72 | 1.87 |
| Total Impurities % | 0.818 | 1.68 | 2.18 | 2.03 | 4.45 |

**Table 2L: Impurities of Formulation Containing 30 wt% Formula (III) with HPMCAS-LF**

| | API | Initial | 4 Week | 3 Months | 6 Months |
|---|---|---|---|---|---|
| Total Specified Impurities % | 0.46 | 0.50 | 0.71 | 0.70 | 0.87 |
| Total Impurities % | 0.82 | 1.33 | 1.68 | 1.51 | 2.22 |

Solubility Studies. In order to develop a meaningful dissolution or kinetic solubility method for the Formula (III) amorphous solid dispersions; the solubility of crystalline API and solid dispersions were evaluated using media of different molar concentrations at pH 6.8 phosphate buffer, 10 mM and 50 mM, alone and in combination with two different concentrations of sodium lauryl sulphate (SLS), 0.5% and 1.0%. Amorphous solid dispersions with 30% drug loading of Formula III with three different stabilizing polymers were evaluated. In particular, excess sample (either Formula (III) milled extrudate or Formula (III) as a control) was added into the bottle containing 120 mL of each media to ensure saturation. The bottle was rotated at 50 rpm up to 240 minutes then the solubility was measured. The results are reported in Table 2M. In the solubility study in which the extrudates and crystalline API were equilibrated overnight in phosphate buffer of different buffer strengths, all three HME based amorphous solid dispersion formulations at 30% loading showed at least about 4-5 times higher solubility (Table 2M) compared to crystalline Formula (III).

**Table 2M**

| Media | | Solubility (ug/mL) | | | |
|---|---|---|---|---|---|
| | | API Only | 3A | 5A | 7A |
| | | No Polymer | Copovidone | L100-55 | HPMCAS |
| 10 mM Phosphate buffer, pH 6.8 | No Surfactant | 23 | 96 | 84 | 108 |
| | 0.5 % SLS | 419 | 275 | 144 | 99 |
| | 1.0 % SLS | 593 | 554 | 138 | Precipitated |
| 50 mM Phosphate buffer, pH 6.8 | No Surfactant | 19 | 84 | 89 | 105 |
| | 0.5 % SLS | 386 | 233 | Precipitated | Precipitated |
| | 1.0 % SLS | 627 | Precipitated | Precipitated | Precipitated |

As shown in Table 2M, the molar concentration of pH 6.8 Phosphate buffer had an insignificant effect on the solubility of Formula (III) and the amorphous solid dispersions. At the same time, SLS diminished the discrimination between the crystalline and amorphous forms. Thus it is advisable to avoid SLS in the dissolution media for kinetic solubility studies. As a result of this evaluation, 50 mM Phosphate buffer, pH 6.8 without surfactant, was selected as the dissolution medium to determine the kinetic solubility of Formula (III) for amorphous solid dispersions (ASDs).

The kinetic solubility results confirmed that all amorphous solid dispersions showed improved solubility of Formula (III) over the 4 hour period compared to crystalline Formula (III). Moreover, L100-55 based ASD formulation 5A had superior kinetic solubility over 4 hours. A higher degree of super-saturation and extended length of super-saturation show that larger amounts of Formula (III) may dissolve in the gastrointestinal tract and stay dissolved in the small intestine while being absorbed into the blood stream.

### Example 3: Pharmaceutical Compositions of Embodiment H

The study was an evaluation of the pharmacokinetics of Formula (III) following PO (oral) administration at 30 mg/kg in male Sprague-Dawley rats. Formulations were tested with four rats in each dose group. Dose level of Formula (III) was chosen to be 30 mg/mL, well below the dose level where toxicity is observed, while still high enough to be discriminating for comparing potential dissolution-limited oral absorption. The dose volume was 5 mL/kg and the concentration was 6 mg/mL (with the exception of Group 3, in which the dose volume was 3 mL/kg and concentration 10 mg/mL). Plasma samples were collected pre-dose, 15, 30 min, 1, 2, 4, 6, 8, and 24 hours. The formulations shown in Table 3A were tested. Corn oil was used to suspend the HME formulation comprises of Copovidone because Copovidone based milled extrudate dissolves rapidly in aqueous formulation vehicle (2% HPC solution with 0.1% Tween 80) and forms crystalline solid soon after dissolution.

**Table 3A**

| Formulation | Composition |
|---|---|
| HME L100-55 | Amorphous Solid Dispersion 20 wt% Formula (III) + 80 wt% L100-55 - Suspension In 2% HPC + 0.1% Tween 80 In water |
| HME Copovidone | Amorphous Solid Dispersion 20 wt% Formula (III) + 80 wt% Copovidone - Suspension In corn oil |

The average plasma profiles and PK parameters for the groups are compared in Table 3B. The highest exposure (AUC) was obtained by the L100-55 based HME extrudate. Copovidone based HME show lower exposure. The slightly extended release profile and overall lower absorption of the Copovidone based HME in corn oil (Group 3) could be partially or entirely caused by suspending the formulation in corn oil.

**Table 3B**

| PK Parameters | T_{1/2} (hr) | Tₘₐₓ (hr) | Cₘₐₓ (ng/mL) | AUCₗₐₛₜ (hr*ng/mL) |
|---|---|---|---|---|
| Formulation | Mean (SD) | Mean (SD) | Mean (SD) | Mean (SD) |
| HME L100-55 | 2.1 (1.1) | 0.9 (0.25) | 4095 (2557) | 7241 (1989) |
| HME Copovidone | 2.2 (1.1) | 0.8 (0.8) | 916 (554) | 2950 (670) |

### Example 4: Pharmaceutical Compositions of Embodiment H

Based on the favorable stability, bioavailability, and ease of manufacture, the HME formulations were further explored. HME: hot melt extrudate milled, mixed with other binder/filler/disintegrant, with or without dry granulation, and compressed into tablets. The formulation is shown in Table 4A.

**Table 4A**

| Ingredient | mg/tablet |
|---|---|
| Formula (III) | 25.0 |
| L100-55 | 57.5 |
| Colloidal Silicon Dioxide | 2.8 |
| Croscarmellose Sodium | 10.0 |
| Microcrystalline Cellulose | 52.7 |
| Sodium Stearyl Fumarate | 2.0 |

Further PK study to confirm solid form bioavailability was conducted using male cynomolgus monkeys. This was a crossover study under fasted condition with one week washout period. The dose level was 25 mg/capsule or tablet using a crossover design. The mean PK parameters (including ± standard deviations) of the HME (L100-55) are shown in Table 4B.

**Table 4B**

| | AUCₗₐₛₜ (ng•hg/mL) | Cₘₐₓ (ng/mL) | Tₘₐₓ (hr) | T_{1/2} (hr) |
|---|---|---|---|---|
| Formulation | mean ± SD | mean ± SD | mean ± SD | mean ± SD |
| HME L100-55 | 36600 ± 10300 | 8830 ± 3200 | 3.0 ± 1.2 | 4.29 ± 2.05 |

### Example 5: Pharmaceutical Compositions of Embodiment H

The extrudate formulations shown in Table 5A were prepared following the process parameters shown in Table 5B. For each formulation, the components were physically mixed in a high shear mixer (Collette 10L), then passed through a US standard #30 screen, and then physically mixed with a high shear mixer (Collette 10L) while optionally adding triethylcitrate. Thereafter, the mixture was subjected to hot melt extrusion by the process parameters shown in Table 5B, then milled, and extruded to form milled extrudates.

**Table 5A**

| Compound | 021A | 022A | 023A |
|---|---|---|---|
| Formula (III) | 30.0 wt% | 30.0 wt% | 30.0 wt% |
| L100-55 | 66.5 wt% | 64.0 wt% | 69.0 wt% |
| Triethylcitrate | 2.5 wt% | 5.0 wt% | 0 wt% |
| Colloidal Silicon Dioxide | 1.0 wt% | 1.0 wt% | 1.0 wt% |

**Table 5B**

| Process step | Process Parameter | Process Parameter Setting | | |
|---|---|---|---|---|
| Extrusion (Leistritz 16 mm) | Diameter Screws (mm) | 16 | | |
| | Screw Speed (rpm) | 150 | | |
| | Feed Rate (g/min) | Manual Feeding (∼ 5) | | |
| | Process Temperature (4 zones) (°C) | 021A | 022A | 023A |
| | | 103 | 106 | 100 |
| | | 150 | 144 | 150 |
| | | 154 | 150 | 150 |
| | | 140 | 139 | 135 |
| Product pressure (psi) | | 135 | 133 | 220 |
| Product temperature (°C) | | 150 | 145 | 150 |
| Milling (Coffee Grinder) | Screen | US # 60 (250 µm) | | |

The milled extrudates were evaluated by XRPD and did not have crystalline peaks and exhibited amorphous halos, indicating that the absence of crystallinity of the compound of Formula (III) in any of the extrudates. The % label claim (LC) and total impurities are shown in Table 5C.

**Table 5C**

| | API | 021A | 022A | 023A |
|---|---|---|---|---|
| % LC | -- | 99.7 | 101.1 | 102.5 |
| Total Impurities % | 0.80 | 0.95 | 0.94 | 0.64 |

The extrudates were stored for up to four weeks at 40°C and 75% relative humidity, and monitored for chemical characterization. The chemical stability data is shown in Tables 5D-5F. The extrudate (023A) without the triethylcitrate had less total impurities when compared to the extrudates with triethylcitrate (021A, 022A).

**Table 5D - Extrudate 021A**

| | API | Initial | 2 Weeks Open | 2 Weeks Closed | 4 Weeks Closed |
|---|---|---|---|---|---|
| % LC | - | 99.7 | 95.5 | 98.1 | 101.1 |
| Total Impurities % | 0.80 | 0.95 | 1.32 | 1.23 | 1.49 |

**Table 5E - Extrudate 022A**

| | API | Initial | 2 Weeks Open | 2 Weeks Closed | 4 Weeks Closed |
|---|---|---|---|---|---|
| % LC | -- | 101.1 | 96.9 | 99.8 | 102.3 |
| Total Impurities % | 0.80 | 0.94 | 1.50 | 1.35 | 1.48 |

**Table 5F - Extrudate 023A**

| | API | Initial | 2 Weeks Open | 2 Weeks Closed | 4 Weeks Closed |
|---|---|---|---|---|---|
| % LC | -- | 102.5 | 97.9 | 101.7 | 103.9 |
| Total Impurities % | 0.80 | 0.64 | 1.28 | 1.26 | 1.49 |

### Example 6: Pharmaceutical Compositions of Embodiment H

An extrudate was used to form a tablet of Formulation 023B with the components shown in Table 6A. The tablets were prepared by mixing the extrudate with the extragranular components, and then by compressing with a single stage compression unit (Carver press), with a punch size of a concave round tablet (0.3150" diameter). The measured tablet hardness was 8 kP, and the tablets had a disintegration time of about 15 seconds in 0.1 N HCl. The tablets were further studied by HPLC-UV and placed on stability under accelerated storage conditions of 40°C, 75% relative humidity in a 45cc HDPE bottle, with the results shown in Table 6B.

**Table 6A**

| Extrudate Component | |
|---|---|
| Formula (III) | 25.0 mg |
| L100-55 | 57.5 mg |
| Colloidal silicon dioxide | 0.8 mg |

| Extragranular Component | |
|---|---|
| Croscarmellose sodium (Ac-Di-Sol) | 10.0 mg |
| Microcrystalline cellulose (MCC102) | 52.7 mg |
| Colloidal silicon dioxide | 2.0 mg |
| Sodium stearyl fumarate | 2.0 mg |

**Table 6B**

| | API | Extrudate | Tablet Initial | 2 Weeks Open | 2 Weeks Closed | 4 Weeks Closed |
|---|---|---|---|---|---|---|
| % LC | -- | 102.5 | 102.0 | 104.8 | 105.7 | 103.7 |
| Total Impurities % | 0.80 | 0.62 | 1.15 | 1.05 | 1.09 | 1.07 |

The tablets were evaluated for dissolution over the stability conditions. Because L100-55 does not dissolve in acidic conditions, dissolution for the tablets was performed in 900 mL of pH 6.8 buffer in a USP-II apparatus (paddle) at 50 rpm.

### Example 7: Pharmaceutical Compositions of Embodiment H

The extrudate shown in Table 7A, was used for a large scale-up batch following the process conditions shown in Table 7B. The extrudate was shown to be amorphous by XRPD, and exhibited the particle size distribution by sieve analysis shown in FIG. 1. Using a mixture of the extrudate and the extragranular components shown in Table 7A, tablets of Formulation 025C were prepared with a single stage compression unit (F-press), with a flat round punch (8.00 mm diameter). The measured tablet hardness was 10 kP, and the tablets had a disintegration time of about 25 seconds in 0.1 N HCl. Properties of the tablets, including weight, thickness, hardness, and disintegration time are shown in Table 7C, and having a final blend bulk density of 0.50 g/mL and a final blend tapped density of 0.62 g/mL. The compressed tablets exhibited an initial assay, % LC of 108.4 and total impurities of 1.15%.

**Table 7A**

| Extrudate Component | |
|---|---|
| Formula (III) | 25.0 mg |
| L100-55 | 57.5 mg |
| Colloidal silicon dioxide | 0.8 mg |

| Extragranular Component | |
|---|---|
| Croscarmellose sodium (Ac-Di-Sol) | 10.0 mg |
| Microcrystalline cellulose (MCC102) | 52.7 mg |
| Colloidal silicon dioxide | 2.0 mg |
| Sodium stearyl fumarate | 2.0 mg |

**Table 7B**

| Process step | Process Parameter | Process Parameter Setting |
|---|---|---|
| Extrusion (Leistritz 16 mm) | Diameter Screws (mm) | 16 |
| | Screw Speed (rpm) | 150 |
| | Feed Rate (g/min) | 10 |
| | Process Temperature (4 zones) (°C) | 105 |
| | | 150 |
| | | 150 |
| | | 140 |
| Product pressure (psi) | | 225 |
| Product temperature (°C) | | 150 |
| Milling (Fitzpatrick Fitzmill Model L1A) Screen | | Medim speed 3500 rpm US #1522-0024 (600 µm) |

**Table 7C**

| Sample | Weight (mg) | Thickness (mm) | Hardness (kP) | Disintegration Time |
|---|---|---|---|---|
| 1 | 152 | 2.78 | 9.2 | 20 seconds |
| 2 | 155 | 2.77 | 10.2 | 25 seconds |
| 3 | 155 | 2.78 | 10.6 | 23 seconds |
| 4 | 156 | 2.75 | 9.8 | |
| 5 | 154 | 2.78 | 10.2 | |
| 6 | 156 | 2.77 | 10 | |
| 7 | 155 | 2.76 | 9.3 | |
| 8 | 154 | 2.77 | 9.4 | |
| 9 | 152 | 2.76 | 9.9 | |
| 10 | 154 | 2.78 | 9.3 | |
| Average | 154.3 | 2.77 | 9.8 | |
| St. Dev. | 1.42 | 0.01 | 0.47 | |
| RSD | 0.9 | 0.4 | 4.8 | |

To evaluate the dissolution rate of the tablets from the extrudate, a two-stage dissolution method was performed using 900 mL of 0.1 N HCl in a USP-II apparatus (paddle) at 50 rpm for 30 minutes, followed by immersion in 900 mL of pH 6.8 phosphate buffer, USP-II apparatus (paddle) at 50 rpm. This two-stage dissolution method was performed because L100-55 does not dissolve below pH 5.5. The two-stage dissolution is shown in FIG. 2. The higher paddle speed (75 rpm) was incorporated to understand the impact of paddle speed on rate and recovery. As can bee seen from FIG. 2, the tablet had a slower rate and less complete dissolution at 50 rpm paddle speed. Linear polymeric erosional behavior was not observed. Further reduction of milled extrudate particle size increased the dissolution rate and eliminated the effect of dissolution paddle speed, and the tablet from the improved process achieved complete dissolution at 50 rpm.

### Example 8: Pharmaceutical Compositions of Embodiment H

An extrudate of Formulation 031A was prepared with the components shown in Table 8A using the process conditions shown in Table 8B. Milled extrudate particles comprising the components shown in Table 8A and having a particle size of 250 microns or less (US standard #60 mesh screen) were then used for blending with extragranular components to form tablets of Formulation 031C as shown in Table 8C. In particular, a mixture of the extrudate and the extragranular components were prepared with a single stage compression unit (F-press), with a concave round punch (9/32" diameter). The measured tablet hardness was 10 kP, and the tablets had a disintegration time of about 20 seconds in 0.1 N HCl. Properties of the tablets, including weight, thickness, hardness, and disintegration time are shown in Table 8D, and having a final blend bulk density of 0.51 g/mL and a final blend tapped density of 0.63 g/mL. The chemical characteristics of the extrudate of Formulation 031A and the tablets of Formulation 031C are shown in Table 8E.

**Table 8A**

| Component | wt % |
|---|---|
| Formula (III) | 30.0 |
| L100-55 | 69.0 |
| Colloidal silicon dioxide | 1.0 |

**Table 8B**

| Process step | Process Parameter | Process Parameter Setting |
|---|---|---|
| Extrusion (Leistritz 16 mm) | Diameter Screws (mm) | 16 |
| | Screw Speed (rpm) | 150 |
| | Feed Rate (g/min) | 10 |
| | Process Temperature (4 zones) (°C) | 101 |
| | | 160 |
| | | 162 |
| | | 149 |
| Product pressure (psi) | | 223 |
| Product temperature (°C) | | 148 |
| Milling (Fitzpatrick Fitzmill Model L1A) Screen | | Medim speed 3500 rpm US #1522-0024 (600 µm) |

**Table 8C**

| Extrudate Component | |
|---|---|
| Formula (III) | 25.0 mg |
| L100-55 | 57.5 mg |
| Colloidal silicon dioxide | 0.8 mg |

| Extragranular Component | |
|---|---|
| Croscarmellose sodium (Ac-Di-Sol) | 10.0 mg |
| Microcrystalline cellulose (MCC102) | 52.7 mg |
| Colloidal silicon dioxide | 2.0 mg |
| Sodium stearyl fumarate | 2.0 mg |

**Table 8D**

| Sample | Weight (mg) | Thickness (mm) | Hardness (kP) | Disintegration Time |
|---|---|---|---|---|
| 1 | 151 | 4.05 | 9.5 | 23 seconds |
| 2 | 152 | 4.04 | 10.1 | 24 seconds |
| 3 | 150 | 4.07 | 9.9 | 26 seconds |
| 4 | 151 | 4.03 | 10.2 | |
| 5 | 149 | 4.06 | 10.3 | |
| 6 | 151 | 4.04 | 9.7 | |
| 7 | 152 | 4.06 | 10.1 | |
| 8 | 150 | 4.05 | 10.2 | |
| 9 | 151 | 4.05 | 9.8 | |
| 10 | 148 | 4.06 | 9.9 | |
| Average | 150.5 | 4.05 | 10 | |
| St. Dev. | 1.2 | 0.01 | 0.24 | |
| RSD | 0.8 | 0.2 | 2.4 | |

**Table 8E**

| | API | Extrudate - 031A | Tablet-031 C |
|---|---|---|---|
| Assay, %LC | -- | 101.4 | 103.9 |
| Total Impurities | 0.80 | 1.14 | 1.30 |

Fifty tablets were stored in 75 cc HDPE bottles with and without 1 gram of desiccant and monitored at controlled room temperature (CRT) conditions of 25°C and 60% relative humidity and accelerated conditions (ACC) of 40°C and 75% relative humidity. The results of the assay, %LC, the total impurities, and the dissolution in 0.1N HCl, then 6.8, USP II, 50 rpm are shown in Table 8F (without desiccant), Table 8G (with desiccant) and FIG. 3.

**Table 8F**

| | API | Initial | 1 month CRT | 1 month ACC | 3 months CRT | 3 months ACC |
|---|---|---|---|---|---|---|
| % LC | -- | 103.9 | 104.8 | 106.3 | 105.9 | 105.5 |
| Total Impurities % | 0.80 | 0.95 | 0.88 | 0.90 | 0.85 | 0.88 |

**Table 8G**

| | API | Initial | 1 month CRT | 1 month ACC | 3 months CRT | 3 months ACC |
|---|---|---|---|---|---|---|
| % LC | -- | 103.9 | 106.3 | 104.1 | 105.9 | 106 |
| Total Impurities % | 0.80 | 0.95 | 0.98 | 0.94 | 0.98 | 0.96 |

### Example 9: Pharmaceutical Compositions of Embodiment M

Microprecipitated bulk powder (MBP) was studied for adenosine A2A receptor antagonists, such as the compound of Formula (III), because it creates an amorphous state while avoiding the high processing temperature required for HME. The core of MBP technology is the controlled co-precipitation of dissolved drug and polymer in an anti-solvent. More particularly, this process uses the exceptional solvent power of polar aprotic solvents, such as N,N-dimethylacetamide (DMA), to dissolve the API and the pharmaceutically acceptable polymer. This API and pharmaceutically acceptable polymer are then co-precipitated out of the solution with acidified cold water into an aqueous medium maintained at a desired pH, temperature, and hydrodynamic conditions. The solvent is extracted from the API:polymer co-precipitate by washing at controlled temperature. The co-precipitated material is isolated by filtration, and followed by drying. The dried material is referred to as microprecipitated bulk powder. The amorphous form prepared using this method not only provided excellent in-vitro and in-vivo performance but also showed remarkable stability. Without intending to be bound by any theory, the excellent stability of the amorphous MBP is attributed to the strength of the ionic interactions between the ionic polymer and the charged drug, providing protection to the amorphous form during storage.

Formulation compositions with 20-30% drug loading, and HPMCAS, Eudragit® L100-55 and Eudragit® L100 as pharmaceutically acceptable polymer were evaluated using the MBP process. DMA was used as the polar aprotic solvent for the compound of Formula (III) and the pharmaceutically acceptable polymer. In particular, the pharmaceutically acceptable polymer was slowly added to the solution containing the compound of Formula (III) and mixed until a clear solution was achieved. The weight ratio of the compound of Formula (III) to DMA was about 1:5. A cold (2°-8°C), dilute acid (pH 3.0) was used as the anti-solvent. In particular, the solution containing pharmaceutically acceptable polymer and the compound of Formula (III) was slowly added to a large amount of the anti-solvent, where the pharmaceutically acceptable polymer and the compound of Formula (III) precipitated as a solid amorphous dispersion, and was then filtered. The coprecipitated material was rinsed with cold acid (pH 3) followed by cold water to remove the residual DMA using a vacuum filter. The coprecipitate was dried at 40°C under vacuum for 36 hours before screening through a US standard 60 mesh screen. This dried coprecipitate, also referred to as a solid amorphous dispersion, a solid composition, and a microprecipitated bulk powder (MBP), are shown in Table 9A.

**Table 9A**

| Formulation Components | 8A | 9A | 10A | 11A | 12A | 13A |
|---|---|---|---|---|---|---|
| Formula (III) | 20% | 30% | 20% | 30% | 20% | 30% |
| HPMCAS | 80% | 70% | -- | -- | -- | -- |
| L100-55 | -- | -- | 80% | 70% | -- | -- |
| L100 | -- | -- | -- | -- | 80% | 70% |
| Total | 100% | 100% | 100% | 100% | 100% | 100% |
| Crystallinity Outcome | crystalline | crystalline | crystalline | crystalline | amorphous | crystalline |

The XRPD scans showed that HPMCAS and L100-55 (Formulations 8A, 9A, 10, and 11A) did not provide a stable amorphous form of the compound of Formula (III) in the MBP. XRPD scans of the L100-based MBP indicated the presence of a stable amorphous dispersion for the 20% drug loaded composition at the initial time point.

Based on the results shown in the Table 9A, a homogenizer was introduced to improve the DMA removal during the precipitation step. As a result, 20% drug loaded formulations of the compound of Formula (III) with L100-55, L100 and HPMCAS were evaluated using this improved precipitation step of the MBP process, the components of which are shown in Table 9B.

**Table 9B**

| Formulation Components | 14A | 15A | 16A |
|---|---|---|---|
| Formula (III) | 20% | 20% | 20% |
| HPMCAS | 80% | -- | -- |
| L100-55 | -- | 80% | -- |
| L100 | -- | -- | 80% |
| Total | 100% | 100% | 100% |
| Crystallinity Outcome | crystalline | amorphous | amorphous |

All samples from the revised MBP process were tested for physical state characterization by XRPD. The XRPD plots showed that the Formulations containing L100-55 and L100 provided a stable, amorphous MBP dispersion, but that the formulation containing HPMCAS did not.

Chemical Characterization of samples. The chemical characterization results for the initial samples of L100-55 and L100 are summarized in the Table 9C.

**Table 9C**

| | Formula (III) | Formulation 15A Formula (III) + L100-55 | Formulation 16A Formula (III) + L100 |
|---|---|---|---|
| Total Specified Impurity % | 0.46 | 0.53 | 0.52 |
| Total Impurity % | 0.82 | 0.91 | 1.05 |

Based on the initial chemical characterization results, both L100-55 and L100 appear to have no significant effect on the impurity profiles of the MBP dispersions. Assay values for both formulations are on the lower side of acceptable range. A number of factors, such as residual moisture of the API, residual moisture in the MBP dispersions, and pH variation of the anti-solvent- diluted acid could have impacted the assay value. Such factors would have been accounted for as part of the MBP process optimization.

Stability Studies. All initial samples of the MBP were kept in closed 75 mL HDPE bottles and monitored at 40°C and 75% relative humidity (RH) for up to 3 months. The physical state and chemical stability were reported by XRPD, and assay and impurity. The XRPD of 3 months accelerated temperature stored MBP showed no evidence of crystalline Formula (III), confirming the physical stability of amorphous MBP at accelerated temperature storage conditions. The assay and impurity results from the MBP dispersions, stored at 40°C and 75% relative humidity closed conditions for up to 3 months, and at 40°C and 75% relative humidity open conditions for up to 2 weeks are reported in the Tables 9D and 9E below.

**Table 9D**

| | API | 15A (20% Formula (III) with L100-55) | | | | |
|---|---|---|---|---|---|---|
| | | Initial | 1 week ACC Open | 2 week ACC Open | 1Month ACC Closed | 3Month ACC Closed |
| Total Specified Imp, % | 0.46 | 0.53 | 0.53 | 0.62 | 0.81 | 0.52 |
| Total Imp, % | 0.82 | 0.91 | 1.14 | 1.54 | 1.39 | 0.98 |

**Table 9E**

| | API | 16A (20% Formula (III) with L100) | | | | |
|---|---|---|---|---|---|---|
| | | Initial | 1 week ACC Open | 2 week ACC Open | 1 Month ACC Closed | 3 Month ACC Closed |
| Total Specified Imp, % | 0.46 | 0.52 | 0.78 | 0.64 | 0.92 | 0.61 |
| Total Imp, % | 0.82 | 1.05 | 1.44 | 1.66 | 1.44 | 1.28 |

Both MBP formulations, 20% DL in both L100-55 and L100, met the physical and chemical stability requirements, which is maintaining the amorphous form, and having a total impurity not more than 1.5% when stored at 40°C and 75% relative humidity for up to 3 months.

Formulation 15A (L100-55 with 20% Formula (III) loading) and Formulation 16A (L100 with 20% Formula (III) loading) were compared with crystalline Formula (III) in kinetic solubility testing. Both amorphous MBP Formulations 15A and 16A showed rapid dissolution within 10 minutes in phosphate buffer at pH 6.8; they also reached supersaturation that is an order of magnitude higher than the solubility reached by neat crystalline Formulation (III), and maintained the supersaturation for over 4 hours. When comparing the dissolution of MBP with that of the hot melt extrusion formulations it was found that almost all amorphous formulations reached similar levels of supersaturation.

### Example 10: Pharmaceutical Compositions of Embodiment M

The study was an evaluation of the pharmacokinetics of Formula (III) following PO (oral) administration at 30 mg/kg in male Sprague Dawley rats. Formulations were tested with four rats in each dose group. Dose level of Formula (III) was chosen to be 30 mg/mL, well below the dose level where toxicity is observed, while still high enough to be discriminating for comparing potential dissolution limited oral absorption. The dose volume was 5 mL/kg and the concentration was 6 mg/mL (with the exception of Group 3, in which the dose volume was 3 mL/kg and concentration 10 mg/mL). Plasma samples were collected pre-dose, 15, 30 min, 1, 2, 4, 6, 8, and 24 hours. The formulations shown in Table 10A were tested.

**Table 10A**

| Formulation | Composition |
|---|---|
| MBP L100-55 | MBP Suspension 20 wt% Formula (III) + 80 wt% L100-55 in 2% HPC + 0.1% Tween 80 |
| MBP L100 | MBP Suspension 20 wt% Formula (III) + 80 wt% L100 in 2% HPC + 0.1% Tween 80 |

The average plasma profiles and PK parameters for the groups are compared in Table 10B.

**Table 10B**

| PK Parameters | T_{1/2} (hr) | Tₘₐₓ (hr) | C ₘₐₓ (ng/mL) | AUCₗₐₛₜ₋₈ (hr*ng/mL) |
|---|---|---|---|---|
| Formulation | Mean (SD) | Mean (SD) | Mean (SD) | Mean (SD) |
| MBP L100-55 | 1.3 (0.4) | 1.1 (0.6) | 2243 (651) | 4509 (522) |
| MBP L100 | 0.9 (0.4) | 0.6 (0.25) | 2660 (819) | 4223 (1085) |

### Example 11: Pharmaceutical Compositions of Embodiment N

Wet milling feasibility studies were conducted in Dyno Mill Multilab 0.6L. The suspension had 30% Formula (III), 2.4% poloxamer 407 and < 0.3% simethicone to prevent foaming. Zirconium oxide beads at 0.5 mm diameter were used as a milling medium. High viscosity developed during milling, and additional poloxamer was not effective in slowing the viscosity buildup. Entrapped air and foaming made further processing problematic, and this occurrence was not inhibited by the addition of simethicone emulsion. A particle size distribution with a D50 of about 1 micron was achieved with the final suspension which was described as a thick mousse. Under the microscope the particles showed a tendency to agglomerate. It was concluded that poloxamer was not effective in stabilizing the particles as demonstrated by the viscosity increase and particle agglomeration.

In high energy nano-milling it is critical to identify the right stabilizers to protect the freshly created surface, to stabilize the milled the particles, and to maintain the particle size distribution with time. The stabilizer is commonly chosen from a water-soluble non-crosslinked polymer, ionic surfactant, nonionic surfactant, or a combination of two or more thereof. Anti-foaming agents may be added if needed.

The feasibility study started with low energy media milling using beads, and was carried out with 10% of Formula (III) in an aqueous suspension in the presence of stabilizers. Initial screening of nine formulations was conducted at the 5 mL scale using 0.5 mm ZrO₂ based YTZ milling media. The formulations used during screening had various stabilizer compositions: 0.1 to 2% polymer or surfactant, alone or in combination of the polymer and surfactant, as shown in Table 11A. The stabilizers included hydroxypropyl cellulose (HPC) and hydroxypropyl methylcellulose (HPMC), polyvinylpyrrolidone, a copolymer of polyvinylpyrrolidone and vinyl acetate (Copovidone), a nonionic surfactant (i.e., polyoxyethylene-polyoxypropylene copolymer (poloxamer 407)), hydrophilic nonionic surfactants (i.e., Tween 80, tocopherol polyethylene glycol succinate (TPGS)), and an anionic surfactant (i.e., dioctyl sulfosuccinate (DOSS))

Each formulation was prepared by combining the excipients listed in Table 11A and Table 11B with purified water, USP in a 20 mL vial. Once the excipients were fully dissolved, Formula (III) was added at a concentration of 10 wt% and about 40 g of 0.5 mm YTZ media was added to each vial. A total of five grams of suspension was prepared for each formulation for a total of 9 vials.

**Table 11A**

| Component (wt%) | B | D | E | F |
|---|---|---|---|---|
| Formula (III) | 10 | 10 | 10 | 10 |
| HPC-SL | 2 | -- | -- | -- |
| PVP | -- | 2 | -- | -- |
| Copovidone | -- | -- | 2 | -- |
| Poloxamer 407 | -- | -- | -- | 2 |
| DOSS | 0.1 | 0.1 | 0.1 | 0.1 |
| Water | 87.9 | 87.9 | 87.9 | 87.9 |
| Total | 100% | 100% | 100% | 100% |

**Table 11B**

| Component (wt%) | A | C | G | H | I |
|---|---|---|---|---|---|
| Formula (III) | 10 | 10 | 10 | 10 | 10 |
| HPC-SL | 2 | -- | -- | -- | -- |
| HPMC-603 | -- | 2 | -- | -- | -- |
| Poloxamer 407 | -- | -- | 2 | -- | -- |
| DOSS | -- | 0.1 | -- | -- | -- |
| Tween 80 | -- | -- | -- | 1 | -- |
| TPGS | -- | -- | -- | -- | 1 |
| Water | 88.0 | 87.9 | 88.0 | 89.0 | 89.0 |
| Total | 100% | 100% | 100% | 100% | 100% |

Each vial was placed on a roller mill until the mean particle size was less than 250 nm or aggregation was noted. Particle size measurements were obtained using water as dispersing media in the fraction cell of the laser diffraction particle size analyzer and analyzed using a refractive index of 1.60 + 0.01i in water. Each formulation was measured three times and the average mean particle size reported in Table 11C. For optical microscopy, a drop of the suspension was added directly to a glass slide with a cover slip.

**Table 11C**

| Formulation | Mean Particle Size (µm) | Milling Duration (hours) |
|---|---|---|
| A | 2.66 | 8.5 |
| B | 0.14 | 5.5 |
| C | 0.50 | 8.5 |
| D | 0.12 | 8.5 |
| E | 0.12 | 8.5 |
| F | 0.12 | 8.5 |
| G | 10.84 | 8.5 |
| H | 4.92 | 8.5 |
| I | 0.33 | 8.5 |

Formulations A, G, H, and I showed aggregation or insufficient reduction in size after 8.5 hours of milling and Formulation C showed aggregation during the particle size distribution analysis.

Formulation B achieved a mean particle size less than 250 nm after only 5.5 hours of processing. Milling was continued on Formulations D, E and F for 8.5 hours total, at which point they had also achieved the required final mean particle size of less than 250 nm. It was noteworthy that when the polymers or surfactants were used as a single component stabilizer (samples A, G, H, and I), they were less effective than a combination of polymer and the ionic surfactant docusate sodium (DOSS).

Particle Size Stability Test at 25°C and 5°C. Formulations (B, D, E and F) that achieved the D90 of 250 nm after low energy milling were stored for one week at 25°C or 5°C and tested again for particle size distribution. Formulations B and E were stable after one week of storage at both temperature conditions. Formulation F showed aggregation at both temperature conditions after 1 week of storage. Formulation D was stable at room temperature but showed aggregation at 5°C.

Particle Size Stability Test at Room Temperature and 2-8°C. Once the initial roller milling was completed, Formulations B, D, E, and F were extracted from the milling media and the suspensions were then split into two glass vials: one stored at 2-8°C and one stored at room temperature (RT). Particle size and optical microscopy were determined for each formulation at T=1 week. Results are shown in Table 11D.

**Table 11D**

| Formulation | Room Temperature | | | 2-8 °C | | |
|---|---|---|---|---|---|---|
| | Mean (µm) | Median (µm) | D90 (µm) | Mean (µm) | Median (µm) | D90 (µm) |
| B | 0.15 | 1.13 | 0.22 | 0.18 | 0.14 | 0.25 |
| D | 0.14 | 0.12 | 0.18 | 1.12 | 0.12 | 0.29 |
| E | 0.13 | 0.12 | 0.18 | 0.23 | 0.12 | 0.20 |
| F | 1.05 | 0.12 | 0.22 | 0.44 | 0.12 | 0.20 |

The physical stability of the formulations after 1 week informal stability can be summarized as follows. Formulation B was stable at both storage conditions. Formulations D and E were stable when stored at room temperature conditions but exhibited tailing at 2-8 °C, suggesting aggregation. Formulation F showed tailing at both storage conditions.

Assay and Related Substance Testing. Formulations B, D, and E were tested for assay and related substances (RS) to detect the compatibility with the excipients. Results are shown in Table 11E in comparison to the standard (Formula (III)), which contained 0.6% RS. Assay/RS was not determined at T0 for this study. Stable formulations in terms of particle size distribution and assay/related substances were selected for scale up.

**Table 11E**

| Formulation | Storage | Target wt% Formula (III) | Assay % Label Claim (n=3) | % RSD | % Related Substances |
|---|---|---|---|---|---|
| B | 2-8 °C | 10.8% | 100.5 | 0.2 | 0.6 |
| | RT | | 101.9 | 0.4 | 0.7 |
| D | 2-8 °C | 10.3% | 127.1 | 0.9 | 0.9 |
| | RT | | 128.3 | 0.3 | 1.0 |
| E | 2-8 °C | 10.3% | 108.2 | 1.7 | 0.7 |
| | RT | | 110.4 | 0.8 | 0.7 |

The stability of the formulations after 1 week can be summarized as follows. Formulation B yielded accurate assay and no change in related substance profile compared to the standard. Formulation E yielded slightly higher than target assay with no change in related substance profile. Formulation D yielded much higher assay than the target, but within experimental error for assay of a nano-suspension prepared at this scale and concentration. Also, related substances increased. Most significantly, RRT 0.80, an oxidation impurity, increased from 0.2 to 0.3% at both storage conditions (room temperature and 2-8 °C) after milling.

Scale-Up. Given the overall particle size distribution and assay and related substances results, Formulations B and E were selected for scale up after the initial stability study. Preliminary scale-up experiments were performed using a temperature controlled, high energy agitator batch mill using 0.5 mm polymeric milling media. Batch size was increased from 5 g (on roller mill) to 100 g (on high energy batch mill) at 10% w/w Formula (III) concentration.

Each formulation was prepared by weighing Formula (III) directly into a 250 mL milling chamber (100-P) and then the required amount of excipient stock solution and water were added to wet Formula (III). The suspension was gently mixed with spatula, to avoid entrapped air, and finally, about 130 g of 0.5 mm polymeric milling media (85% of chamber volume) was added to the suspension. The suspension was milled using a pegged agitator until the required particle size was attained. Chamber cooling temperature was set at 5°C throughout milling. Samples at different time points were withdrawn and checked for particle size distribution by laser diffraction and appearance by optical microscopy. A summary of the preliminary scale up experiments on the high energy batch mill is shown in Table 11F

**Table 11F**

| Formulation | Milling Speed and Duration | % Process Yield | Mean Particle Size |
|---|---|---|---|
| B | 1500 rpm for 25 min and then 2500 rpm from 25 to 38 min | 87% | 136.8 nm |
| E | 2500 rpm for 35 min | 85% | 238.0 nm |

The process may be further optimized as follows: (i) using smaller milling media (0.25 mm instead of 0.5 mm); (ii) increasing the speed of the agitator during milling; (iii) avoiding very low temperature for cooling during milling (as tailing was observed in the samples stored at 2-8°C condition during 1 week stability study on R&D samples produced on roller mill); (iv) decreasing the overhead space by adding more media to the milling chamber (increase from 85% to 90-95% of chamber volume); or (v) a combination of the foregoing.

Formulation B was prepared with higher amounts of Formula (III). Various modifications of Formulation B (with increased concentrations of Formula (III)) were made on the roller mill and high energy batch mill. A summary of the formulation compositions and results of the study are shown in Table 11G and Table 11H.

**Table 11G**

| | B1 | B2 | B3 | B4 | B5 |
|---|---|---|---|---|---|
| Formula (III) (wt%) | 15 | 20 | 30 | 20 | 30 |
| HPC-SL (wt%) | 2 | 2 | 2 | 6 | 6 |
| DOSS (wt%) | 0.1 | 0.1 | 0.1 | 0.3 | 0.3 |
| Water | QS | QS | QS | QS | QS |

**Table 11H**

| Formulation | Equipment | Results |
|---|---|---|
| B1 | Roller mill | Target PSD achieved after milling for about 3 hour |
| B2 | Roller mill | Target PSD achieved after milling for about 3 hours |
| B3 | Roller mill | Achieved PSD was about 300 nm (with tailing and crystals of 7-10 µm observed under microscope) even after milling for 9 h |
| B4 | Roller mill | Target PSD was not achieved even after milling for 25 hours |
| B5 | Roller mill | Target PSD was not achieved even after milling for 25 hours |
| B5 | High energy batch mill | Target PSD achieved after milling for 80 minutes |

Formulation B5 was selected for further preparation in batch sizes 350 g and 2 kg with a Netzsch mill. To prepare the 350 g batch, a 150 mL milling chamber was used with the Netzsch re-circulating mill. The initial slurry of Formula (III) was prepared by weighing 105.0 g of Formula (III) into a 1 liter stainless steel beaker, and the required amount (201.95 g) of HPC-SL and DOSS stock solution (10.40% w/w HPC-SL and 0.52% w/w DOSS in water) was added to Formula (III). The slurry was then mixed gently with a spatula until Formula (III) was uniformly wetted. Care was taken to avoid entrapped air in the suspension. The milling chamber was loaded with about 471 g of 0.5 mm YTZ milling media. A portion of the total water in the formulation was set aside for rinsing the beaker in which the initial slurry was made. The prepared slurry was transferred into the Netzsch mill reservoir by turning the pump on. The agitator (set at 1500 RPM) was then turned on only after seeing the product in the milling chamber outlet tubing. The water that was kept aside for rinsing was used to rinse the beaker into the reservoir. The chamber cooling temperature was set and maintained at 15°C with a recirculating chiller. PSD and optical microscopy were performed on the product at different time points during milling. The required particle size was attained after milling for 1.2 hours. The yield in this process was about 69%.

To prepare a 2 kg batch of Formulation B5, a 300 mL milling chamber with about 950 g of 0.5 mm YTZ milling media (about 85% of chamber volume media load) was used with the Netzsch recirculating mill. The initial slurry of Formula (III) was prepared by mixing the required amount of Formula (III) and stock solution of HPC-SL and DOSS in a 4 liter stainless steel beaker. Care was taken to avoid entrapped air during mixing. A portion of water was set aside for rinsing the beaker after transferring the slurry into the reservoir. The agitator speed was 1500 RPM, and the required particle size was attained after milling for 4.75 hours.

Formulations B, D, and E with the least aggregation tendency were tested for assay and impurity using HPLC. Formulations B and E did not chemically degrade during the milling process. In contrast, Formulation D (PVP+DOSS) showed a slight increase of oxidation impurity at RRT about 0.80 (0.2% to 0.3%) after milling, indicating a potential excipient interaction.

Out of the four formulations passed the initial particle size screening, B and E show good particle size distribution post milling, and are shown to be stable for at least one week with minimum particle size change. Both formulations were scaled up to 100 g in a high energy mill using 0.5 mm polymeric media. The outcome from this milling process is summarized in Table 8 below. Some tailing was observed with prolonged milling time in Lot 5450 of Formulation E, indicating aggregation and formation of large particles. Further process optimization will be required to minimize the tailing if this formulation is selected for further development. Both formulations were entered into stability studies for 3 months at 25 °C or 40°C/75%RH (accelerated stability condition)

**Table 11I**

| Formulation | B (Lot 5449) | E (Lot 5450) |
|---|---|---|
| Composition | 10% Formula (III) | 10% Formula (III) |
| | 2% HPC-SL | 2% PVP-VA |
| | 0.1% sodium docusate | 0.1% sodium docusate |
| Milling condition | 1500 rpm for 25 min, then 2500 rpm from 25 to 38 min | 2500 rpm for 35 min |
| Yield | 87% | 85% |
| Observation | N/A | Tailing after 35min |
| Mean Particle Size | 137 nm | 238 nm |

Dissolution improvement. In previous studies, Formula (III) exposure in vivo was directly correlated with dissolution rate and sustained dissolution. Both Formulation B and E dissolved significantly faster than the un-milled bulk Formula (III). Un-milled Formula (III) fully dissolved in 45 minutes, while Formulations B and E completely dissolved within 5 minutes.

Dissolution at non-sink conditions was tested to further compare un-milled and milled Formula (III) formulations. The solubility of Formula (III) increases significantly below pH 3. A non-sink condition was achieved in a dissolution medium that contained phosphate buffer at pH 6.8. A typical dose of Formula (III), 100 mg, was not fully soluble in 900 mL phosphate buffer at pH 6.8 used for dissolution testing. Both Formulations B and E reached equilibrium solubility after an initial transient period (approximately 30 minutes) of supersaturation; in contrast less of the un-milled Formula (III) dissolved in the buffer over a longer period of time.

In conclusion, dissolution comparisons showed significantly improved dissolution characteristic of nano-milled suspension compared to umilled Formula (III) at low and neutral pH consitions in both sink (FIG. 4A, FIG. 4B) and non-sink conditions (FIG. 4C).

Chemical stability. No significant changes were observed for potency or purity of the two nanosuspensions (Formulations B and E) during 3 months of storage at 25 °C/60% relative humidity (RH) or 40 °C /75% relative humidity (Tables 11J and Table 11K). This was consistent with the fact that nanomilled Formula (III) remained crystalline, which preserved the good chemical stability of the crystalline bulk drug substance.

**Table 11J**

| | Test Conditions | API | HPC + DOSS (Formulation B) | | | | |
|---|---|---|---|---|---|---|---|
| | | | T=0 | T=1 month | | T=3 months | |
| | | | | 25°C/60%RH | 40°C/75%RH | 25°C/60%RH | 40°C/75%RH |
| | Potency (Assay%) | 100 | 103.1 | 106.0 | 106.4 | 107.9 | 110.1 |
| | Total Impurity % | 0.6 | 0.8 | 1.0 | 0.8 | 0.4 | 0.4 |
| % Impurity by RRT | 0.80 | 0.2 | 0.2 | 0.2 | 0.2 | 0.2 | 0.2 |
| | 0.83 | -- | 0.1 | 0.1 | 0.1 | -- | -- |
| | 0.94 | 0.2 | 0.3 | 0.3 | 0.2 | -- | -- |
| | 0.98 | -- | -- | 0.2 | 0.1 | -- | -- |
| | 1.04 | -- | -- | -- | -- | -- | -- |
| | 10.6 | 0.2 | 0.2 | 0.2 | 0.2 | 0.02 | 0.2 |

**Table 11K**

| | Test Conditions | API | PVP-VA + DOSS (Formulation E) | | | | |
|---|---|---|---|---|---|---|---|
| | | | T=0 | T=1 month | | T=3 months | |
| | | | | 25°C/60%RH | 40°C/75%RH | 25°C/60%RH | 40°C/75%RH |
| | Potency (Assay%) | 100 | 108.1 | 110.1 | 110.2 | 114.3 | 105.7 |
| | Total Impurity % | 0.6 | 0.8 | 0.7 | 0.7 | 0.7 | 0.5 |
| % Impurity by RRT | 0.80 | 0.2 | 0.2 | 0.2 | 0.2 | 0.3 | 0.2 |
| | 0.83 | -- | 0.1 | 0.1 | 0.1 | -- | -- |
| | 0.94 | 0.2 | 0.3 | 0.2 | 0.2 | 0.1 | 0.1 |
| | 0.98 | -- | -- | -- | -- | -- | -- |
| | 1.04 | -- | -- | -- | -- | 0.1 | -- |
| | 10.6 | 0.2 | 0.2 | 0.2 | 0.2 | 0.02 | 0.2 |

Physical stability (PSD). The effectiveness of the stabilizer system in preventing nanoparticle aggregation and agglomeration was investigated at 25°C or 40° for 3 months. The D10 and D50 for both formulations remained constant under both conditions at 1 month and 3 months (Table 11L). The increasing D90 was attributed to the formation of a small percentage of large aggregates. The small amount of larger particles was unlikely to have a significant impact on the dissolution profiles as discussed in the next section.

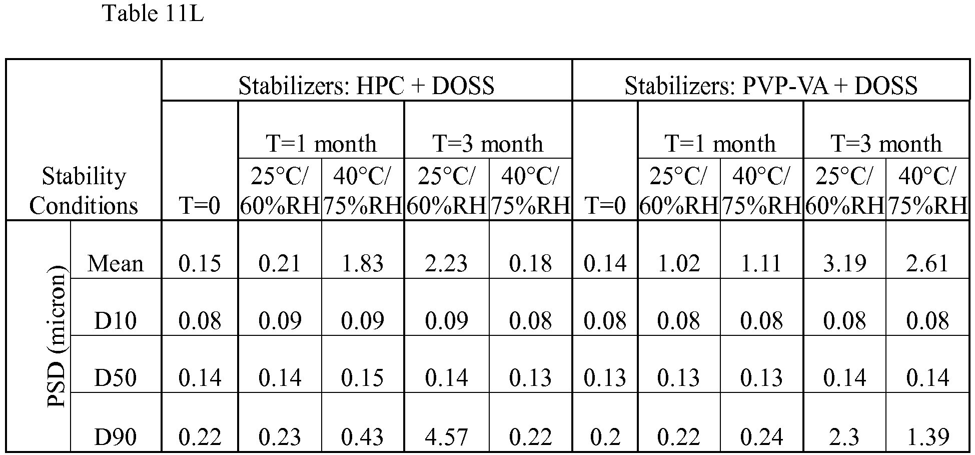

Dissolution stability. No change in dissolution profile was observed for up to 3 months of storage at 25°C or 40°C. Both formulations showed high consistency in the dissolution profile. The small change in the dissolution end point could be attributed to suspension evaporation.

### Example 12: Pharmaceutical Compositions of Embodiment N

The study was an evaluation of the pharmacokinetics of Formula (III) following PO (oral) administration at 30 mg/kg in male Sprague-Dawley rats. Formulations were tested with four (4) rats in each dose group. Dose level of Formula (III) was chosen to be 30 mg/mL, well below the dose level where toxicity is observed, while still high enough to be discriminating for comparing potential dissolution-limited oral absorption. The dose volume was 5 mL/kg and the concentration was 6 mg/mL. Plasma samples were collected Pre-dose, 15, 30 min, 1, 2, 4, 6, 8, and 24 hours. The formulations shown in Table 12A were tested.

**Table 12A**

| Formulation | Composition | Dose (mg/kg) | Dose Vol (ml/kg) | Concentration (mg/mL) |
|---|---|---|---|---|
| Nano HPC | 10 wt% nano suspension in 2% HPC+ 0.1%Tween (HPC) | 30 | 5 | 6 |
| Nano Copovidone | 10 wt% nano suspension in 2% HPC, 0.1%Tween (Copovidone) | 30 | 5 | 6 |

The average plasma profiles and PK parameters for the groups are compared in Table 12B and Table 12C. The highest exposure (AUC) was obtained HPC based nanosuspension. The slightly extended release profile and overall lower absorption of the Copovidone based HME in corn oil could be partially or entirely caused by suspending the formulation in corn oil.

**Table 12B**

| PK Parameters | T_{1/2} (hr) | Tₘₐₓ (hr) | Cₘₐₓ (ng/mL) | AUCₗₐₛₜ (hr*ng/mL) |
|---|---|---|---|---|
| Formulation | Mean (SD) | Mean (SD) | Mean (SD) | Mean (SD) |
| Nanoparticles HPC | 2.9 (1.5) | 0.6 (0.25) | 3583 (1434) | 7278 (4295) |
| Nanoparticles Copovidone | 3.0 (1.0) | 0.8 (0.29) | 1228 (290) | 2764 (357) |

### Example 13: Pharmaceutical Compositions of Embodiment N

Crystalline drug particles of Formula (III) having the particle size distribution shown in Table 13A were used to prepare Nanosuspension #001-35 shown in Table 13B. The hydroxypropyl cellulose (HPC-SL) can have an average molecular weight of about 100,000 Daltons.

**Table 13A**

| Particle Size Distribution (nm) | | |
|---|---|---|
| D10 | D50 | D90 |
| 73 | 169 | 714 |

**Table 13B**

| Compound | % w/w |
|---|---|
| Formula (III) | 30.0 |
| Hydroxypropyl Cellulose (HPC) | 6.0 |
| dioctyl sulfosuccinate (DOSS) | 0.3 |
| Water | q.s. |

The nanosuspension shown in Table 13B was then mixed with various binders to prepare a casting suspension suitable for casting onto inert cores, such as sugar spheres, to form an outer layer around the inert cores. The casting suspensions are shown in Table 13C. The casting process was as follows: the polymer (HPC/HPMC) and/or sucrose were slowly dissolved in a calculated amount of water to obtain a 20% solids content; then the required amount of the nanosuspension of Table 13B was added and mixed until uniform; then the mixed suspension was cast onto an aluminum foil or pan and kept at 40°C overnight; and then the cast film was broken with a grinder and the particle size distribution was measured with Malvern (deionized water was used as the medium for testing. The samples were ground and screened through a standard #60 (250 micron) screen prior to being sonicated for 30 seconds to de-agglomerate all the partings in the medium. The particle size distributions of Formula (III) from the casted films is shown in Table 13D. With reference to Table 13D, the particles of Formula (III) in certain formulations agglomerated to larger particles. Based on the film casting experiments, it was determined that Formulation 8004C provided the best stability of the nanoparticles of Formula (III).

**Table 13C**

| Component | 8004A | 8004B | 8004C | 8004D | 8004E | 8004F |
|---|---|---|---|---|---|---|
| Formula (III) solid content from suspension of Table 4B | 100.0 | 50.0 | 45.5 | 45.5 | 83.3 | 41.7 |
| Sucrose | | 50.0 | 45.5 | 45.5 | -- | 41.7 |
| HPC (KLUCEL® EF) | | -- | 9.0 | -- | 16.7 | 16.6 |
| HPMC (METHOCEL® E5) | | -- | -- | 9.0 | -- | -- |
| Total | 100.0 | 100.0 | 100.0 | 100.0 | 100.0 | 100.0 |

**Table 13D**

| | Particle Size Distribution (nm) | | |
|---|---|---|---|
| | D10 | D50 | D90 |
| Table 4B Suspension | 73 | 169 | 714 |
| 8004A | 86 | 286 | 6,261 |
| 8004B | 73 | 167 | 744 |
| 8004C | 73 | 167 | 626 |
| 8004D | 77 | 190 | 1,674 |
| 8004E | 79 | 201 | 1,608 |
| 8004F | 70 | 150 | 374 |

### Example 14: Pharmaceutical Compositions of Embodiment N

Beads containing an inert core and a drug layer were prepared, and a 100 mg dose of Formula (III) was placed into a capsule size "0" and the fill weight was about 550 mg based on the density of the inert core (sugar sphere #30-55, 500-600 microns). Formulation 8024B is shown in Table 14A, and the process conditions to prepare it are shown in Table 14B.

**Table 14A**

| Formula (III) Nanosuspension | |
|---|---|
| Formula (III) | 100.0 mg |
| HPC | 20.0 mg |
| DOSS | 1.0 mg |

| Additions to form Casting Suspension | |
|---|---|
| Sucrose | 100.0 mg |
| HPC (KLUCEL® EF) | 20.0 mg |

| Inert Core | |
|---|---|
| Sugar Sphere #30-35 (500-600 microns) | 300.0 mg |

**Table 14B**

| Process Conditions | |
|---|---|
| Fluid Bed | Vector FL-1 Fluid Bed |
| Product Temperature | 50°C |
| Inlet Temperature | 54°C to 64°C |
| Spray Rate | 0.2 to 2 g/minute |
| Atomization Air Pressure | 30-34 bar |
| Air Volume | 63-64 CFM |

Beads were prepared having coating levels of 25%, 50%, 75%, and 100% of the total coating level in the formula in Table 14A to determine the particle size distribution of the layered beads by the Malvern 200, with the results being shown in FIGS 5A-5F. FIG. 5A shows particle size distribution of the drug film. FIG. 5B shows particle size distribution of insert cores. FIG. 5C shows particle size distribution of beads having a 25% coating layer. FIG. 5D shows particle size distribution of beads having a 50% coating layer. FIG. 5E shows particle size distribution of beads having a 75% coating layer. FIG. 5F shows particle size distribution of beads having a 100% coating layer.

The amount of the smaller size drug nanoparticles increased as the level of coating increased, with the smaller hump representing the particle size distribution of the drug nanoparticle suspension and the larger hum representing the particle size distribution of the sugar spheres, which largely remained unchanged throughout the process. These results confirm that the drug nanoparticle suspension process using fluid bed processor had no impact on the drug nanoparticle size distribution. The particle size distribution of the drug nanoparticles on sugar spheres was similar to that of the particle size distribution of drug nanoparticles in the film. The initial chemical characterization of the beads is provided in Table 14C.

**Table 14C**

| | Formula (III) | Beads with 100% Coating |
|---|---|---|
| Assay, % LC | NA | 74.2 |
| Total Impurities, % | 0.80 | 0.67 |

As shown in Table 14C, the total impurity was well below the target limit of 1.5%. The assay was low, most likely due to the size of the batch (200 grams) since the minimum amount of drug suspension had to be used, which is smaller than the optimum size for the Vector FL1 fluid bed.

### Example 15: Pharmaceutical Compositions of Embodiment N

Beads containing an inert core and a drug layer (30% drug loading) were prepared, and a 25 mg dose of Formula (III) was placed into a capsule size #3 (opaque white gelatin capsule) and the fill weight was about 135 mg based on the density of the inert core (sugar sphere #30-55, 500-600 microns). Formulation 8030B is shown in Table 15A, and the process conditions to prepare it are shown in Table 15B.

**Table 15A**

| Formula (III) Nanosuspension | |
|---|---|
| Formula (III) | 25.0 mg |
| HPC | 5.0 mg |
| DOSS | 0.25 mg |

| Additions to form Casting Suspension | |
|---|---|
| Sucrose | 25.0 mg |
| HPC (KLUCEL® EF) | 5.0 mg |

| Inert Core | |
|---|---|
| Sugar Sphere #30-35 (500-600 microns) | 71.4 mg |

| Overcoat Layer | |
|---|---|
| HPC (KLUCEL® EF) | 2.86 |
| Talc | 0.46 |

**Table 15B**

| Process Conditions | |
|---|---|
| Fluid Bed | Vector FL-1 Fluid Bed |
| Product Temperature | 50°C |
| Batch Size | 600 grams |
| Nozzle Tip Diameter | 1.0 mm |
| Inlet Temperature | 54°C to 64°C |
| Exhaust Temperature | 48°C to 53°C |
| Spray Rate | 0.2 to 2 g/minute |
| Atomization Air Pressure | 30-40 bar |
| Air Volume | 63-64 CFM |

The coated sugar spheres were tested for dissolution in 900 mL of 0.1N HCl, USP-I apparatus (basket) at 100 rpm, and the dissolution profile is shown in FIG. 6. The small lag time was caused by the disintegration of the capsule and the overcoat layer.

The capsules were stored and monitored at controlled room temperature (CRT, 25°C/60%RH) and accelerated conditions (ACC, 40°C/75%RH), and evaluated for chemical characterization up to 3 months, as shown in Table 15C and Table 15D, respectively.

**Table 15C**

| | | 8030A Capsules | | | |
|---|---|---|---|---|---|
| | API | Initial | 1 month CRT | 3 months CRT | 3 months CRT |
| Assay, % LC | NA | 101.9 | 101.5 | 102.3 | 101.2 |
| Total Impurities | 0.80 | 0.74 | 0.65 | 0.54 | 0.61 |

**Table 15D**

| | | 8030A Capsules | | | |
|---|---|---|---|---|---|
| | API | Initial | 1 month ACC | 3 months ACC | 3 months ACC |
| Assay, % LC | NA | 101.9 | 101.7 | 101.7 | 102.5 |
| Total Impurities | 0.80 | 0.74 | 0.73 | 0.60 | 0.59 |

The dissolution provide of the capsules after storage for up to 3 months at CRT and ACC conditions are shown in FIG. 7, which along with Tables 15C and 15D show that there was no significant change in chemical characterization or dissolution profile.

While various embodiments and aspects are shown and described herein, it will be obvious to those skilled in the art that such embodiments and aspects are provided by way of example only. Numerous variations, changes, and substitutions will now occur to those skilled in the art. It should be understood that various alternatives to the embodiments described herein may be employed.

## Claims

1. An amorphous solid dispersion comprising a compound of Formula (III) or a pharmaceutically acceptable salt thereof and a pharmaceutically acceptable polymer; wherein the compound of Formula (III) is:

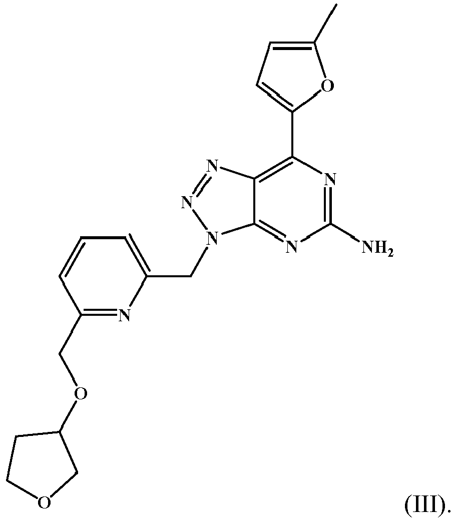

2. The amorphous solid dispersion of claim 1, wherein the pharmaceutically acceptable polymer is a thermoplastic polymer.

3. The amorphous solid dispersion of claim 2 having a glass transition temperature between about 65°C and about 150°C.

4. The amorphous solid dispersion of claim 2 or 3, wherein the thermoplastic polymer comprises: (i) a polyvinylpyrrolidone polymer, (ii) a polyvinylpyrrolidone copolymer, (iii) an acrylic polymer, (iv) an acrylic copolymer, (v) hydroxypropyl methylcellulose acetate succinate, or (vi) a combination of two or more of the foregoing.

5. The amorphous solid dispersion of claim 2 or 3, wherein the thermoplastic polymer comprises: (i) a copolymer of polyvinylpyrrolidone and vinyl acetate; (ii) a methyl methacrylate polymer; (iii) an ethyl acrylate polymer; (iv) a copolymer of methyl methacrylate and ethyl methacrylate; (v) a copolymer of methacrylic acid and methyl methacrylate; or (vi) a copolymer of methacrylic acid and ethyl acrylate.

6. The amorphous solid dispersion of any one of claims 2 to 5, comprising about 20 wt% to about 40 wt% of the compound of Formula (III) and about 60 wt% to about 80 wt% of the thermoplastic polymer.

7. The amorphous solid dispersion of claim 1, wherein the pharmaceutically acceptable polymer is an enteric polymer.

8. The amorphous solid dispersion of claim 1, wherein the pharmaceutically acceptable polymer is an acrylic polymer or an acrylic copolymer.

9. The amorphous solid dispersion of claim 1, wherein the pharmaceutically acceptable polymer is a methyl methacrylate polymer, an ethyl acrylate polymer, or a copolymer of methyl methacrylate and ethyl acrylate.

10. The amorphous solid dispersion of claim 1, 8, 9, or 10, comprising about 5 wt% to about 25 wt% of the compound of Formula (III), and about 75 wt% to about 95 wt% of the pharmaceutically acceptable polymer.

11. A pharmaceutical composition comprising: (i) a compound of Formula (III) or a pharmaceutically acceptable salt thereof having a size distribution with a D90 of about 1000 nm or less, measured by laser diffraction spectroscopy; and (ii) a pharmaceutically acceptable polymer, a surfactant, or a combination thereof; wherein the compound of Formula (III) is:

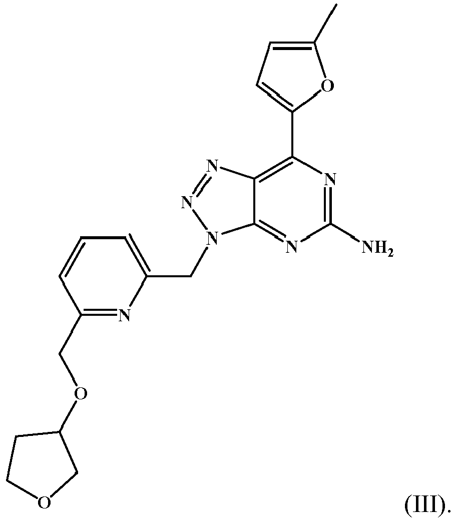

12. The composition of claim 11, comprising from about 10 wt% to about 45 wt% of the compound of Formula (III).

13. The composition of claim 11 or 12, wherein (ii) comprises about 0.5 wt% to about 10 wt% of the pharmaceutically acceptable polymer and about 0.005 wt% to about 1.0 wt% of the surfactant.

14. The composition of any one of claims 11 to 13, wherein the surfactant is sodium lauryl sulfate, ammonium lauryl sulfate, sodium laureth sulfate, sodium myreth sulfate, sodium stearate, dioctyl sodium sulfosuccinate, or a combination of two or more thereof.

15. The composition of any one of claims 11 to 14, wherein the pharmaceutically acceptable polymer comprises a polyvinylpyrrolidone polymer, a polyvinylpyrrolidone copolymer, a cellulose compound, or a combination of two or more of the foregoing.
